# EUROPEAN PATENT APPLICATION

(11) **EP 0 805 147 A1**
(43) Date of publication of application: **05.11.1997**
(21) Application number: 97302997.8
(22) Date of filing: 01.05.1997
(51) Int. Cl.: C07C 237/04, C07C 237/20, C07C 255/60, C07C 323/60, C07D 213/57, C07D 333/24, C07D 233/54, C07D 277/04, C07D 207/16, A61K 31/16, A61K 31/275, A61K 31/40

(54) **Novel N-substituted alpha aminoacid amides as calcium channel modulators**

(30) Priority: 03.05.1996 GB 9609299; 26.03.1997 GB 9706302
(71) Applicant: LILLY INDUSTRIES LIMITED, Basingstoke Hants RG21 2XA (GB)
(72) Inventor: Ambler, Samantha Jayne, Lilly Res. Ctr. lim., Windlesham, Surry GU20 6PH (GB); Dell, Colin Peter, Lilly Res. Ctr. lim., Windlesham, Surry GU20 6PH (GB); Gilmore, Jeremy, Lilly Res. Ctr. lim., Windlesham, Surry GU20 6PH (GB); Hotten, Terrence Michael, Lilly Res. Ctr. lim., Windlesham, Surry GU20 6PH (GB); Sanchiz-Suarez, Ana, Lilly Res. Ctr. lim., Windlesham, Surry GU20 6PH (GB); Simmonds, Robin George, Lilly Res. Ctr. lim., Windlesham, Surry GU20 6PH (GB); Timms, Graham Henry, Lilly Res. Ctr. lim., Windlesham, Surry GU20 6PH (GB); Tupper, David Edward, Lilly Res. Ctr. lim., Windlesham, Surry GU20 6PH (GB); Urquhart, Michel William John, Lilly Res. Ctr. Lim, Windlesham, Surry GU20 6PH (GB)
(74) Representative: Hudson, Christopher Mark

(57) **Abstract**

The compounds of formula I and derivatives thereof have been found to be active in tests that show modulation of voltage-dependent calcium channels, and are thus indicated for use in the treatment of diseases in which such modulation is beneficial, in particular diseases of the central nervous system.

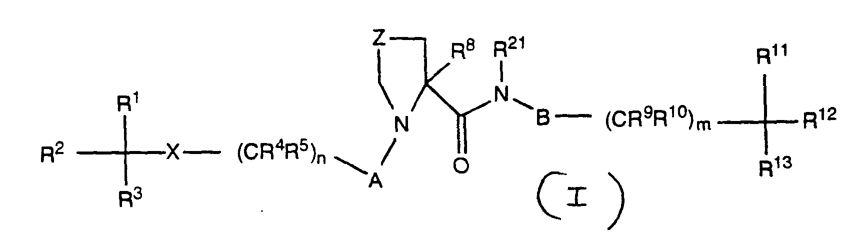

## Description

This invention relates to novel compounds and their use as pharmaceuticals.

One group of compounds that has activity in tests designed to show utility in the treatment of disorders of the central nervous system can be broadly described as phenylalkylamines. Compounds of this type include emopamil, verapamil and noremopamil disclosed, for example, in EP-147707.

The compounds of the invention have the following formula: 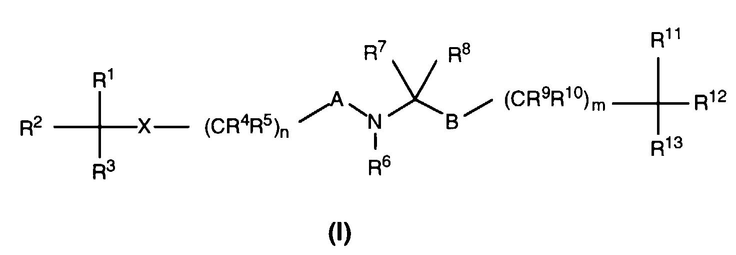 in which
R¹ is alkyl, -SO₂R¹⁴ where R¹⁴ is alkyl, optionally substituted aryl or optionally substituted heteroaryl,
R² is hydrogen, alkyl optionally substituted with one or more hydroxy or C₁₋₄ alkoxy groups, optionally substituted aryl, optionally substituted heteroaryl, -OR¹⁵, -P(O) (OR¹⁵) (OR¹⁶) or -NR¹⁵R¹⁶ where R¹⁵ and R¹⁶ are each hydrogen or alkyl, or -SO₂R¹⁷ where R¹⁷ is hydrogen, alkyl, optionally substituted aryl or optionally substituted heteroaryl,
R³ is hydrogen, -CN, -CO₂R¹⁸, -CONR¹⁸R¹⁹ ,-CH¹⁸ ≡CHR¹⁹ , -C≡CR¹⁸, -OCH₂CO.R¹⁸, -SO₂R¹⁸, -CH₂OR¹⁸, -CH₂OCO.R¹⁸, -CH₂NHCOR¹⁸, -CH₂N(COR¹⁸)₂, -CHO, -OR¹⁸, -CH₂NR¹⁸R¹⁹, -CH₂OR¹⁸, -CH=NR¹⁸ or -CH=N-OR¹⁸, where R¹⁸ and R¹⁹ are each hydrogen or alkyl,
X is a bond or -SO₂-,
R⁴, R⁵, R⁶, R⁸, R⁹ and R¹⁰ are each hydrogen or alkyl,
n is 1 to 4,
A is -CH₂- or -YCO- where Y is a bond, -O-, optionally substituted phenylene, -CH=CH-, or -NR²⁰- where R²⁰ is hydrogen or alkyl,
R⁷ is the C-α substituent of an amino acid or an ester thereof, or R⁶ and R⁷ together form a C₁₋₃ alkylene chain optionally substituted by C₁₋₄ alkyl or hydroxyl, or a group of the formula -CH₂-Z-CH₂- where Z is -CO-, -S-, -SO-or -SO₂-,
or R⁷ and R⁸ together form a C₃ to C₅ alkylene chain optionally substituted by C₁₋₄ alkyl or hydroxyl,
B is -CON(R²¹)-, -CON(R²¹)N(R²²)- or 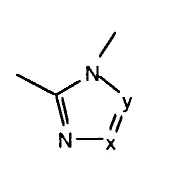 where -x=y- is -C(R²²)=N-, -N=C(R²²)- or -N=N-, where R²¹ is hydrogen, alkyl or -NR²³ R²⁴ where R²³ and R²⁴ are each hydrogen or alkyl, and where R²² is hydrogen or alkyl, or R⁸ and R²¹ together form a C₂₋₄ alkylene chain,
m is 0 to 2,
R¹¹ is hydrogen or alkyl,
R¹² is hydrogen, alkyl, optionally substituted aryl, optionally substituted heteroaryl, -CN, -OH, -CO₂R²⁵, -CONR²⁵R²⁶, where R²⁵ and R²⁶ are each hydrogen or alkyl, and
R¹³ is alkyl, optionally substituted aryl or optionally substituted heteroaryl, or R¹² and R¹³ together form a C₃₋₈ cycloalkyl group or C₃₋₈ cycloalkyl fused to optionally substituted phenyl;
or a salt or ester thereof.

The compounds of the invention have been found to be active in tests that show modulation of voltage-dependent calcium channels, and are thus indicated for use in the treatment of diseases in which such modulation is beneficial, in particular diseases of the central nervous system.

In the above Formula (I), optionally substituted aryl includes optionally substituted phenyl or naphthyl, and is preferably optionally substituted phenyl. Substituents on the aryl nucleus can be selected from, for example, C₁₋₄ alkyl, especially methyl, C₁₋₄ alkoxy, especially methoxy and ethoxy, carboxy, hydroxy, cyano, halo, especially chloro, bromo and fluoro, trifluoromethyl, trifluoromethoxy, nitro, amino, C₁₋₄ acylamino and C₁₋₄ alkylthio. There can be one or more substituents and preferably one to three substituents. When the optionally substituted aryl group is substituted phenyl, it is preferably substituted by one or two substituents selected from halo, C₁₋₄ alkyl, C₁₋₄ alkoxy, trifluoromethyl, nitro and trifluoromethoxy.

A heteroaryl group can be any aryl group having one or more hetero atoms in the ring. The term includes fused ring structures. Preferably the heteroaryl group contains one or two hetero atoms selected from oxygen, nitrogen and sulphur. It preferably contains from 5 to 10 carbon atoms, and for example may be of the formula: 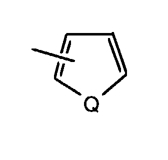 where Q is -O-, -S- or -NR-, and R is hydrogen or C₁₋₄ alkyl. Alternatively, a heteroaryl group comprises a benzene fused ring as, for example: 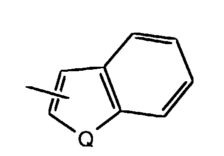 and further heteroaryl groups include:

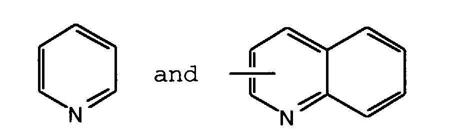

Especially preferred heteroaryl groups are pyridyl, in particular 2- and 3-pyridyl.

The groups R², R¹² and R¹³ are preferably optionally substituted aryl and more preferably optionally substituted phenyl, especially unsubstituted phenyl.

Groups R⁴, R⁵, R^{6,} R⁸, R⁹ and R¹⁰ are preferably hydrogen, or R⁴, R⁵, R⁸, R⁹ and R¹⁰ are hydrogen and R⁶ and R⁷ together form a ring. It will further be appreciated that the values of R⁴ and R⁵ in each alkylene unit are not necessarily the same, and when m is 2 values of R⁸ and R⁹ in the two alkylene units may differ.

When reference is made to alkyl, the alkyl group is preferably C₁₋₆ alkyl. Larger alkyl groups can be employed as, for example, those of up to 10 carbon atoms. The alkyl group can be branched or unbranched and, for example, can be methyl, ethyl, propyl, isopropyl, butyl, isobutyl, t-butyl or neopentyl, and is, in the case of R⁴ to R⁷, R⁸ to R¹⁰ and R¹⁴ to R²⁶, preferably methyl or ethyl. When R² is alkyl it can be substituted by hydroxy or alkoxy groups and preferred examples of such substituents are -CH₂OH, -CH₂CH₂OH, -CH₂OCH₃, -CH₂OC₂H₅ and -CH₂CH₂OCH₃. R² is preferably unsubstituted alkyl, especially C₁₋₆ alkyl, preferred values being methyl, ethyl, isopropyl, isobutyl and neopentyl. An especially preferred value of R² is isopropyl.

The group R⁷ can comprise the C-α substituent of an amino acid or an ester thereof, that is to say, a side chain derived from an amino acid of general formula R₇CH(NH₂)CO₂H. Ester derivatives are particularly those in which R⁷ contains a carboxy group esterified, for example, by a C₁₋₄ alkanol. The amino acid can be any conventional acid either natural or synthetic. R⁷ is preferably the residue of a naturally-occurring amino acid, for example the residue of glycine, alanine, valine, leucine, isoleucine, serine, threonine, cysteine, cystine, methionine, phenylalanine, tyrosine, tryptophan, lysine, hydroxylysine, arginine, histidine, aspartic acid, an ester thereof, glutamic acid or an ester thereof, all of these acids being preferably in the S-configuration. Thus R⁷ can be, for example, hydrogen, or C₁₋₄ alkyl optionally substituted by one or more hydroxy, mercapto, C₁₋₄ alkylthio, optionally substituted phenyl, amino, guanidino, carboxy, carbomethoxy, carboethoxy, imidazolyl or indolyl group. An optionally substituted phenyl is phenyl or phenyl substituted with one or more of the substituents defined above for optionally substituted aryl. Examples of R⁷ substituents include hydrogen, -CH₃, -CH(CH₃)₂, -CH₂CH(CH₃)₂, -CH(CH₃)CH₂CH₃, -CH₂OH, -CH(OH)CH₃, -CH₂SH, -CH₂SSCH₂CH(NH₂)COOH, -CH₂CH₂SCH_{3,} -CH₂Ph, -CH₂Ph-4OH, -CH₂CH₂CH₂CH₂NH₂, -CH₂CH₂CH(OH)CH₂NH₂, -CH₂CH₂CH₂NHC(=NH)NH₂, -CH₂COOH, -CH₂COOCH₃, -CH₂COOC₂H₅, -CH₂CH₂COOH, -CH₂CH₂COOCH₃ and -CH₂CH₂COOC₂H₅. Preferred examples are the residues of glycine (hydrogen), tyrosine (-CH₂Ph-4OH), leucine (-CH₂CH(CH₃)₂), methionine (-CH₂CH₂SCH₃), valine (-CH(CH₃)₂), isoleucine (-CH(CH₃)(CH₂CH₃)), and aspartic acid (-CH₂COOH), especially methyl ester (-CH₂COOCH₃). As mentioned above, R⁶ and R⁷ can together form an alkylene ring as can be derived, for example, from proline or hydroxyproline.

It will be appreciated that the compounds of the invention can contain one or more asymmetric carbon atom which gives rise to enantiomers. The compounds can be prepared as racemates or can be made from enantiomeric intermediates. Both racemates and enantiomers form part of the present invention.

It will also be understood that salts of the compounds of the invention can be prepared and such salts are included in the invention. They can be any of the well known base or acid addition salts. Examples of base salts are those derived from ammonium hydroxide and alkali and alkaline earth metal hydroxides, carbonates and bicarbonates, as well as salts derived from aliphatic and aromatic amines, aliphatic diamines and hydroxy alkylamines. Bases especially useful in the preparation of such salts include ammonium hydroxide, potassium carbonate, sodium bicarbonate, lithium hydroxide, calcium hydroxide, methylamine, diethylamine, ethylene diamine, cyclohexylamine and ethanolamine. The potassium, sodium and lithium salt forms are particularly preferred.

Acid addition salts are preferably the pharmaceutically acceptable non-toxic addition salts with suitable acids, such as those with inorganic acids, for example hydrochloric, hydrobromic, nitric, sulphuric or phosphoric acids, or with organic acids, such as organic carboxylic acids, for example glycollic, maleic, fumaric, malic,oxalic, tartaric, citric, salicylic or o-acetoxybenzoic acids, or organic sulphonic acids, methane sulphonic, 2-hydroxyethane sulphonic, toluene-p-sulphonic or naphthalene-2-sulphonic acids.

In addition to pharmaceutically-acceptable salts, other salts are included in the invention. They may serve as intermediates in the purification of compounds or in the preparation of other, for example pharmaceutically-acceptable, salts, or are useful for identification, characterisation or purification.

The compounds can also be utilised in ester form, such esters being aliphatic or aromatic. The esters principally concerned are those derived from compounds in which R³ is -COOH or in which the R³ group bears one or more -COOH. The most preferred esters are alkyl esters derived from C₁₋₄ alkanols, especially methyl and ethyl esters. Other esters that can be employed are hydroxylated esters derived from, for example, glycollic, malic, tartaric, citric, salicylic or 2-hydroxyethane sulphonic acids.

A particular group of compounds of the invention is of formula (I) above, in which R¹ and R¹³ are each optionally substituted aryl or optionally substituted heteroaryl, R² is hydrogen, alkyl optionally substituted with one or more hydroxy or C₁₋₄ alkoxy group, optionally substituted aryl, optionally substituted heteroaryl, -OR¹⁵ , -SO₂R¹⁵, -P(O)(OR¹⁵)(OR¹⁶), -NR¹⁵R¹⁶, where R¹⁵ and R¹⁶ are each hydrogen or alkyl,
R³ is hydrogen, -CN, -CO₂R¹⁸, -CONR¹⁸R¹⁹, -CR¹⁸=CHR¹⁹, -C≡CR¹⁸, -CHO, -OR¹⁸, -CH₂NR¹⁸R¹⁹, -CH₂OR¹⁸, -CH=NR¹⁸ or -CH=N-OR¹⁸, where R¹⁸ and R¹⁹ are each hydrogen or alkyl,
X is a bond,
R⁴, R⁵, R⁶, R⁸, R⁹ and R¹⁰ are each hydrogen or alkyl,
n is 2 to 4 and m is 0 to 2,
A is -CH₂- or 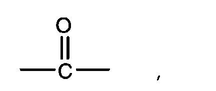
R⁷ is the C-α substituent of an amino acid or an ester thereof, or R⁶ and R⁷ or R⁷ and R⁸, together form a C₃ to C₅ alkylene chain optionally substituted by C₁₋₄ alkyl or hydroxyl,
B is a group of the formula: 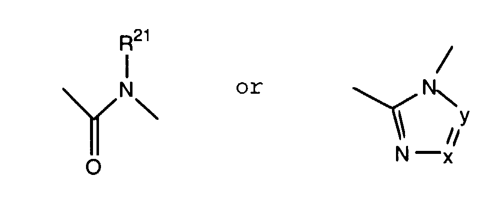
where -x=y- is -C(R²¹)=N-, -N=C(R²¹)- or -N=N-, where R²¹ is hydrogen or alkyl,
R¹¹ is hydrogen or alkyl, and
R¹² is hydrogen, alkyl, optionally substituted aryl, optionally substituted heteroaryl, -CN, -OH, -CO₂R²⁵, -CONR²⁵R²⁶ where R²⁵ and R²⁶ are each hydrogen or alkyl;
or a salt or ester thereof.

A preferred group of compounds of formula (I) is one in which R¹ and R¹³ are optionally substituted phenyl, R² is alkyl, R³ is -CN, X is a bond, R⁴, R⁵, R^{6,} R⁸, R⁹ and R¹⁰ are hydrogen, n is 2, m is 0 or 1, A is -CH₂-, R⁷ is hydrogen, or C₁₋₄ alkyl optionally substituted by one or more hydroxy, mercapto, C₁₋₄ alkylthio, optionally substituted phenyl, amino, guanidino, carboxy, imidazolyl or indolyl, or R⁶ and R⁷ together form a C₃ alkylene chain, B is 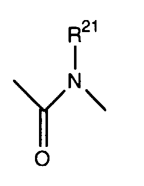 where R²¹ is hydrogen or alkyl, R¹¹ is hydrogen or alkyl, and R¹² is hydrogen, optionally substituted phenyl or -CO₂R²⁵ where R²⁵ is hydrogen or alkyl. A particularly preferred group of compounds are those in which R⁷ is hydrogen, -CH₂Ph-4OH, -CH₂CH(CH₃)₂, -CH₂CH₂SCH₃, -CH(CH₃)₂, -CH(CH₃)(CH₂CH₃) or -CH₂COOCH₃.

It is often preferable that the compounds should be in pure enantiomer form. The most active compounds often have an S-configuration at the carbon atom to which R⁷ is attached, that is to say, they are compounds derived from S-amino acids. Thus, for example, compounds in which R¹ is optionally substituted phenyl, R² is isopropyl and R³ is -CN, are preferably S-enantiomers.

A further preferred group of compounds is of the following formula: 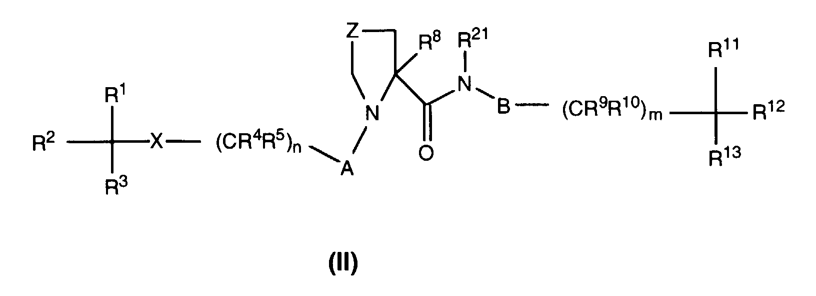 in which R¹ is C₁₋₆ alkyl or -SO₂R¹⁴ where R¹⁴ is C₁₋₆ alkyl,
R² is optionally substituted aryl, optionally substituted heteroaryl or -SO₂R¹⁷ where R¹⁷ is C₁₋₆ alkyl, optionally substituted aryl or optionally substituted heteroaryl,
R³ is -CN, -CO₂R¹⁸, -CONR¹⁸R¹⁹, -C≡CR¹⁸, -OCH₂CO.R¹⁸, -SO₂R¹⁸, -CH₂OR¹⁸, -CH₂OCO.R¹⁸, -CH₂NHCOR¹⁸ or -CH₂N(COR¹⁸)₂, where R¹⁸ and R¹⁹ are each hydrogen or C₁₋₆ alkyl,
R⁴, R⁵, R⁹ and R¹⁰ are each hydrogen or C₁₋₆ alkyl,
R⁸ is hydrogen or C₁₋₆ alkyl,
R²¹ is hydrogen, C₁₋₆ alkyl or -NR²³R²⁴ where R²³ and R²⁴ are each hydrogen or C₁₋₆ alkyl,
or R⁸ and R²¹ together form a C₂₋₄ alkylene group,
R¹¹ is hydrogen or C₁₋₆ alkyl,
R¹² is hydrogen, C₁₋₆ alkyl, optionally substituted aryl, optionally substituted heteroaryl, -CN, -OH, -CO₂R²⁵, -CONR²⁵R²⁶ where R²⁵ and R²⁶ are each hydrogen or C₁₋₆ alkyl,
R¹³ is C₁₋₆ alkyl,optionally substituted aryl or optionally substituted heteroaryl,
or R¹² and R¹³ together form a C₃₋₈ cycloalkyl group or C₃₋₈ cycloalkyl fused to optionally substituted phenyl,
n is 1 to 4 and m is 0 to 2,
X is a bond or -SO₂-,
A is -YCO- where Y is a bond or -O-, optionally substituted phenylene, -CH=CH- or -NR²⁰ - where R²⁰ is hydrogen or C₁₋₆ alkyl,
Z is -CH₂-, -CH(OH)-, -CO-, -S-, -SO- or -SO₂-, and
B is a bond or -N(R²²)-
where R²² is hydrogen or C₁₋₆ alkyl;
or a salt or ester thereof.

Preferred compounds of formula (II) have one or more of the following features:

(i) R¹ is C₁₋₆ alkyl (preferably isopropyl), (ii) R² is optionally substituted phenyl and preferably unsubstituted phenyl, also pyridyl, phenylsulphonyl and thienylsulphonyl, (iii) R³ is -CN, (iv) R³ is -CO₂R¹⁸, -CH₂OR¹⁸, -CH₂OCOR¹⁸ or -CH₂NHCOR¹⁸, (v) R⁴, R⁵, R⁹ and R¹⁰ are hydrogen, (vi) R⁸ is hydrogen, (vii) R²¹ is hydrogen, (viii) when R⁸ and R²¹ are combined they are -(CH₂)₂-, (ix) R¹¹ is optionally substituted phenyl, (x) R¹² is hydrogen or optionally substituted phenyl, preferably unsubstituted phenyl, (xi) R¹³ is hydrogen, (xii) n is 3 or 4, (xiii) m is 0, (xiv) Y is a bond, -O- or -NH-, (xv) A is -CH₂-, (xvi) B is a bond, (xvii) X is a bond.

Preferably the moiety -X-(CR⁴R⁵)ₙ-Y- is of five atoms' length.

Particularly preferred groups of compounds of formula (II) have one of the following combination of features:

(i) X is a bond, n is 4 and Y is a bond, (ii) X is a bond, n is 3 and Y is -O- or -NH-, (iii) X is SO₂, n is 3 and Y is a bond, (iv) R¹ is C₁₋₆ alkyl, R² is phenyl and R³ is -CN, A is -CH₂CO-, B is a bond and m is 0.

An especially preferred group of compounds is one of the formula: 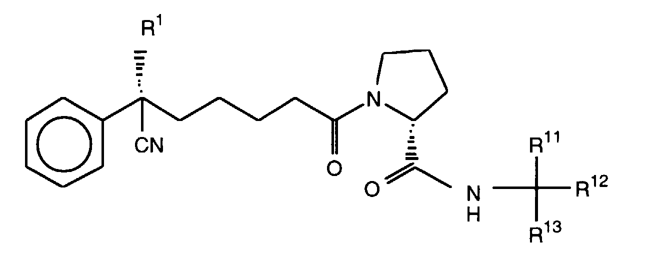 in which R¹ is C₁₋₆ alkyl, R¹¹ is hydrogen, R¹² is hydrogen or optionally substituted phenyl and R¹³ is C₁₋₆ alkyl or optionally substituted phenyl, or a salt or ester thereof.

This invention also includes a process for producing a compound according to formula (I) above, which comprises:
1) reacting a compound of the formula: 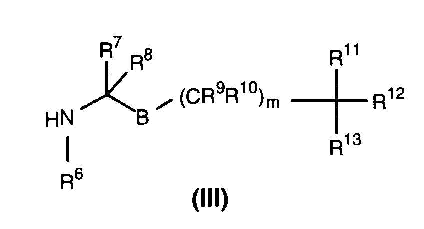 with a compound of the formula: 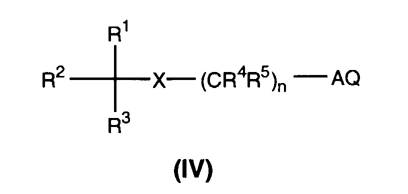 where Q is a leaving group and A is -CH₂- or -YCO-.where Y is a bond, optionally substituted phenylene or -CH=CH-, and the remaining substituents have the values given above;
2) reducing a compound of the formula 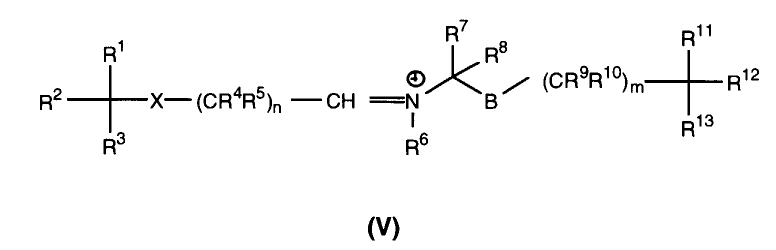 to give a compound of the formula (I) in which A is -CH₂-;
3) reacting a compound of the formula: 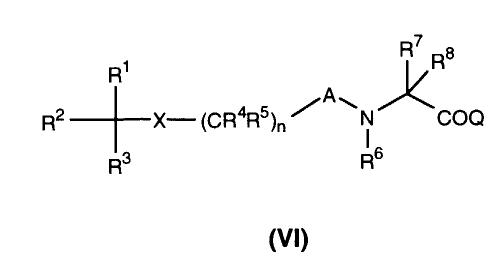 where Q is a leaving group such as halogen, or is -OH,
   with a compound of the formula: 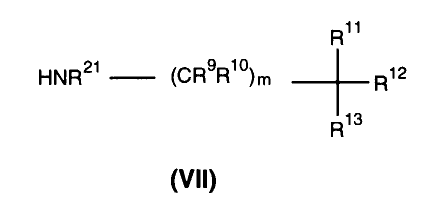 in the presence of a coupling reagent;
4) converting a compound of the formula: 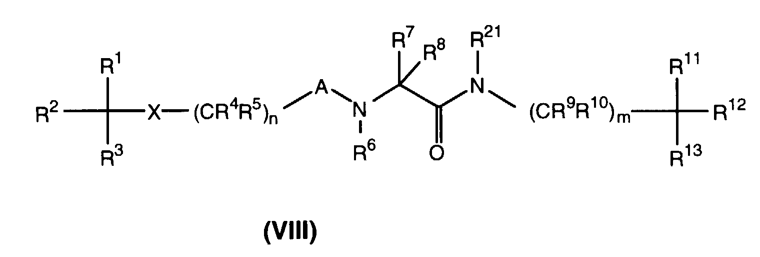 to a compound of formula (I) in which B is 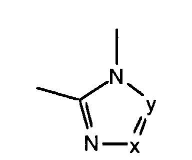
5) reacting a compound of formula (III) above with a compound of the formula 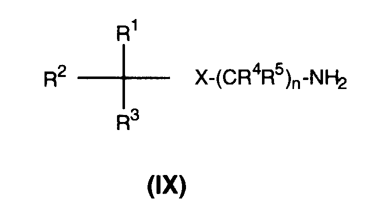 in the presence of a phosgene derived coupling agent, to give a compound of formula (I) in which Y is -NH-;
6) reacting a compound of formula (III) above with a compound of the formula: 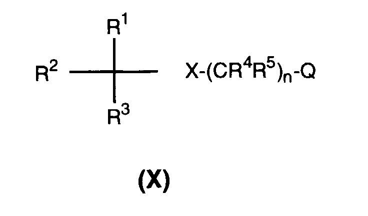 where Q is halo, in the presence of an alkaline metal carbonate, to give a compound of formula (I) in which Y is -O-;
7) oxidising a compound of formula (I) in which Z is -S- or -CH(OH)-,
   to give a compound in which Z is -SO-, -SO₂- or -CO-.

With regard to process variant (1), the leaving group, Q, can take any conventional value. For example, when A is -CH₂-, Q can be halo, especially chloro, mesylate or tosylate, and in these circumstances the reaction is preferably carried out in the presence of a base, for example, sodium or potassium carbonate in DMF or acetone at a temperature, for example, of 50-100° C
When A is -Y(CO)-, the leaving group, Q, can be, for example, halo or -OH. When Q is halo, especially chloro,it is preferred to carry out the reaction in the presence of a base such as, for example, pyridine or triethylamine, and an organic solvent such as, for example, chloroform or dichloromethane, at a temperature of from 0° C. to 50° C. When Q is -OH the reaction is preferably carried out with the assistance of a coupling agent such as is standard in peptide synthesis.

Examples of coupling agents include carbodiimides such as dicyclohexyl carbodiimide, carbonyldiimidazole or 2-chloro-1-methyl-pyridinium iodide. The reaction is preferably performed in an organic solvent, such as, for example,chloroform, dichloromethane or acetonitrile, and at a temperature of from -10° C. to 25° C.

In process variant (1) and in other reactions of the invention it may be desirable to protect substituent groups such as R¹, R², R³, R¹¹, R¹² and R¹³ as described, for example, by E. Haslam in Protective Groups in Organic Chemisty, to avoid undesired side reactions, and subsequently to de-protect to give the desired product.

Compounds of formula (III) employed in process variant (1) can be made by conventional methods, for example, by coupling a protected amino acid with an appropriate amine: 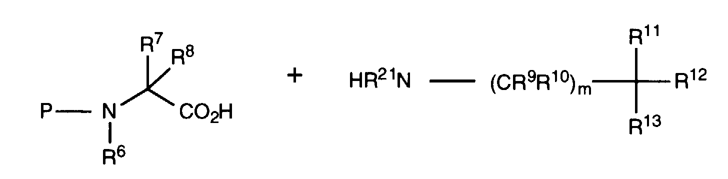 where P is a protecting group such as t-butyloxycarbonyl or benzyloxycarbonyl, with a coupling agent such as, for example, dicyclohexylcarbodiimide, N,N-carbonyldiimidazole or 2-chloro-1-methylpyridinium iodide, and then removing the protecting group.

Intermediates of formula (III), in which B is 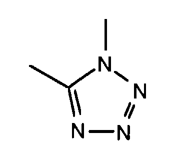 can be prepared by reacting the protected amide produced as above, with trimethylsilyl azide under the conditions of a Mitsunobu reaction, or via the chloroimine and an azide.

Intermediate compounds of formula (IV) can be produced by conventional synthetic routes. The preferred chiral form of the compound of formula (IV) is produced by use of a chiral auxiliary to allow separation of either enantiomer (J. Chem. Soc., Perkin 1, 1996, 2845-2850).

Compounds of formula (IV) in which Y is a bond, -O-, 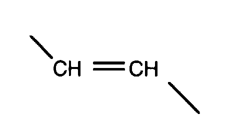 or -NR²⁰-, can be made by starting from an aldehyde or nitrile, by routes such as the following:

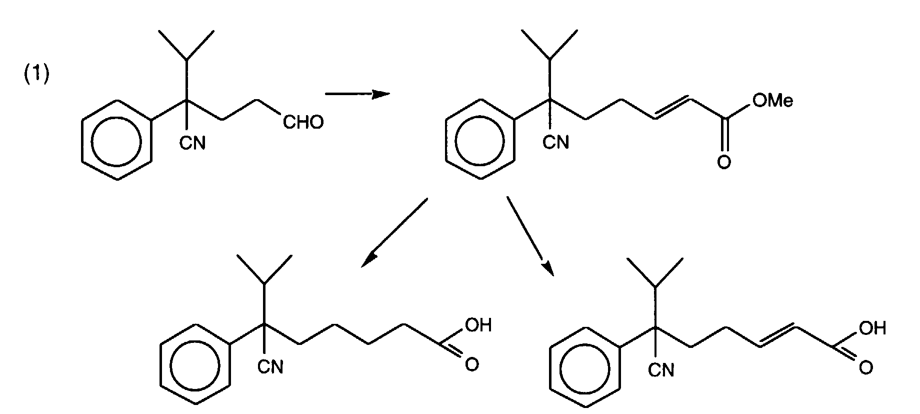

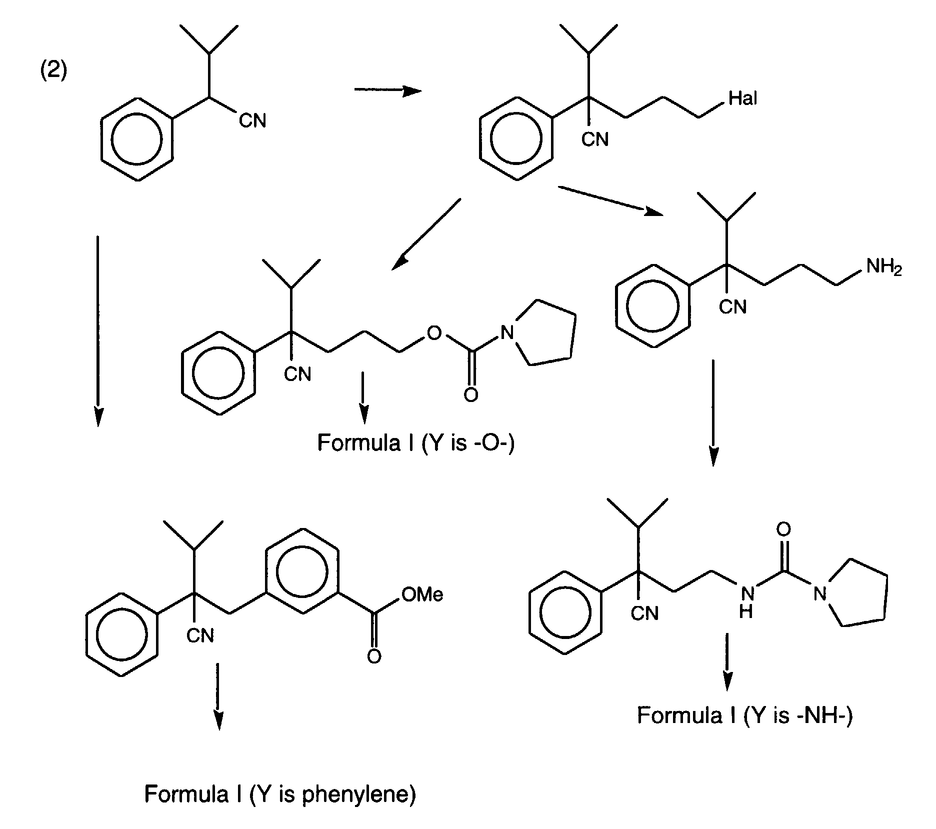

With regard to process variant (2), the reaction is preferably carried out in an organic solvent such as, for example, methanol, ethanol or acetonitrile, with a reducing agent selected from sodium borohydride or sodium cyanoborohydride (when R⁶ is alkyl) and at a room temperature of from -10° C. to 25° C. The intermediate compound of formula (V) is preferably made *in situ* by condensing a compound of formula: 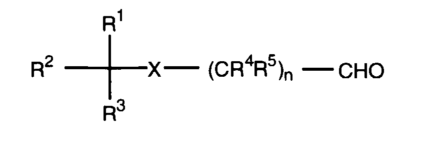 with the amine of formula: 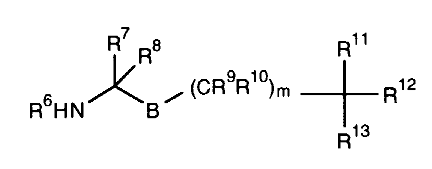 employing a dehydrating agent such as, for example, 3Å molecular sieve.

Conversion of the amide thus produced to intermediates in which B is: 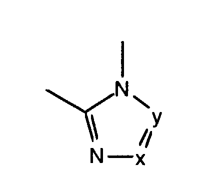 can be effected under the conditions of a Mitsunobu reaction or via the chloroimine, as described above.

With regard to process variant (3), the reaction is preferably carried out under similar conditions to that of process variant (1), in the presence of a base, for example, cesium, sodium or potassium carbonate in dimethylformamide or acetone. An especially preferred base for use in this reaction is cesium carbonate.

The intermediates of process variant (3) of formula (VI) can be prepared by condensation of the appropriate carboxyl protected amino acid of formula: 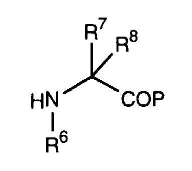 where P is an appropriate procecting group, with a compound of formula (III), or with an aldehyde of formula: 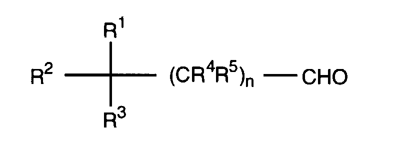 followed by reduction and then removal of the protecting group.

With regard to process variant (4), the process is preferably carried out under the conditions of a Mitsunobu reaction or via the chloroimine and an azide.

The oxidation reaction of process variant (7) is preferably carried out using as oxidising agent such as 3-chloroperbenzoic acid or hydrogen peroxide to oxidise -S- to -SO- or SO₂-, or manganese dioxide, pyridinium chlorochromate or pyridinium dichromate to oxidise -CHOH- to -CO-.

As mentioned above, the compounds of the invention are active in tests that indicate their utility in the treatment of diseases of the central nervous system. The compounds modulate the activity of calcium channels and, in particular, they block voltage sensitive calcium channels as determined in a test based on Minta et al. Journal of Biological Chemistry Vol. 264 No. 14 pp. 8171-8178, 1989, in which measurements of calcium flux using calcium sensitive dyes are made. The compounds described in the following Examples were found to inhibit voltage-dependent calcium channels in either a rat synaptosome preparation or in cloned human cell lines expressing specific voltage-dependent calcium channels with an IC₅₀ of less than 3 µ**M**.

The compounds of the invention are thus indicated for use in the treatment of anoxia, ischaemia, stroke and heart failure, migraine, pain, epilepsy, traumatic head or spinal injury, AIDS related dementia and blindness, amnesia, neurodegenerative diseases such as Alzheimer's, Parkinson's and Huntington's diseases and age-related memory disorders, Down's syndrome, mood disorders, drug or alcohol addition withdrawal, nausea from chemotherapy, and carbon monoxide or cyanide poisoning.

The invention also includes a pharmaceutical composition comprising a pharmaceutically acceptable diluent or carrier in association with the compound of the invention or a pharmaceutically acceptable salt or ester thereof.

The compound may be administered by various routes, for example by the oral or rectal route, topically or parenterally, for example by injection or infusion, being usually employed in the form of a pharmaceutical composition. Such compositions are prepared in a manner well known in the pharmaceutical art and comprise at least one active compound. In making the compositions of the present invention, the active ingredient will usually be mixed with a carrier, or diluted by a carrier, and/or enclosed within a carrier which may, for example, be in the form of a capsule, sachet, paper or other container. When the carrier serves as a diluent, it may be a solid, semi-solid, or liquid material which acts as a vehicle, excipient or medium for the active ingredient. Thus, the composition may be in the form of tablets, lozenges, sachets, cachets, elixirs, suspensions, ointments containing, for example, up to 10% by weight of the compound, soft and hard gelatin capsules, suppositories, injection solutions and suspensions and sterile packaged powders.

Some examples of suitable carriers are lactose, dextrose, sucrose, sorbitol, mannitol, starches, gum acacia, calcium phosphate, alginates, tragacanth, gelatin, syrup, methyl cellulose, methyl- and propyl- hydrobenzoate, talc magnesium stearate and mineral oil. The compositions of the injection may, as is well known in the art, be formulated so as to provide quick, sustained or delayed release of the active ingredient after administration to the patient.

Where the compositions are formulated in unit dosage form, it is preferred that each unit dosage form contains from 5 mg to 500 mg. The term 'unit dosage form' refers to physically discrete units suitable as unit dosages for human subjects and animals, each unit containing a predetermined quantity of active material calculated to produce the desired therapeutic effect, in association with the required pharmaceutical carrier.

The active compound is effective over a wide dosage range and, for example, dosages per day will normally fall within the range of from 0.5 to 300 mg/kg, more usually in the range of from 5 to 100 mg/kg. However, it will be understood that the amount administered will be determined by the physician in the light of the relevant circumstances including the conditions to be treated, the choice of compound to be administered and the chosen route of administration, and therefore the above dosage ranges are not intended to limit the scope of the invention in any way.

The invention is illustrated by the following Preparations and Examples.

### PREPARATION 1

### Preparation of 4-cyano-5-methyl-4-phenylhexanal

### a) 3-Methyl-2-phenylbutanenitrile

To a suspension of washed sodium hydride (60% in mineral oil) (47.0g, 1.18mol) in dry THF (660mL) was added dropwise phenylacetonitrile (137.8g, 1.18mol) in THF (190mL). The reaction mixture was heated to 40°C when an exotherm occurred with the internal temperature rising to 60°C. The heat source was removed and the reaction cooled to 40°C. The temperature was maintained at 40°C for a further 30 minutes when hydrogen evolution was complete. The reaction was cooled to room temperature and a solution of 2-bromopropane (144.6g, 1.18mol) in THF (230mL) was added dropwise, maintaining the internal temperature below 30°C. The reaction was stirred at room temperature overnight before being poured into ice/water and extracted into diethyl ether. The combined organic extracts were washed with brine, dried (MgSO₄), filtered and evaporated *in vacuo.*

### b) 4-Cyano-5-methyl-4-phenylhexanal

To a suspension of washed sodium hydride (50% in mineral oil) (14.4g, 0.30mol) in dry THF (200mL) was added 3-methyl-2-phenylbutanenitrile (40.0g, 0.25mol) in THF (100mL) over 10 minutes. The reaction mixture was heated to 60°C and maintained at that temperature until hydrogen evolution ceased. The reaction mixture was cooled to room temperature and 3-chloropropionaldehyde diethyl acetal (50g, 0.30mol) added dropwise. The reaction was heated at 60°C until complete by GC, then cooled, poured into ice/water and extracted into diethyl ether. The combined organic extracts were washed with brine, dried (MgSO₄), filtered and evaporated *in vacuo* to a brown oil.

The crude acetal was dissolved in acetone (200mL), and oxalic acid (24.9g, 0.28mol) in water (250mL) added. The reaction mixture was heated at reflux for 60 minutes, cooled to room temperature and neutralised by addition of solid potassium carbonate. Solids were filtered off and the filtrate concentrated *in vacuo.* The product was extracted from water into diethyl ether and washed with brine. The combined organic extracts were dried (MgSO₄), filtered and evaporated. The resultant oil was purified by vacuum distillation (B.Pt. 102°C at 0.6mBar).

The following aldehydes were prepared by the same method, substituting 2-bromopropane with the appropriate alkyl halide:
4-Cyano-4-phenylpentanal
4-Cyano-4-phenylhexanal
4-Cyano-6-methyl-4-phenylheptanal
4-Cyano-5-ethyl-4-phenylheptanal

The following aldehydes were also prepared by the same method, substituting phenylacetonitrile with the appropriate substituted phenylacetonitrile or heteroarylacetonitrile:
4-Cyano-5-methyl-4-(3-fluorophenyl)hexanal
4-Cyano-5-methyl-4-(3-methoxyphenyl)hexanal
4-Cyano-5-methyl-4-(4-chlorophenyl)hexanal
4-Cyano-5-methyl-4-(4-fluorophenyl)hexanal
4-Cyano-5-methyl-4-(4-methoxyphenyl)hexanal
4-Cyano-5-methyl-4-(4-ethylphenyl)hexanal
4-Cyano-5-methyl-4-(2-pyridyl)hexanal
4-Cyano-5-methyl-4-(3-pyridyl)hexanal

### PREPARATION 2

### Preparation of (S)-4-cyano-5-methyl-4-phenylhexanal

### a) 2-(S)-((R)-2,2-Dimethyl-1,3-dioxolan-4-ylmethyl)-3-methyl-2-phenylbutanenitrile.

A 2.0 M solution of lithium diisopropylamide in THF (34.8mL, 69.8mmol) was added dropwise to a stirred solution of 3-methyl-2-phenylbutanenitrile (11.1g, 69.8mmol) and 1,3- dimethylimidazolidin-2-one (7.98g, 69.8mmol) in THF (100mL) at -70ºC under nitrogen. The resulting dark mixture was stirred at -60ºC for 1h. and treated dropwise with a solution of (*R*)-1-(2,2-dimethyl-1,3-dioxolan-4-yl)methyl 4-methyl-benzenesulfonate (20.0g, 69.8mmol) in dry THF (15mL). The mixture was allowed to warm gradually to room temperature over *ca* 2h. and stirred at this temperature for 15h. The reaction mixture was quenched with brine, extracted into diethyl ether (3x), and the combined organic extracts were washed with water (3x), brine and dried (MgSO₄). Concentration under reduced pressure gave the crude product as a yellow oil. Flash chromatography on silica, eluting with ethyl acetate-hexane (1:9), gave the required dioxolane as the faster running diastereomer - the colourless oil crystallised on standing.

### b) (S)-3-Cyano-4-methyl-3-phenylpentanal

A solution of 2-(*S*)-((*R*)-2,2-dimethyl-1,3-dioxolan-4-ylmethyl)-3-methyl-2-phenyl-butanenitrile (15.7g, 57 mmol) in glacial acetic acid (270mL) and water (90mL) was stirred for 18 h at room temperature. The reaction mixture was concentrated under reduced pressure to give the crude diol as a colourless oil. This was taken up in dichloromethane (750mL) to which was then added an aqueous solution (380mL) of sodium bicarbonate (29.1g, 0.35mol). To the stirred biphasic system was added dropwise a solution of sodium periodate (49.5g, 0.23mol) in water (250mL) over 2 h. After addition was complete, the suspension was stirred for a further 2 h at room temperature when GC confirmed the reaction to be complete. The organic phase was separated, washed with water, dried, filtered and concentrated under reduced pressure. The residue was purified by flash chromatography on silica, eluting with diethyl ether-hexane (0-1:4), to yield (*S*)-3-cyano-4-methyl-3-phenylpentanal as a colourless oil, B.Pt. 106ºC at 0.5 mBar.

### c) (S)-4-Cyano-5-methyl-4-phenylhexanal

To a stirred suspension of powdered methoxymethyltriphenylphosphonium chloride (33.2g, 96.8mmol) in dry THF (500mL) at -78ºC under nitrogen was added *n*-butyllithium (1.6M in THF) (54.7mL, 87.5mmol) over 10 min. The orange suspension was allowed to warm to 0ºC and stirred for 1 h. The resultant deep red solution was recooled to -78ºC and (*S*)-3-cyano-4-methyl-3-phenylpentanal (9.75g, 48.4mmol) in dry THF (50mL) was added dropwise over 10 min. The reaction mixture was stirred at -78ºC for 45 min, allowed to warm to RT and quenched with water. The product was extracted into diethyl ether, the organic extracts washed with water, then brine, dried, filtered and concentrated under reduced pressure. The residue was purified by flash chromatography on silica, eluting with diethyl ether-hexane (0-3:20), to yield the intermediate 5-methoxy-2-(1-methylethyl)-2-phenylpent-4-enenitrile as a mixture of geometric isomers (Z/E : 3/2 by GC).

This was immediately hydrolysed by dissolving in 2-propanol (60mL) and water (60mL) containing 4-methylbenzenesulfonic acid (0.24 g) and heating under reflux for 3 h. On cooling, the reaction mixture was diluted with water and extracted into diethyl ether. The combined organic extracts were washed with aqueous sodium bicarbonate, then brine, dried, filtered and concentrated under reduced pressure. The crude product was purified by flash chromatography on silica, eluting with diethyl ether-hexane (0-1:4), to yield (*S*)-4-cyano-5-methyl-4-phenylhexanal as a colourless oil, B.Pt. 89-91°C at 0.07 mBar.

### PREPARATION 3

### Preparation of 4-methoxy-5-methyl-4-phenylhexanal

### a) 2-Methyl-3-phenylhex-5-en-3-ol.

Allylmagnesium chloride (2M in THF, 22mL, 44mmol) was added dropwise to a solution of isobutyrophenone (5.92g, 40mmol) in dry THF (50mL). The mixture was stirred for 30min, excess 10% aqueous ammonium chloride added dropwise and the solution extracted with ethyl acetate (3x50mL). The combined organic extracts were washed with brine and dried (MgSO₄), and evaporated under reduced pressure. The crude oil was distilled in a short-path distillation apparatus to give 2-methyl-3-phenylhex-5-en-3-ol.

### b) 4-Methoxy-5-methyl-4-phenylhex-1-ene

A solution of 2-methyl-3-phenylhex-5-en-3-ol (3.8g, 20mmol) in dry dimethylformamide (25mL) was added dropwise to a stirred suspension of sodium hydride (50% oil dispersion) (1.25g, 26mmol) in dry DMF (15mL). The mixture was stirred for 30min then iodomethane (1.9mL, 30mmol) was added. The mixture was stirred at ambient temperature for 20h, poured onto ice and extracted with ethyl acetate (3x50mL). The combined extracts were washed with water, dried (MgSO₄) and the solvent removed under reduced pressure. The crude product was purified by flash chromatography on silica, eluting with 5% ethyl acetate in hexane.

### c) 4-Methoxy-5-methyl-4-phenylhexan-1-ol

To a stirred ice-cooled solution of 4-methoxy-5-methyl-4-phenylhex-1-ene (2.0g, 9.8mmol) in dry THF (50mL) under nitrogen was added dropwise borane (1M in THF, 6.6mL). The icebath was removed and stirring continued at room temperature for 1.25h. The solution was recooled in an icebath and 2M sodium hydroxide (9mL) added cautiously. The clear solution was stirred for a further 0.25h before the dropwise addition of 30% hydrogen peroxide (5mL). After a further 0.5h stirring at icebath temperature, the solution was diluted with water (20mL), the layers separated and the aqueous phase extracted with ethyl acetate. The combined organic extracts were washed with water, dried (MgSO₄), filtered and concentrated to yield a colourless oil (2.2g). This was purified by flash chromatography on silica, eluting with ethyl acetate-hexane (3:7), providing the alcohol as a colourless oil.

### d) 4-Methoxy-5-methyl-4-phenylhexanal

A solution of 4-methoxy-5-methyl-4-phenylhexan-1-ol (1.35g, 6.1mmol) in dry dichloromethane (25mL) was added dropwise to a stirred suspension of pyridinium chlorochromate (2.6g, 12mmol) in dry dichloromethane (25mL) at 0-5°C. The mixture was stirred at ambient temperature for 2h, then diluted with diethyl ether (50mL). The mixture was filtered through a short column of silica, washing with diethyl ether. The residue, after removal of solvent, was purified by flash chromatography on silica, eluting with 10-20% ethyl acetate in hexane to give the title compound as a colourless oil.

### PREPARATION 4

### Preparation of 4-cyano-4-(1-methylethylsulfonyl)-4-phenylbutanal

### a) (1-Methylethylsulfonyl)phenylacetonitrile

A mixture of iodobenzene (13.9g, 68mmol), (1-methylethylsulfonyl)acetonitrile (20.0g, 136mmol), copper (I)iodide (1.29g, 6.8mmol) and potassium carbonate (37.6g, 272mmol) in dimethylsulfoxide (100mL) was stirred at 120 °C under an atmosphere of nitrogen for 40h. The dark blue mixture was allowed to cool to room temperature, poured into 2M hydrochloric acid (300mL) and extracted with ether (200mL) and ethyl acetate (2 x 150mL). The combined organic extracts were washed with water (8 x 125mL), dried (MgSO₄), filtered and evaporated *in vacuo* to yield a black oil. This was dissolved in dichloromethane and filtered through a large pad of "flash" silica, eluting with dichloromethane. Fractions containing the product were combined and concentrated, yielding a yellow oil. This was distilled in a short-path distillation apparatus, providing a yellow oil (B.Pt. 230 °C at 0.3 mBar).

### b) 4-Cyano-4-(1-methylethylsulfonyl)-4-phenylbutanal

To a solution of sodium (0.46g, 20mmol) in anhydrous ethanol (40mL) under nitrogen was added a solution of (1-methylethylsulfonyl)phenylacetonitrile (2.23g, 10mmol) in ethanol (5mL), After stirring at room temperature for 0.5h, a solution of 3-chloropropionaldehyde diethyl acetal (3.33g, 20mmol) in ethanol (5mL) was added, followed by anhydrous sodium iodide (3.0g, 20mmol). The solution was heated at reflux for 168h. The brown mixture was allowed to cool, poured into saturated aqueous ammonium chloride and extracted with ethyl acetate (3x). The combined organic extracts were washed with water (2x) and concentrated to an oil. This was taken up in acetone (30mL). A solution of oxalic acid (3.15g, 35mmol) in water (30mL) was added and the resulting solution heated at reflux for 1h. After cooling, the acetone was removed *in vacuo* and the aqueous residue extracted with ethyl acetate (3x) . The combined organic extracts were washed with water, dried (MgSO₄), filtered and concentrated. The residue was purified by flash chromatography on silica, eluting with ethyl acetate/hexane, to yield the aldehyde as a pale yellow oil.

### PREPARATION 5

### Preparation of 4-(diethylphosphonyl)-4-(4-fluorophenyl)-4-methoxybutanal

### a) 4-(Diethylphosphonyl)-4-(4-fluorophenyl)-4-methoxybut-1-ene

To a stirred solution of diethyl (4-fluoro-1'-methoxy)benzylphosphonate (6.0g, 20mmol) in dry THF (50mL) at -78 °C under nitrogen was added dropwise *n*-butyllithium (1.6M in hexane, 15mL, 24mmol). The resulting bright yellow solution slowly deposited a copious precipitate. After stirring for 1h at -78 °C, a solution of allyl bromide (2.9g, 24mmol) in THF (5mL) was added. Following further stirring at -78 °C (2h), the colourless solution was allowed to warm to room temperature. It was then poured into saturated aqueous ammonium chloride solution and the organic layer separated. The aqueous phase was extracted with ethyl acetate (2x), the combined organic extracts washed with water, dried (MgSO₄), filtered and concentrated to yield an oil. Chromatography on silica gel, with ethyl acetate as eluant, provided a pale yellow oil, which was distilled in a short-path distillation apparatus to provide a yellow oil (B.Pt. 205 °C at 0.3 mBar).

### b) 4-(Diethylphosphonyl)-4-(4-fluorophenyl)-4-methoxybutan-1-ol

To a stirred ice-cooled solution of 4-(diethylphosphonyl)-4-(4-fluorophenyl)-4-methoxybut-1-ene (1.58g, 5mmol) in dry THF (30mL) under nitrogen was added dropwise a solution of borane (1M in THF, 3.3mL, 3.3mmol). The clear, colourless solution was stirred at icebath temperature for 2h and then at room temperature for 2h. The solution was recooled in an icebath and aqueous sodium hydroxide (2M, 4mL) added dropwise followed after 0.25h by 30% hydrogen peroxide. The cloudy mixture was stirred for 0.5h and then diluted with water (10mL). The layers were separated and the aqueous phase was extracted with ethyl acetate (2x). The combined organic extracts washed with water, dried (MgSO₄), filtered and concentrated to yield an oil. This was purified by flash chromatography on silica, eluting with ethyl acetate, to provide the product as a colourless oil.

### c) 4-(Diethylphosphonyl)-4-(4-fluorophenyl)-4-methoxybutanal

To a stirred ice-cooled solution of pyridinium chlorochromate (1.17g) in dichloromethane (10mL) was added dropwise a solution of 4-(diethylphosphonyl)-4-(4-fluorophenyl)-4-methoxybutan-1-ol (911mg, 2.7mmol) in dichloromethane (3mL). After 0.5h, the icebath was removed and stirring continued at room temperature for a further 3h. Another portion of pyridinium chlorochromate (0.5g) was added and the mixture stirred for an additional 1h at room temperature. The solution was washed with 2M hydrochloric acid then water, dried over MgSO₄, filtered and concentrated to a brown oil. Chromatography on silica eluting with ethyl acetate provided the aldehyde as a pale yellow oil.

### PREPARATION 6

### Preparation of 4-cyano-5-methyl-4-phenylhexanoic acid

To a solution of 4-cyano-5-methyl-4-phenylhexanal (5.0g, 23.3mmol) in DMSO (24mL) was added a 10% aqueous solution of sodium chlorite (2.6g, 33mmol) dropwise over 2h. Stirring was continued for a further 15h when water (100mL) was added and the reaction product extracted into ethyl acetate. The combined organic extracts were washed with water, then brine, dried (MgSO₄) and evaporated *in vacuo.* The resultant oil was partitioned between diethyl ether and 2N sodium hydroxide, and the separated aqueous layer acidified with 2N hydrochloric acid. The acid was extracted into diethyl ether, washed with water, dried (MgSO₄) and evaporated *in vacuo* to an off-white solid.

### PREPARATION 7

### Preparation of 6-cyano-7-methyl-6-phenyloctanoic acid

### Method A:

### a) Ethyl 6-cyano-7-methyl-6-phenyloct-2-enoate

To a solution of triethylphosphonoacetate (1.0g, 4.5mmol) in dry THF (45mL) at -78°C was added sodium hydride (60% dispersion in mineral oil) (0.2g, 5,12mmol) . The reaction mixture was stirred for 45 minutes maintaining the temperature below -40°C. The reaction was cooled to -78°C and a solution of 4-cyano-5-methyl-4-phenylhexanal (1g, 4.65mmol) in THF (5ml) was added. The reaction was allowed to warm to room temperature and stirred overnight before being poured into water and extracted into ethyl acetate. The combined organic layers were washed with brine, dried (MgSO₄), filtered and evaporated *in vacuo.*

### b) Ethyl 6-cyano-7-methyl-6-phenyloctanoate

To a solution of ethyl 6-cyano-7-methyl-6-phenyloct-2-enoate (0.67g, 2.35mmol) in methanol (20mL) was added palladium (10% on charcoal) (0.17g). The reaction was shaken under hydrogen (60 p.s.i.) for 1 hour, filtered through Celite and evaporated *in vacuo* .

### c) 6-Cyano-7-methyl-6-phenyloctanoic acid

To a solution of ethyl 6-cyano-7-methyl-6-phenyloctanoate (1,3g, 4.7 mmol) in dioxane (2mL) was added potassium hydroxide (0.62g, 9.4mmol) in water (6mL) and heated under reflux for 7 hours. The reaction mixture was diluted with ethyl acetate, washed with 2N HCl solution, dried (MgSO₄), filtered and evaporated *in vacuo.*

### Method B:

To sodium hydride (1.6g 50% oil dispersion, 30mmol) in dry DMF (50mL) was added 3-methyl-2-phenylbutanenitrile (3.18g, 20mmol), followed by methyl 5-bromovalerate (4.4g, 20mmol). The mixture was stirred under nitrogen at 60°C for 1 hour, poured onto ice and extracted with ethyl acetate (3X 50mL). The combined extracts were washed with water, and dried over anhydrous magnesium sulphate. The solvent was removed under reduced pressure to leave the crude ester which was purified by flash chromatography on silica gel, eluting with 10-100% ethyl acetate in hexane to give the pure ester as a colourless oil. This ester was hydrolysed to the acid by adding a solution in ether (25mL) to a stirred suspension of potassium tert-butoxide (9g, 80mmol) in ether (50mL) under a nitrogen atmosphere. To the stirred mixture was added 2 drops of water and stirring was continued for 20 hours. The mixture was poured onto ice-water, extracted with ethyl acetate (3x50mL) and the extracts dried (MgSO₄). The crude acid was purified by flash chromatography on silica gel, eluting with 2:3 ethyl acetate: dichloromethane.

### PREPARATION 8

### Preparation of 1-chloro-4-cyano-5-methyl-4-phenylhexane

A mixture of 3-methyl-2-phenylbutanenitrile (37.6g, 0.24mol), 1-bromo-3-chloropropane (71.6g, 0.46mol) and tetrabutylammonium chloride (2.3g) in THF (540mL) was stirred as solid potassium hydroxide (91.1g, 1.62mol) was added in portions. The stirred mixture was heated at reflux under nitrogen for 1h. The mixture was cooled to room temperature and filtered through Celite. The filtrate was washed with water, dried (MgSO₄), filtered and evaporated *in vacuo.* The crude oil was purified by vacuum distillation to yield 1-chloro-4-cyano-5-methyl-4-phenylhexane (B.Pt. 100-104°C at 0.5mBar).

### PREPARATION 9

### Preparation of N'-(t-butyloxycarbonyl) -N-diphenylmethyl-(S)-methioninamide

*N,N*'-Carbonyldiimidazole (1.43g, 8.8mmol). was added to *N*'-(*t*-butyloxy-carbonyl)-(*S*)-methionine (2g, 8.0mmol) in dichloromethane (20ml). After 2 h aminodiphenylmethane (1.6g, 8.8mmol) was added and the reaction mixture stirred overnight. The organic phase was washed successively with 0.1M potassium bisulphate, saturated aqueous sodium bicarbonate and water, then dried (MgSO₄) and evaporated to give an oil. The product was purified by flash chromatography on silica, eluting first with 20% hexane in ethyl acetate, then with ethyl acetate, to give the product as a solid.

The following amino acid amides were similarly prepared from the appropriate *N*-(*t*-butyloxycarbonyl)-protected amino acid:
*N*'-(*t*-Butyloxycarbonyl)-*N*-diphenylmethyl-(*S*)-valinamide
*N*'-(*t*-Butyloxycarbonyl)-*N*-diphenylmethyl-(*R*)-valinamide
*N*'-(*t*-Butyloxycarbonyl)-*N*-diphenylmethyl-(*S*)-leucinamide
*N*'-(*t*-Butyloxycarbonyl)-*N*-diphenylmethyl-(*S*)-isoleucinamide
*N*'-(*t*-Butyloxycarbonyl)-*N*-(diphenylmethyl)-(*S*)-*allo*-isoleucinamide
*N*'-(*t*-Butyloxycarbonyl)-*N*-(diphenylmethyl)-2(*S*)-aminohexanamide
*N*'-(*t*-Butyloxycarbonyl)-*N*-(diphenylmethyl)-*S*-ethyl-(*S*)-cysteinamide
*N*'-(*t*-Butyloxycarbonyl)-*N*-(diphenylmethyl)-(*S*)-alaninamide
*N*'-(*t*-Butyloxycarbonyl)-*N*-(diphenylmethyl)-2 (*S*)-aminobutanamide
*N*'-(*t*-Butyloxycarbonyl)-3,3-dimethyl-*N*-(diphenylmethyl)-2(*S*)-aminobutanamide
*N*'-(*t*-Butyloxycarbonyl)-*N*-(diphenylmethyl)-3-(2-thienyl)-(*S*)-alaninamide
*N*'-(*t*-Butyloxycarbonyl)-*N*-(diphenylmethyl)-*O*-benzy-(*S*)-tyrosinamide

*N*'-(*t*-Butyloxycarbonyl)-*N*-(2,2-diphenylethyl)-glycinamide
*N*'-(*t*-Butyloxycarbonyl)-*N*-(2,2-diphenylethyl)-(*S*)-alaninamide
*N*'-(*t*-Butyloxycarbonyl)-*N*-(2,2-diphenylethyl)-(*S*)-methioninamide
*N*'-(*t*-Butyloxycarbonyl)-*N*-(2,2-diphenylethyl)-*N*-methyl-(*S*)-methioninamide
*N*'-(*t*-Butyloxycarbonyl)-*N*-(2,2-diphenylethyl)-(*S*)-leucinamide
*N*'-(*t*-Butyloxycarbonyl)-*N*-(2,2 -diphenylethyl)- (*S*)-valinamide
*N*'-(*t*-Butyloxycarbonyl)-*N*-(2,2-diphenylethyl)- (*R*)-valinamide
*N*(6), *N*'-Di(*t*-butyloxycarbonyl)-*N*-(2,2-diphenylethyl)-(*S*)-histidinamide
*N*'-(*t*-Butyloxycarbonyl)-*N*(6)-(benzyloxycarbonyl)-*N*-(2,2-diphenylethyl)-(*S*-lysinamide

*N*'-(*t*-Butyloxycarbonyl)-*N*-(3,3-diphenylpropyl)-(*S*)-methioninamide
*N*'-(*t*-Butyloxycarbonyl)-*N*-(3,3-diphenylpropyl)-(*S*)-leucinamide

*N*-Benzyl-*N*'-(*t*-Butyloxycarbonyl)-(*S*)-valinamide
*N*-Benzyl-*N*'-(*t*-Butyloxycarbonyl)-(*S*)-methioninamide
*N*-Benzyl-*N*'-(*t*-Butyloxycarbonyl)-*N*-methyl-(*S*)-methioninamide

*N*'-(*t*-ButyloxycarbonyI)-*N*-[1-(*R*)-phenylethyl]-(*S*)-valinamide
*N*'-(*t*-Butyloxycarbonyl)-*N*-[1-(*R*)-phenylethyl]-(*S*)-methioninamide
*N*'-(*t*-Butyloxycarbonyl)-*N*-[1-(*R*)-phenylethyl]-(*S*)-thiazolidine-4-carboxamide
*N*'-(*t*-Butyloxycarbonyl)-*N*(6)-(2-chlorobenzyloxycarbonyl)-*N*-[1-(*R*)-phenylethyl]-(*R*)-lysinamide
*N*'-Benzyloxycarbonyl-*N*-[1-(*R*)-phenylethyl]-(*S*)-aspartamide, 4-methyl ester

*N*'-(*t*-Butyloxycarbonyl)-*N*- [1-(*S*)-phenyl-1-carbomethoxymethyl)-(*S*)-valinamide
*N*'-(*t*-Butyloxycarbonyl)-*N*-[1-(*R*)-phenyl-1-carbethoxymethyl)-(*S*)-valinamide

*N*'-(*t*-Butyloxycarbonyl)-*N*-(2-phenyl-1-(*S*)-carbethoxyethyl)-(*S*)-valinamide
*N*'-(*t*-ButyloxycarbonyI)-*N*-[1-(*R*)-phenyl-2-hydroxyethyl)-(*S*)-valinamide

*N*'-(*t*-Butyloxycarbonyl)-*N*-[2-phenylethyl]-(*S*)-methioninamide
*N*'-(*t*-Butyloxycarbonyl)-*N*(6)-(benzyloxycarbonyl)-*N*-[2-phenylethyl]-(*S*)-lysinamide
*N*'-(*t*-Butyloxycarbonyl)-*N*(6)-(benzyloxycarbonyl)-*N*-[2-phenylethyl]-(*R*)-lysinamide

### PREPARATION 10

### Preparation of N-diphenylmethyl-(S)-methioninamide

*N*'-(*t*-Butyloxycarbonyl)-*N*-diphenylmethyl-(*S*)-methioninamide (2.1g, 5mmol) was dissolved in dichloromethane (20ml) and trifluoroacetic acid (3ml) was added. After stirring for one day the mixture was evaporated, and water then saturated aqueous sodium bicarbonate solution added and the product extracted into dichloromethane. The organic phase was washed with water, dried (MgSO₄) and evaporated to give a solid. The crude product was purified by flash chromatography on silica, eluting with ethyl acetate, then 20% methanol in ethyl acetate to give the product as a solid.

The following amino acid amides were similarily prepared by deprotection of the appropriate *N*'-(*t*-butyloxycarbonyl) derivative:
*N*-(Diphenylmethyl)-(*S*)-valinamide
*N*-(Diphenylmethyl)-(*R*)-valinamide
*N*-(Diphenylmethyl)-(*S*)-leucinamide
*N*-(Diphenylmethyl)-(*S*)-isoleucinamide
*N*-(Diphenylmethyl)-(*S*)-*allo*-isoleucinamide
*N*-(Diphenylmethyl)-2(*S*)-aminohexanamide
*N*-(Diphenylmethyl)-*S*-ethyl-(*S*)-cysteinamide
*N*-(Diphenylmethyl)-(*S*)-alaninamide
*N*-(Diphenylmethyl)-2(*S*)-aminobutanamide
3,3-Dimethyl-*N*-(diphenylmethyl)-2(*S*)-aminobutanamide
*N*-(Diphenylmethyl)-*O*-benzyl-(*S*)-tyrosinamide
*N*-(Diphenylmethyl)-3-(2-thienyl)-(*S*)-alaninamide
*N*-(2,2-Diphenylethyl)-glycinamide
*N*-(2,2-Diphenylethyl)-(*S*)-alaninamide
*N*-(2,2-Diphenylethyl)-(*S*)-methioninamide
*N*-(2,2-Diphenylethyl)-*N*-methyl-(*S*)-methioninamide *N*-(2,2-Diphenylethyl)-(*S*)-leucinamide
*N*-(2,2-Diphenylethyl)-(*S*)-valinamide
*N*-(2, 2-Diphenylethyl)-(*R*)-valinamide
*N*-(2,2-Diphenylethyl)-(*S*)-histidinamide
*N*(6)-(Benzyloxycarbonyl)-*N*-(2,2-diphenylethyl)-(*S*)-lysinamide

*N*-(3,3-Diphenylpropyl)-(*S*)-methioninamide
*N*-(3,3-Diphenylpropyl)-(*S*)-leucinamide

*N*-Benzyl-(*S*)-valinamide
*N*-Benzyl-(*S*)-methioninamide
*N*-Benzyl-*N*-methyl-(*S*)-methioninamide

*N*-[1-(*R*)-Phenylethyl]-(*S*)-valinamide
*N*-[1-(*R*)-Phenylethyl]-(*S*)-methioninamide
*N*-[1-(*R*)-Phenylethyl]-(*S*)-thiazolidine-4-carboxamide
*N*(6)-2-chlorobenzyloxycarbonyl-*N*-[1-(*R*)-phenylethyl]-(*R*)-lysinamide

*N*-[1-(*S*)-phenyl-1-carbomethoxymethyl)-(*S*)-valinamide
*N*-[1-(*R*)-phenyl-1-carbethoxymethyl)-(*S*)-valinamide
*N*-[1-(*R*)-phenyl-2-hydroxyethyl)-(*S*)-valinamide
*N*-(2-phenyl-1-(*S*)-carbethoxyethyl)-(*S*)-valinamide
*N*-(2-Phenylethyl)-(*S*)-methioninamide
*N*(6)-(Benzyloxycarbonyl)-*N*-[2-phenylethyl]-(*S*)-lysinamide
*N*(6)-(Benzyloxycarbonyl)-*N*-[2-phenylethyl]-(*R*)-lysinamide
*N*-[1-(4-fluorophenyl)-2-methylprop-2-yl]-(*S*)-valinamide

### EXAMPLE 1

### N'-[4(S)-Cyano-5-methyl-4-phenylhexyl]-N-(diphenylmethyl)-(S)-methioninamide, maleate

*N*-Diphenylmethyl-(*S*)-methioninamide (0.64g, 2.05mmol), (*S*)-4-cyano-5-methyl-4-phenylhexanal (0.4g, 1.9mmol) and ethanol (40ml) were stirred for 2 h. Sodium borohydride (0.43g 11.2mmol) was added over two hours then the mixture was left to stir overnight. After evaporation, water and saturated aqueous sodium bicarbonate were added and the product extracted into dichloromethane. The organic extracts were washed with water, dried (MgSO₄) and evaporated to give an oil. The crude product was purified by flash chromatograph on silica, eluting with 40% hexane in ethyl acetate, then ethyl acetate, to give the product as an oil. The amine was converted to the crystalline maleate salt (mp 87-9ºC from ethyl acetate/hexane).

The following products were similarly prepared. Where racemic aldehyde was used in the reductive amination, the diastereomeric products could be separated by HPLC on a Kromasil column (60Å, 5 micron) - the faster eluting fraction is referred to as Isomer 1 and the slower fraction as Isomer 2:
*N*'-[4(*R*)-Cyano-5-methyl-4-phenylhexyl]-*N*-(diphenylmethyl)-(*S*)-methioninamide, maleate (mp 157ºC)
*N*'-[4(*R*)-Cyano-5-methyl-4-phenylhexyl]-*N*-(diphenylmethyl)-(*R*)-methioninamide, maleate (mp 88-93ºC)
*N*'-(4-Cyano-4-phenylhexyl]-*N*-(diphenylmethyl)-(*S*)-methionamide, maleate (Isomer 1) (mp 102-3ºC)
*N*'-(4-Cyano-4-phenylhexyl]-*N*-(diphenylmethyl)-(*S*)-methionamide, fumarate (Isomer 2) (mp 136-8ºC)
*N*'-[4-Cyano-4-(4-fluorophenyl)-5-methylhexyl]-*N*-(diphenylmethyl)-(*S*)-methioninamide, fumarate (Isomer 1) (mp 93-6ºC)
*N*'-[4-Cyano-4-(4-fluorophenyl)-5-methylhexyl]-*N*-(diphenylmethyl)-(*S*)-methioninamide, fumarate (Isomer 2) (mp 82-4ºC)
*N*'-[4-Cyano-4-(4-ethylphenyl)-5-methylhexyl]-*N*-(diphenylmethyl)-(*S*)-methioninamide, fumarate (Isomer 2) (mp 121-3ºC)
*N*'-[4-Cyano-4-(4-methoxyphenyl)-5-methylhexyl]-*N*-(diphenylmethyl)-(*S*)-methioninamide, fumarate (mp 90-2ºC)
*N*'-[4-Cyano-5-methyl-4-(3-pyridyl)hexyl]-*N*-(diphenylmethyl)-(*S*)-methioninamide, fumarate ([M+H]⁺ = 515)
*N*'-(4-Cyano-5-methyl-4-phenylhexyl)-*N*-diphenylmethyl-(*S*)-leucinamide, hydrochloride (Isomer 1) (mp 161-3ºC)
*N*'-(4-Cyano-5-methyl-4-phenylhexyl)-*N*-diphenylmethyl-(*S*)-leucinamide, maleate (Isomer 2) (mp 112-4ºC)
*N*'-(4-Cyano-4-phenylhexyl]-*N*-(diphenylmethyl)-(*S*)-leucinamide, maleate (mp 130ºC)
*N*'-(4-Cyano-4-phenylpentyl)-*N*-diphenylmethyl-(*S*)-leucinamide, maleate (mp 147-8ºC)
*N*'-[4-Cyano-4-(4-fluorophenyl)-5-methylhexyl]-*N*-(diphenylmethyl)-(*S*)-leucinamide, fumarate (Isomer 1) (mp 151-3ºC)
*N*'-[4-Cyano-4-(4-fluorophenyl)-5-methylhexyl]-*N*-(diphenylmethyl)-(*S*)-leucinamide, fumarate (Isomer 2) (mp 184-6ºC)
*N*-(Diphenylmethyl)-*N*'-(4-methoxy-5-methyl-4-phenylhexyl)-(*S*)-leucinamide, maleate (mp 101-2ºC)
*N*'-(4-Cyano-5-methyl-4-phenylhexyl)-*N*-diphenylmethyl-(*S*)-isoleucinamide, maleate (Isomer 1) (mp 90-2ºC)
*N*'-(4-Cyano-5-methyl-4-phenylhexyl)-*N*-diphenylmethyl-(*S*)-isoleucinamide, hydrochloride (Isomer 2) (mp 105-7ºC)
*N*'-(4-Cyano-4-isopropylsulphonyl-4-phenylbutyl)-*N*-diphenylmethyl-(*S*)-isoleucinamide, oxalate (Isomer 1) (mp 95ºC)
*N*'-(4-Cyano-4-isopropylsulphonyl-4-phenylbutyl)-*N*-diphenylmethyl-(*S*)-isoleucinamide, oxalate (Isomer 2) (mp 102ºC)
*N*'-[4(*S*)-Cyano-5-methyl-4-phenylhexyl]-*N*-(diphenylmethyl)-(*S*)-*allo*-isoleucinamide, maleate (mp 90-1ºC)
*N*'-[4(*S*)-Cyano-5-methyl-4-phenylhexyl]-*N*-(diphenylmethyl)-2(*S*)-aminohexanamide, maleate (mp 92-5ºC)
*N*'-(4-Cyano-4-phenylhexyl]-*N*-(diphenylmethyl)-2(*S*)-aminohexanamide, maleate (Isomer 1) (mp 115ºC)
*N*'-(4-Cyano-4-phenylhexyl]-*N*-(diphenylmethyl)-2(*S*)-aminohexanamide, maleate (Isomer 2) (mp 67-9ºC)
*N*'-(4-Cyano-5-methyl-4-phenylhexyl)-*N*-(diphenylmethyl)-(*S*)-valinamide, maleate (Isomer 1) (mp 114-6ºC)
*N*'-(4-Cyano-5-methyl-4-phenylhexyl)-*N*-(diphenylmethyl)-(*S*)-valinamide, maleate (Isomer 2) (mp 175-6ºC)
*N*'-(4-Cyano-5-methyl-4-phenylhexyl)-*N*-(diphenylmethyl)-(*R*)-valinamide, maleate (Isomer 1) (mp 98-101ºC)
*N*'-(4-Cyano-5-methyl-4-phenylhexyl)-*N*-(diphenylmethyl)-(*R*)-valinamide, hydrochloride (Isomer 2) ([M+H]⁺ = 482)
*N*'-(4-Cyano-5-methyl-4-phenylhexyl)-*N*-(diphenylmethyl)-(*S*)-alaninamide, maleate (Isomer 1) (mp 185-8ºC)
*N*'-(4-Cyano-5-methyl-4-phenylhexyl)-*N*-(diphenylmethyl)-(*S*)-alaninamide, maleate (Isomer 2)
*N*'-(4(*S*)-Cyano-5-methyl-4-phenylhexyl)-*N*-(diphenylmethyl)-2(*S*)-aminobutanamide, maleate (mp 89-90ºC)
*N*'-[4(*S*)-Cyano-5-methyl-4-phenylhexyl]-3,3-dimethyl-*N*-(diphenylmethyl)-2(*S*)-aminobutanamide (mp 124-5ºC)
*N*'-[4(*S*)-Cyano-5-methyl-4-phenylhexyl]-*N*-(diphenylmethyl)-3-(2-thienyl)-(*S*)-alaninamide, maleate (mp 85-8ºC)
*N*'-[4(*S*)-Cyano-5-methyl-4-phenylhexyl]-*N*-(diphenylmethyl)-*S*-ethyl-(*S*)-cysteinamide, hydrochloride, maleate (mp 196-200ºC)
*N*'-[4(*S*)-Cyano-5-methyl-4-phenylhexyl]-*N*-(diphenylmethyl)-*O*-benzyl-(*S*)-tyrosinamide

*N*'-(4-(*S*)-Cyano-5-methyl-4-phenylhexyl)-*N*-(2,2-diphenylethyl)glycinamide, fumarate (mp 195-7ºC)
(±)-*N*'-(4-Cyano-4-phenylhexyl)-*N*-(2,2-diphenylethyl)glycinamide, maleate (mp 179-80ºC)
(±)-*N*'-[4-Cyano-5-methyl-4-(4-chlorophenyl)hexyl]-*N*-(2,2-diphenylethyl)glycinamide, maleate (mp 172-4ºC)
(±)-*N*'-[4-Cyano-4-(4-fluorophenyl)-5-methylhexyl]-*N*-(2,2-diphenylethyl)glycinamide, fumarate (mp 188-90ºC)
(±)-*N*'-[4-Cyano-4-(4-methoxyphenyl)-5-methylhexyl]-*N*-(2,2-diphenylethyl)glycinamide, fumarate (mp 146-8ºC)
(±)-*N*'-[4-Cyano-4-(3-methoxyphenyl)-5-methylhexyl] -*N*-(2,2-diphenylethyl)glycinamide, fumarate (mp 156-7ºC)
(±)-*N*'-[4-Cyano-4-(3-fluorophenyl)-5-methylhexyl] -*N*-(2,2-diphenylethyl)glycinamide, fumarate (mp 182-4ºC)
(±)-*N*'-[4-Cyano-5-methyl-4-(2-pyridyl)hexyl]-*N*-(2,2-diphenylethyl)glycinamide, fumarate (mp 198-9ºC)
(±)-*N*'-[4-Diethylphosphono-4-(4-fluorophenyl)-4-methoxybut-yl]-*N*-(2,2-diphenylethyl) glycinamide, maleate (mp 149-51°C)
*N*'-(4-Cyano-5-methyl-4-phenylhexyl)-*N*-(2,2-diphenylethyl)-(*S*)-alaninamide, maleate (Isomer 1) (mp 164-8°C)
*N*'-[4-(*S*)-Cyano-5-methyl-4-phenylhexyl]-*N*-(2, 2-diphenylethyl)-(*S*)-methioninamide, hydrochloride (mp 180-5°C)
*N*'-[4-(*S*)-Cyano-5-methyl-4-phenylhexyl]-*N*-(2, 2-diphenylethyl)-*N*-methyl-(*S*)-methioninamide, hydrochloride
*N*'- [4-Cyano-4-isopropylsulphonyl-4-phenylbutyl]-*N*-(2,2-diphenylethyl)-(*S*)-methioninamide, maleate (Isomer 1) (mp 109-10°C)
*N*'-[4-Cyano-4-isopropylsulphonyl-4-phenylbutyl]-*N*-(2,2-diphenylethyl)-(*S*)-methioninamide, maleate (Isomer 2) (mp 127°C)
*N*'-[4(*S*)-Cyano-5-methyl-4-phenylhexyl]-*N*-(2,2-diphenylethyl)-(*S*)-leucinamide, fumarate (mp 156-7°C)
*N*'-(4-Cyano-4-phenylpentyl)-*N*-(2,2-diphenylethyl)-(*S*)-leucinamide, oxalate (mp 101-5°C)
*N*'-(4-Cyano-4-phenylhexyl)-*N*-(2,2-diphenylethyl)-(*S*)-leucinamide, oxalate (mp 151°C)
*N*'-(4-Cyano-6-methyl-4-phenylheptyl]-*N*-(2,2-diphenylethyl)-(*S*)-leucinamide, maleate (Isomer 1) (mp 171-3°C)
*N*'-(4-Cyano-6-methyl-4-phenylheptyl]-*N*-(2,2-diphenylethyl)-(*S*)-leucinamide, maleate (Isomer 2) (mp 149-50°C)
*N*'-(4-Cyano-5-ethyl-4-phenylheptyl]-*N*-(2,2-diphenylethyl)-(*S*)-leucinamide, maleate (mp 89-90°C)
*N*'-(4-Cyano-5-methyl-4-phenylhexyl)-*N*-(2,2-diphenylethyl)-(*S*)-valinamide, maleate (Isomer 1) (mp 186-8°C)
*N*'-(4-Cyano-5-methyl-4-phenylhexyl)-*N*-(2,2-diphenylethyl)-(*R*)-valinamide, maleate (Isomer 1) (mp 186-8°C)
*N*'-[4-Cyano-5-methyl-4-(4-chlorophenyl)hexyl]-*N*-(2,2-diphenylethyl)-(*S*)-valinamide, maleate (Isomer 1) (mp 190-2°C)
*N*'-[4-Cyano-5-methyl-4-(4-chlorophenyl)hexyl]-*N*-(2,2-diphenylethyl)-(*S*)-valinamide, maleate (Isomer 2) (mp 148-50°C)
*N*'-(4-Cyano-5-methyl-4-phenylhexyl)-*N*-(2,2-diphenylethyl)-(*S*)-histidinamide, dimaleate (Isomer 1) (mp 135-8°C)
*N*'-(4-Cyano-5-methyl-4-phenylhexyl)-*N*-(2,2-diphenylethyl)-(*S*)-histidinamide, dimaleate (Isomer 2) (mp 114-8°C)
*N*(6)-(Benzyloxycarbonyl)-*N*(2)-(4-cyano-5-methyl-4-phenylhexyl)-*N*-(2,2-diphenylethyl)-(*S*)-lysinamide (Isomer 1)
*N*(6)-(Benzyloxycarbonyl)-*N*(2)-(4-cyano-5-methyl-4-phenylhexyl)-*N*-(2,2-diphenylethyl)-(*S*)-lysinamide (Isomer 2)
*N*'-[4-(*S*)-Cyano-5-methyl-4-phenylhexyl]-*N*-(3, 3-diphenylpropyl)-(*S*)-methioninamide, hydrochloride (mp 100-4°C)
*N*'-[4(*S*)-Cyano-5-methyl-4-phenylhexyl]-*N*-(3,3-diphenylpropyl)-(*S*)-leucinamide, fumarate (mp 119-20°C)

*N*-Benzyl-*N*'-[4(*S*)-cyano-5-methyl-4-phenylhexyl]-(*S*)-valinamide, maleate (mp 135-7°C)
*N*-Benzyl-*N*'-[4(*S*)-cyano-5-methyl-4- phenylhexyl]-(*S*)-methioninamide, fumarate (mp 144-5°C)
*N*-Benzyl-*N*'-[4-(*S*)-cyano-5-methyl-4-phenylhexyl]-*N*-methyl-(*S*)-methioninamide, fumarate (mp 131-2°C)

*N*'-[4-(*S*)-Cyano-5-methyl-4-phenylhexyl]-*N*-[1-(*R*)-phenylethyl]-(*S*)-thiazolidine-4-carboxamide, maleate ([M+H]⁺ = 436)
*N*(6)-2-Chlorobenzyloxycarbonyl-*N*(2)-[(4(*S*)-cyano-5-methyl-4-phenylhexyl)-*N*-[1-(*R*)-phenylethyl]-(*R*)-lysinamide
*N*'-(4(*S*)-Cyano-5-methyl-4-phenylhexyl)-*N*-[1-(*S*)-phenylethyl)-(*S*)-valinamide, maleate (mp 160-2°C)
*N*'-[4(*S*)-Cyano-5-methyl-4-phenylhexyl]-*N*-[1-(*R*)-phenylethyl]-(*S*)-valinamide, maleate (mp 158-60°C)
*N*'-[4-(*S*)-Cyano-5-methyl-4-phenylhexyl]-*N*-[1-(*R*)-phenylethyl]-(*S*)-methioninamide, maleate (mp 55-7°C)
*N*'-[4(*S*)-Cyano-5-methyl-4-phenylhexyl]-*N*-[1(*S*)-phenyl-1-carbomethoxymethyl]-(*S*)-valinamide, maleate (mp 153-5°C)
*N*'-(4-(*S*)-Cyano-5-methyl-4-phenylhexyl)-*N*-[1-(*R*)-phenyl-1-carboethoxymethyl)-(*S*)-valinamide, maleate (mp 104-6°C)
*N*'-(4(*S*)-cyano-5-methyl-4-phenylhexyl)-*N*-[1-(*R*)-phenyl-2-hydroxyethyl)-(*S*)-valinamide, maleate (mp 128-30°C)
*N*'-[4-(*S*)-Cyano-5-methyl-4-phenylhexyl]-*N*-(2-phenylethyl)-(*S*)-methioninamide, maleate (mp 114-5°C)
*N*'-[4-Cyano-5-methyl-4-(2-pyridyl)hexyl]-*N*-(2-phenylethyl)-(*S*)-methioninamide, fumarate (mp 120-122°C)
*N*'-(4-Cyano-4-isopropylsulphonyl-4-phenyl-butyl)-*N*-(2-phenylethyl)-(*S*)-methioninamide, hydrochloride (Isomer 1), maleate (mp 70-2°C)
*N*'-(4-Cyano-4-isopropylsulphonyl-4-phenyl-butyl)-*N*-(2-phenylethyl)-(*S*)-methioninamide, hydrochloride (Isomer 2), maleate (mp 76-9°C)
*N*(6)-(Benzyloxycarbonyl)-*N*(2)-[4(*S*)-cyano-5-methyl-4-phenylhexyl)-*N*-[2-phenylethyl]-(*S*)-lysinamide
*N*(6)-(Benzyloxycarbonyl)-*N*(2)-[4(*S*)-cyano-5-methyl-4-phenylhexyl)-*N*-[2-phenylethyl]-(*R*)-lysinamide
*N*'-[4(*S*)-Cyano-5-methyl-4-phenylhexyl]-*N*-[1-(4-fluorophenyl)-2-methylprop-2-yl]-(*S*)-valinamide, maleate (mp 120-2°C)

### EXAMPLE 2

### N'-[4(S)-Cyano-5-methyl-4-phenylhexyl]-N-(diphenymethyl]-(S)-tyrosinamide, maleate

*N*'-[4(*S*)-cyano-5-methyl-4-phenylhexyl]-*N*-(diphenylmethyl)-*O*-benzyl-(*S*)-tyrosinamide (0.28g) was hydrogenated at 60 p.s.i. in acetic acid (20ml) in the presence of 10% palladium/charcoal (50mg) for 24h. The catalyst was filtered off and the filtrate evaporated to give an oil, which was converted to the maleate salt (EtOH/ether) ([M+H]⁺ = 546).

### EXAMPLE 3

### N(2)-[(4(S)-Cyano-5-methyl-4-phenylhexyl)-N-[1-(R)-phenylethyl]-(R)-lysinamide,dihydrochloride

*N*(6)-2-Chlorobenzyloxycarbonyl-N(2)-[(4(S)-cyano-5-methyl-4-phenylhexyl)-N-[1-(R)-phenylethyl]-(R)-lysinamide (0.23g) was hydrogenated at 60 p.s.i. in ethanol (20ml) in the presence of 10% palladium/charcoal (50mg) using a Parr hydrogenator. After three hours, the cataylst was filtered off and the filtrate evaporated to give an oil, which was converted to the hydrochloride salt (mp 122-5°C from EtOH/ether).

### EXAMPLE 4

### N(2)-(4-Cyano-5-methyl-4-phenylhexyl)-N-(2,2-diphenylethyl)-(S)-lysinamide, dihydrochloride (Isomer 2)

*N*(6)-(Benzyloxycarbonyl)-*N*(2)-(4-cyano-5-methyl-4-phenylhexyl)-*N*-(2,2-diphenylethyl)-(*S*)-lysinamide (0.32g) was hydrogenated at 60 p.s.i. in ethanol (20ml) in the presence of 10% palladium/charcoal (50mg) for 2 h. The catalyst was filtered off and the filtrate evaporated to give an oil. The oil was purified by chromatography on silica, eluting with increasing amounts of 0.88 ammonia (1, 2, 5, 10%) in methanol. The product was crystallised from ethanolic hydrogen chloride and ether as the hydrochloride salt (mp 200-206° C).

The following examples were similarily prepared:
*N*(2)-(4-Cyano-5-methyl-4-phenylhexyl)-*N*-(2,2-diphenylethyl)-(*S*)-lysinamide, dihydrochloride (Isomer 1) (mp 234-235°C)
*N*(2)-[4(*S*)-Cyano-5-methyl-4-phenylhexyl)-*N*-[2-phenylethyl]-(*R*)-lysinamide, dihydrochloride (mp 215-7°C)
*N*(2)-[4(*S*)-Cyano-5-methyl-4-phenylhexyl)-*N*-[2-phenylethyl]-(*S*)-lysinamide, dihydrochloride ([M+H]⁺ = 449)

### EXAMPLE 5

### N'-[4(S)-Cyano-5-methyl-4-phenylhexyl]-N-[1-(R)-phenylethyl]-(S)-aspartamide, 4-methyl ester, maleate

A mixture of (*S*)-4-cyano-5-methyl-4-phenylhexanal (0.1g, 0.47 mmol), *N*'-benzyloxycarbonyl-*N*-[1-(*R*)-phenylethyl]-(*S*)-aspartamide, 4-methyl ester (0.18g, 0.47mmol), 10% palladium on charcoal (20mg) and ethanol (30 ml) was hydrogenated at 60 p.s.i. for 1 day. The catalyst was filtered off and the filtrate evaporated to dryness to give an oil. This was purified by flash chromatography on silica, eluting with 1:1 hexane:ethyl acetate, then ethyl acetate to give the product as an oil. This was converted to the maleate salt (EtOH, ether) ([M+H]⁺ = 450)

### EXAMPLE 6

### N'-(4(S)-Cyano-5-methyl-4-phenylhexyl)-N-[1(S)-phenyl-1-carbamoylmethyl)-(S)-valinamide

### a) N-Benzyloxycarbonyl-(S)-phenylglycinamide

To a solution of *N*-benzyloxycarbonyl-(*S*)-phenylglycine (1.0g, 3.5mmol) in dry 1,2-dimethoxyethane (3.5ml) at -15 °C was added *N*-methylmorpholine (0.39ml), followed by isobutyl chloroformate (0.48mL). The reaction was stirred for 15 minutes, maintaining the temperature at -15 °C. The solution is filtered and washed with 1,2-dimethoxyethane (2x5mL). The filtrate was cooled to -15 °C, concentrated ammonia (1mL) added and the solution stirred at room temperature overnight. The reaction mixture was diluted with ethyl acetate, washed with aqueous sodium bicarbonate, then brine, dried (MgSO₄), filtered and evaporated *in vacuo.* The product was purified by flash chromatography on silica, eluting with ethyl acetate/hexane/chloroform (7:3:1).

### b) (S)-Phenylglycinamide

To a solution of *N*-benzyloxycarbonyl-(*S*)-phenylglycinamide (0.83g, 2.92mmol) in methanol (10mL) was added palladium (10% on charcoal) (0.2g). The reaction mixture was shaken under hydrogen (60 p.s.i.) for 1 hour. The reaction mixture was filtered through Celite and evaporated *in vacuo.*

### c) N'-Benzyloxycarbonyl-N-[1-(S)-phenyl-1-carbamoylmethyl)-(S)-valinamide

To a solution of *N*-benzyloxycarbonyl-(*S*)-valine (0.55g, 2.2mmol) in dry dichloromethane (10mL) was added 1,1-carbonyldiimidazole (0.39g, 2.42 mmol). The reaction was stirred for two hours at room temperature before adding (*S*)-phenylglycinamide (0.33g, 2.2mmol) in dichloromethane (2mL). Stirring was continued overnight at room temperature. The reaction mixture was diluted with ethyl acetate, washed with aqueous sodium carbonate, then brine, and finally aqueous citric acid, dried(MgSO₄), filtered and evaporated *in vacuo.*

### d) N'-(4-(S)-Cyano-5-methyl-4-phenylhexyl)-N-[1-(S)-phenyl-1-carbamoylmethyl)-(S)-valinamide

To a solution of *N*'-benzyloxycarbonyl-*N*-[1-(*S*)-phenyl-1-carbamoylmethyl]-(*S*)-valinamide (0.17g, 0.4mmol) and 4-(*S*)-cyano-5-methyl-4-phenylhexanal (0.1g, 0.44mmol) in methanol (12mL) was added palladium (10% on charcoal) (0.05g). The reaction mixture was shaken overnight under hydrogen (60 p.s.i), then filtered through Celite and evaporated *in vacuo.* (mp 85-90°C)

### EXAMPLE 7

### N'-[4-(S)-Cyano-5-methyl-4-phenylhexyl]-N-[1-(S)-hydroxy-3-phenylprop-2-yl]-(S)-valinamide, maleate

A solution of *N*-(2-phenyl-1-(*S*)-carbethoxyethyl)-(*S*)-valinamide (0.15g) and 4-(*S*)-cyano-5-methyl-4-phenylhexanal (0.1g, 0.44mmol) in ethanol (10ml) was stirred at room temperature for 2h, then sodium borohydride (0.10g) added The reaction mixture was stirred for 18h, quenched by pouring into water and extracted into ethyl acetate. The combined organic extracts were washed with water, dried (MgSO₄), filtered and evaporated *in vacuo.* The crude amine was purified by flash chromatography on silica, eluting with ethyl acetate. The product was crystallised as its maleate salt from ethyl acetate / hexane (mp 90-2°C).

### EXAMPLE 8

### N'-(4-Cyano-5-methyl-4-phenylhexyl)-N-(diphenylmethyl)-(R)-prolinamide

To a solution of *N*-(diphenylmethyl)-(*R*)-prolinamide (1.2g, 4.2mmol) in dry dimethylformamide (20ml) were added 4-cyano-5-methyl-4-phenylhexyl chloride (0.99g, 4.2mmol), potassium carbonate (0.89g, 6.43 mmol) and a catalytic amount of potassium iodide. The reaction mixture was stirred at 60°C for 48 hours, diluted with ethyl acetate andwashed with brine. The organic extracts were dried (MgSO₄), filtered and evaporated *in vacuo.* The crude product was purified by flash chromatography on silica gel, eluting with 1:1 ethyl acetate / hexane (Yield 1.6g). The product was further purified by HPLC on silica, eluting with 80% hexane / 2% ethanol in dichloromethane to give Isomer 1 and Isomer 2. The two diastereomers were crystallised as their maleate salts from ethanol/ether (mp 160-4°C and 120-30°C respectively).

*N*'-(4-Cyano-5-methyl-4-phenylhexyl)-*N*-(diphenylmethyl)-(*S*)-prolinamide (mp 195-7°C), maleate was prepared by the same method.

### EXAMPLE 9

### N-[4-Cyano-5-methyl-4-phenylhexanoyl)-N'-(1-(R)-phenyethyl)-(R)-prolinamide

To a solution of 4-cyano-5-methyl-4-phenylhexanoic acid (0,1g, 0.43mmol) in dry dichloromethane (3mL) at 0 °C was added 2-chloro-1-methylpyridinium iodide (0.12g, 0.476mmol) and triethylamine (0.12mL, 0.86mmol). The reaction mixture was stirred at room temperature for 40 minutes before *N*-(1-(*R*)-phenylethyl)-(*R*)-prolinamide (0.14g, 0.43mmol) was added in dichloromethane (1mL). The reaction mixture was stirred at room temperature overnight, then diluted with ethyl acetate. The organic extract was washed with aqueous sodium carbonate, brine, and aqueous citric acid, dried (MgSO₄), filtered and evaporated *in vacuo.* The product was purified by flash chromatography on silica, eluting with ethyl acetate. ([M+H]⁺ = 432)

### EXAMPLE 10

### (S,S)-5-[2-Aza-6-cyano-7-methyl-1-(2-methylthioethyl)-6-phenyloctyl]-1-benzyl-1H-tetrazole, oxalate

### a) (S)-5-(1-t-Butyloxycarbonylamino-3-methylthiopropyl)-1-benzyl-1H-tetrazole.

To a solution of *N*'-(*t*-butyloxycarbonyl)-*N*-benzyl-(*S*)-methioninamide (1.2g, 3.6mmol) in THF (30mL) was added diethyl azodicarboxylate (0.61mL, 3.9mmol), triphenylphosphine (1.02g, 3.9mmol) and trimethylsilyl azide (0.52mL, 3.9mmol), and the reaction mixture stirred at room temperature for 24h. A further equivalent of diethyl azodicarboxylate, triphenylphosphine and trimethylsilyl azide was added and stirred for an additional 24h. The reaction mixture was evaporated *in vacuo* and purified by flash chromatography on silica, eluting with ethyl acetate in hexane (1:5 to 2:5).

### b) (S)-5-(1-Amino-3-methylthiopropyl)-1-benzyl-1H-tetrazole.

(*S*)-5-(1-*t*-Butyloxycarbonylamino-3-methylthiopropyl)-1-phenylmethyl-1*H*-tetrazole (0.3g, 0.83mmol) was dissolved in dichloromethane (10mL) and stirred as trifluoroacetic acid (5mL) was added. The reaction mixture was stirred overnight, then evaporated in vacuo. The crude product was taken up in ethyl acetate, washed with aqueous sodium carbonate, dried filtered and evaporated.

### c) (S,S)-5-[2-Aza-6-cyano-7-methyl-1-(2-methylthioethyl)-6-phenyloctyl]-1-benzyl-1H-tetrazole, oxalate.

A solution of (*S*)-5-(1-Amino-3-methylthiopropyl)-1-benzyl-1*H*-tetrazole (0.20g, 0.76mmol) and (*S*)-4-cyano-5-methyl-4-phenylhexanal (0.15g, 0.70mmol) in methanol (5mL) was stirred for 2h, then sodium borohydride (27mg) added over 2 min. After stirring for 1h, the reaction mixture was diluted with ethyl acetate and washed with water, then brine. The organic extracts were dried (MgSO₄), filtered and evaporated. The product was purified by flash chromatography on silica, eluting with ethyl acetate in hexane (2:5 to 3:5). The amine was crystallised as its oxalate salt ([M+H]⁺ = 463).

Similarly prepared from the appropriate *N*'-(*t*-butyloxycarbonyl)-protected amino acid amide:
(*S,S*)-5-[2-Aza-6-cyano-7-methyl-1-(2-methylthioethyl)-6-phenyloctyl]-1-(2-phenylethyl)-1*H*-tetrazole, oxalate ([M+H]⁺ = 477).
(*±*)-5-(2-Aza-6-cyano-7-methyl-6-phenyloctyl)-1-(2,2-diphenylethyl)-1*H*-tetrazole, oxalate (mp 88-90°C)
(*±*)-5-[2-Aza-6-cyano-6-(4-fluorophenyl)-7-methyloctyl]-1-(2,2-diphenylethyl)-1*H*-tetrazole, oxalate (mp 99-102°C)

### EXAMPLE 11

### (+)-N'-(4-Cyano-5-methyl-4-phenylhexyl)-N'-methyl-N-(2,2-diphenylethyl)-glycinamide, maleate

To a solution of (±)-*N*'-(4-cyano-5-methyl-4-phenylhexyl)-*N*-(2, 2-diphenylethyl) glycinamide (0.15g) in DMF (5ml) was added 40% formaldehyde (2ml) and 90% formic acid (2ml) and the reaction mixture heated at reflux for 2h. Water was added and the product extracted into ethyl acetate. The combined organic extracts were washed with water, dried (MgSO₄) and evaporated *in vacuo.* The crude oil was purified by flash chromatography on silica, eluting with 10% ethyl acetate in dichloromethane. The product was crystallised as its maleate from ethyl acetate / hexane ([M+H]⁺ = 468).

The following was similarly prepared:

*N*'-[4-(*S*)-Cyano-5-methyl-4-phenylhexyl]-*N*'-methyl-*N*-(diphenylmethyl)-(*S*)-valinamide (mp 143°C)

### PREPARATION 11

### Preparation of (S)-6-cyano-6-phenyl-7-methyloctanoic acid

### a) (S)-6-Cyano-6-phenyl-7-methyl-3-octen-1-ol

To a stirred suspension of hydroxypropyltriphenylphosphonium bromide (1.10g, 2.73mmol) in dry THF (20mL) at -10°C under nitrogen was added dropwise a 1.6M solution of *n*-butyl lithium in hexane (3.2mL, 5.24mmol). The resultant red/brown solution was stirred at 0°C for 30 min. and treated with chlorotrimethylsilane (0.37mL, 2.95mmol) in THF (3.0mL). The mixture was stirred at 0°C for 10 min. then a solution of (*S*)-3-cyano-3-phenyl-4-methylpentanal (0.50g, 2.49mmol) in THF (10mL) was added dropwise. After stirring at 0 - 25°C for 1h. the reaction mixture was quenched with 2M HCl(aq) (30mL) and stirred for an additional 10 min. The mixture was then diluted with 2M HCl(aq) (70mL) and water (100mL), extracted into ethyl acetate (3 × 70mL), the combined organic extracts washed with water (200mL), dried (MgSO₄) and the solvent removed *in vacuo* to give a pale yellow oil. Flash chromatography over silica eluting with ethyl acetate/hexane (20:80) provided the title compound as a colourless oil.

### b) (S)-6-cyano-6-phenyl-7-methyloctan-1-ol

A mixture of (*S*)-6-cyano-6-phenyl-7-methyl-3-octen-1-ol (0.93g, 3.79mmol) and 10% palladium on carbon catalyst (0.10g) in methanol (80mL) was hydrogenated at ca. 65 psi for 5h. The mixture was filtered through celite, the residue washed with methanol (50mL) and the filtrate evaporated *in vacuo* to give the title compound as a colourless oil.

### c) (S)-6-cyano-6-phenyl-7-methyloctanoic acid

To a stirred solution of (*S*)-6-cyano-6-phenyl-7-methyloctan-1-ol (0.66g, 2.69mmol) in carbon tetrachloride (5.4mL), acetonitrile (5.4mL) and water (8mL) was added successively sodium periodate (1.73g, 8.1mmol) and ruthenium oxide hydrate (8.6mg, 0.065mmol) . The dark biphasic mixture was vigorously stirred at 25°C for 90 min., diluted with dichloromethane (50mL) and water (20mL), the aqueous phase was separated and the organic layer dried (MgSO₄). Solvent was removed *in vacuo* to give a dark oil which was diluted with diethyl ether (60mL), filtered through celite and the residue washed with diethyl ether (2 × 30mL). The filtrate was evaporated *in vacuo* to yield the title compound as a grey oil.

### PREPARATION 12

### Preparation of 6-carbomethoxy-6-phenyl-7-methyloctanoic acid

### a) Methyl 3-methyl-2-phenylbutanoate

To a stirred solution of lithium diisopropylamide (50.0mmol) in THF (100mL) at -78°C under nitrogen was added a solution of methyl phenylacetate (6.76g, 45.0mmol) in dry THF (10mL) dropwise. A fawn solid precipitated and the resulting suspension was stirred at -70°C for 1h. then treated with a solution of 1,3-dimethylimidazolidinone (5.70g, 50.0mmol) and 2-bromopropane (6.80g, 55.0mmol) in THF (10mL) dropwise. The mixture was allowed to warm to 25°C over 3h., then stirred for 16h. The mixture was diluted with water (400mL), extracted with diethyl ether (4 × 100mL), and the combined organic extracts washed with water (3 × 100mL), dried (MgSO₄) and solvent removed *in vacuo* to yield the title compound as an orange/yellow oil. The product was purified by vacuum distillation at ca. 100-120°C at 0.01mBar to yield a pale yellow oil.

### b) Methyl 2-(1-methylethyl)-2-phenyloct-7-enoate

To a stirred solution of methyl 3-methyl-2-phenylbutanoate (7.0g, 36.4mmol) (50.0mmol) and 1,3-dimethylimidazolidinone (4.16g, 40^{.}1mmol) in dry THF (100mL) at -78°C under nitrogen was added a 2.0M solution of lithium diisopropylamide in THF (20mL, 40,1mmol) dropwise. The orange mixture was stirred at -70°C for 1h. and treated dropwise with a solution of 6-bromohexene (6.5g, 40.1mmol) in THF (20mL) . The mixture was allowed to warm to 25°C over 3h. then stirred at 25°C for 16h., quenched with water (300mL), and extracted with diethyl ether (4 × 100mL). The combined organic extracts were washed with water (3 × 100mL), dried (MgSO₄) and the solvent removed *in vacuo* to yield the title compound as an orange oil. The product was purified by vacuum distillation at ca. 140°C at 0.01mBar to yield a yellow oil.

### c) 6-Carbomethoxy-6-phenyl-7-methyloctanoic acid

To a stirred solution of methyl 2-(1-methylethyl)-2-phenyloct-7-enoate (1.0g, 3.7mmol) in carbon tetrachloride (7mL), acetonitrile (7mL) and water (11mL) was added successively sodium periodate (3.24g, 15.2mmol) and ruthenium oxide hydrate (11.9mg, 0.089mmol) . The dark biphasic mixture was vigorously stirred at 25°C for 3h., diluted with dichloromethane (100mL) and water (100mL), the aqueous phase was separated and the organic layer dried (MgSO₄) . Solvent was removed *in vacuo* to give a dark oil which was adsorbed onto silica (ca. 4.0g) and purified by flash chromatography on silica. Elution with ethyl acetate/hexane (30:70) provided the title compound as a colourless oil.

### PREPARATION 13

### Preparation of 6-methoxymethyl-6-phenyl-7-methyloctanoic acid

### a) 2-(1-Methylethyl)-2-phenylhex-5-enenitrile

To a stirred suspension of sodium hydride (60% dispersion in oil) (1.38g, 34.6mmol) in dry DMF (60mL) at 25°C under nitrogen was added a solution of 2-phenyl-3-methylbutanenitrile (5.0g, 31.4mmol) in DMF (40mL) dropwise. The resulting mixture was heated at *ca.* 40-50°C for 1h. after which a solution of 4-bromobutene (5.1g, 38mmol) in DMF (10mL) was added dropwise. The mixture was heated at 40ºC for 18h., cooled to 25ºC and quenched with brine (400mL). The mixture was extracted with diethyl ether (4 × 100mL), the combined organic extracts washed with water (3 × 100mL), dried (MgSO₄) and the solvent removed *in vacuo* to give an orange oil. The crude product was adsorbed onto silica (*ca*. 10g) and purified by flash chromatography on silica. Elution with ethyl acetate/hexane (2.5:97.5) provided the title compound as a colourless oil.

### b) 2-(1-Methylethyl)-2-phenylhex-5-enal

To a stirred solution of 2-(1-methylethyl)-2-phenylhex-5-enenitrile (1.0g, 4.69mmol) in dry toluene (15mL) at -70ºC under nitrogen was added a 1.5M solution of DIBAL-H in toluene (3.75mL, 5.63mmol) dropwise. The mixture was allowed to warm to 25ºC over 2h. and quenched with water (200mL) and conc. hydrochloric acid (15mL). The mixture was extracted into ethyl acetate (3 × 50mL), and the combined organic extracts washed with brine (2 × 100mL), dried (MgSO₄) and the solvent removed *in vacuo* to give an orange/yellow oil. The crude product was adsorbed onto silica (*ca*. 4g) and purified by flash chromatography on silica. Elution with ethyl acetate/hexane (5:95) provided the title compound as a pale yellow oil.

### c) 2-(1-Methylethyl)-2-phenylhex-5-en-1-ol

To a stirred solution of 2-(1-methylethyl)-2-phenylhex-5-enal (0.45g, 2.08mmol) in methanol (20mL) at 0ºC under nitrogen was added sodium borohydride (0.10g, 2.64mmol) in one portion. The mixture was allowed to warm to 20ºC over 30min., the solvent was removed *in vacuo* and the residue dissolved in diethyl ether (100mL). the organic extracts were washed with water (2 × 100mL), dried (MgSO₄) and evaporated *in vacuo* to give a yellow oil. The crude product was adsorbed onto silica *(ca.* 4g) and purified by flash chromatography on silica. Elution with ethyl acetate/hexane (10:90) provided the title compound as a colourless oil.

### d) 2-(1-Methylethyl)-2-phenylhex-5-en-1-yl methyl ether

To a stirred suspension of sodium hydride (60% dispersion in mineral oil) (0.27g, 6.87mmol) in dry THF (20mL) at 25ºC under nitrogen was added a solution of 2-(1-methylethyl)-2-phenylhex-5-en-1-ol (1.0g, 4.58mmol) in dry THF (10mL) dropwise. The brown solution was heated to 60ºC over 2min., allowed to cool to 25ºC and stirred for 1h., then iodomethane (0.5mL, 8.03mmol) added in one portion. The mixture was stirred at 25ºC for 24h, quenched with water (200mL), and extracted into chloroform (3 × 70mL). The combined organic extracts were dried (MgSO₄) and solvent removed *in vacuo* to give an orange oil. The crude product was adsorbed onto silica (*ca*. 4.0g) and purified by flash chromatography on silica, eluting with ethyl acetate/hexane (10:90) to give the title compound as a yellow oil.

### e) 4-Methoxymethyl-5-methyl-4-phenylhexanal

To a stirred solution of 2-(1-methylethyl)-2-phenylhex-5-en-1-yl methyl ether (0.85g, 3.66mmol) in dioxane (7.5mL) and water (2.5mL) was added successively sodium periodate (1.66g, 7.76mmol) and osmium tetroxide (90mg, 0.354mmol). The fawn brown suspension was vigorously stirred at 25ºC for 3h. during which time the colour faded to a yellow coloration. The mixture was diluted with diethyl ether (150mL), and dried (MgSO₄). The solvent was removed *in vacuo* to give a yellow oil which was adsorbed onto silica (ca. 4.0g) and purified by flash chromatography on silica. Elution with ethyl acetate/hexane (10:90) provided the title compound as a colourless oil.

### f) Methyl 6-methoxymethyl-7-methyl-6-phenyloct-2-enoate

To a stirred suspension of sodium hydride (60% dispersion in mineral oil) (0.095g, 2.37mmol) in THF (50mL) at 0ºC under nitrogen was added a solution of trimethyl phosphonoacetate (0.43g, 2.37mmol) in THF (10mL) dropwise. The Wittig-Horner reagent was stirred at 25ºC for 30min., cooled to 0ºC and treated with a solution of 4-methoxymethyl-5-methyl-4-phenylhexanal (0.50g, 2.15mmol) in THF (10mL) dropwise. The mixture was allowed to warm to 25ºC, stirred for 18h., quenched with water (300mL), and extracted into diethyl ether (3 × 50mL). The combined organic extracts were washed with brine (100mL) and dried (MgSO₄). Evaporation *in vacuo* gave the title compound as a yellow oil.

### g) Methyl 6-methoxymethyl-7-methyl-6-phenyloctanoate

A mixture of methyl 6-methoxymethyl-7-methyl-6-phenyloct-2-enoate (0.58g, 2.0mmol) and 10% palladium on carbon catalyst (0.10g) in MeOH (80mL) was hydrogenated at ca. 65 psi for 3h. The mixture was filtered through celite, the residue washed with methanol (50mL) and the filtrate evaporated *in vacuo* to give the title compound as a pale yellow oil.

### h) 6-Methoxymethyl-7-methyl-6-phenyloctanoic acid

To a solution of methyl 6-methoxymethyl-7-methyl-6-phenyloctanoate (0.52g, 1.79mmol) and water (0.07mL) in dry Et₂O (20mL) was added potassium *tert*-butoxide (1.59g, 15.50mmol) in one portion. The suspension was stirred at 25ºC under nitrogen for 4 days, carefully quenched with water (200mL) and extracted with diethyl ether (3 × 70mL). The combined organic extracts were discarded and the aqueous phase was acidified (conc.HCl). The aqueous suspension was extracted with chloroform (3 × 70mL), the combined organic extracts washed with water (200mL) and dried (MgSO₄), and the solvent removed *in vacuo* to yield the title compound as an orange/yellow oil.

### PREPARATION 14

### Preparation of 6-t-butyldimethylsilyloxymethyl-7-methyl-6-phenyloctanoic acid

### a) 2-(1-Methylethyl)-2-phenyloct-7-en-1-ol

To a stirred suspension of lithium aluminium hydride (0.92g, 24.1mmol) in dry diethyl ether (40mL) at -5ºC under nitrogen was carefully added aluminium trichloride (1.07g, 8.03mmol) . The resulting grey suspension was stirred at 25ºC for 30 min., cooled to 0ºC and treated with a solution of methyl 2-(1-methylethyl)-2-phenyl-7-octenoate (2.52g, 9.18mmol) in diethyl ether (10mL) dropwise. The mixture was stirred at 25ºC for 3h., carefully quenched with ice (*ca*. 50g) and 2M HCl(aq) (200mL), filtered through celite and the residue washed with ethyl acetate (3 × 50mL). The aqueous phase was extracted with ethyl acetate (30mL) and the combined organic phases dried (MgSO₄). The solvent was removed *in vacuo* to afford the title compound as a colourless oil.

### b) 2-(1-Methylethyl)-2-phenyloct-7-en-1-yl t-butyldimethylsilyl ether

To a stirred solution of 2-(1-methylethyl)-2-phenyl-7-octen-1-ol (0.80g, 3.25mmol) and imidazole (0.55g, 8.12mmol) in dry DMF (5mL) at 25ºC under nitrogen was added *t*-butyldimethylsilyl chloride (0.74g, 4.88mmol) in one portion. The mixture was stirred at 25ºC for *ca.* 25h., quenched with water (200mL), and extracted into diethyl ether (3 × 70mL). The combined organic phases were washed with water (3 × 70mL) and dried (MgSO₄) and the solvent removed *in vacuo* to give the title compound as a pale yellow oil.

### c) 6-t-Butyldimethylsilyloxymethyl-7-methyl-6-phenyloctanoic acid

To a stirred solution of 2-(1-methylethyl)-2-phenyloct-7-en-1-yl *t*-butyldimethylsilyl ether (1.13g, 3.13mmol) in carbon tetrachloride (9.4mL), acetonitrile (9.4mL) and water (15mL) was added successively sodium periodate (2.76g, 12.9mmol) and ruthenium oxide hydrate (10.1mg, 0.076mmol). The dark biphasic mixture was vigorously stirred at 25ºC for 20h., diluted with dichloromethane (70mL), washed with water (2 × 100mL) and the organic phase dried (MgSO₄). Solvent was removed *in vacuo* to give a dark oil which was adsorbed onto silica (*ca*. 6.0g) and purified by flash chromatography on silica. Elution with ethyl acetate/hexane (20:80) provided the title compound as a colourless oil.

### PREPARATION 15

### Preparation of 6-acetamidomethyl-7-methyl-6-phenyloctanoic acid

### a) 2-(1-Methylethyl)-2-phenyloct-7-enenitrile

A stirred solution of 2-phenyl-3-methylbutanenitrile (10.0g, 62.84mmol) in dry THF (150mL) at -70ºC under nitrogen was treated dropwise with a 2.0M solution of lithium diisopropylamide in THF (34.6mL, 69.2mmol). The orange solution was stirred at -70ºC for 1h. then treated with a solution of 6-bromohexene (11.28g, 69^{.}12mmol) and 1,3-dimethylimidazolidinone (8.0g, 70.1mmol) in THF (50mL) dropwise. The mixture was allowed to warm to 25ºC over 2h., stirred at this temperature for 18h and diluted with water (400mL). The mixture was extracted with diethyl ether (4 × 100mL), the combined organic extracts washed with water (3 × 100mL), dried (MgSO₄) and the solvent removed *in vacuo* to yield the title compound as an orange/yellow oil. The product was purified by vacuum distillation at ca. 100-120ºC at 0.01mBar to yield a pale yellow oil.

### b) 2-(1-Methylethyl)-2-phenyloct-7-enamine

To a stirred suspension of lithium aluminium hydride (2.07g, 54.4mmol) in dry diethyl ether (200mL) at -5ºC under nitrogen was carefully added aluminium trichloride (2.42g, 18.1mmol). The resulting grey suspension was stirred at 25ºC for 30 min., cooled to 0ºC and treated with a solution of 2-(1-methylethyl)-2-phenyl-7-octenenitrile (5.0g, 20.7mmol) in diethyl ether (30mL) dropwise. The mixture was stirred at 25ºC for 3h., carefully quenched with ice (*ca*. 100g) and 2M NaOH(aq) (200mL), filtered through celite and the residue washed with ethyl acetate (3 × 100mL). The aqueous phase was extracted with ethyl acetate (30mL), the organic phases combined and dried (MgSO₄) . Solvent was removed *in vacuo* to afford the title compound as a colourless oil.

### c) N-[2-(1-Methylethyl)-2-phenyloct-7-en-1-yl] acetamide

A solution of 2-(1-methylethyl)-2-phenyloct-7-enamine (3.0g, 12.23mmol) in acetic anhydride (20mL) was heated at 100°C for *ca.* 1h., cooled to 25ºC and quenched with water (200mL). The mixture was heated to 80ºC, stirred at this temperature for 90min., cooled to 25ºC and extracted into ethyl acetate (3 × 100mL). The combined organic extracts were washed with 2M NaOH(aq) (300mL), water (200mL) and dried (Na₂SO₄). The solvent was removed *in vacuo* to give a brown oil which was adsorbed onto silica (ca. 12g) and purified by flash chromatography on silica. Elution with ethyl acetate/hexane (50:50) provided the title compound as a light brown oil.

### d) 6-Acetamidomethyl-7-methyl-6-phenyloctanoic acid

To a stirred solution of N-[2-(1-methylethyl)-2-phenyloct-7-en-1-yl] acetamide (1.80g, 6.27mmol) in carbon tetrachloride (11mL), acetonitrile (11mL) and water (17mL) was added successively sodium periodate (5.49g, 25.65mmol) and ruthenium oxide hydrate (20.3mg, 0.15mmol) . The dark biphasic mixture was vigorously stirred at 25ºC for 16h., diluted with dichloromethane (120mL), washed with water (2 × 100mL) and the organic phase dried (MgSO₄). Solvent was removed *in vacuo* to give a dark oil which was adsorbed onto silica (ca. 6.0g) and purified by flash chromatography on silica. Elution with ethyl acetate provided the title compound as a yellow oil.

### PREPARATION 16

### Preparation of 6-(2,5-dioxo-1-pyrrolidino)methyl-7-methyl-6-phenyloctanoic acid

### a) N-[2-(1-Methylethyl)-2-phenyloct-7-en-1-yl]-2,5-dioxopyrrolidine

To a stirred solution of succinic acid (0.53g, 4.47mmol) in dry DMF under nitrogen was added 1,1'-carbonyldiimidazole (1.45g, 8.94mmol) in portions. Once evolution of carbon dioxide had ceased a solution of 2-(1-methylethyl)-2-phenyloct-7-enamine (0.87g, 3.55mmol) in dry DMF (10mL) was added. The mixture was heated to *ca*. 80-90ºC and stirred at this temperature for 24h. The mixture was quenched with water (300mL), extracted into diethyl ether (3 × 70mL), and the combined organic extracts washed with water (3 × 70mL) and dried (MgSO₄). Solvent was removed *in vacuo* to give a dark oil which was adsorbed onto silica (*ca*. 4.0g) and purified by flash chromatography on silica. Elution with ethyl acetate/hexane (30:70) provided the title compound as a golden yellow oil.

### b) 6-(2,5-dioxo-1-pyrrolidino)methyl-7-methyl-6-phenyloctanoic acid

Prepared in an analogous fashion to 6-acetamido-7-methyl-6-phenyloctanoic acid.

### PREPARATION 17

### Preparation of 6-cyano-6-phenylsulfonyl-7-methyloctanoic acid

### a) 3-Methyl-2-phenylsulfonylbutanenitrile

To a stirred suspension of sodium hydride (60% dispersion in oil)(0.72g, 18mmol) in dry DMF (25mL) at room temperature was added portionwise phenylsulfonylacetonitrile (2.72g, 15mmol) over five minutes (vigorous effervescence and a mild exotherm). Thirty minutes later 2-bromopropane (2.05g, 17mmol) was added dropwise. After sixteen hours at room temperature, more sodium hydride (0.2g, 5mmol) was added, followed twenty minutes later by 2-bromopropane (0.62g, 5mmol). Sixteen hours later, the solution was poured into water (100mL) and the product extracted with ethyl acetate (3 x 30 mL). The combined extracts were washed with water (2 x30 mL), dried (MgSO₄), filtered and concentrated *in vacuo.* The resulting oil was subjected to flash chromatography on silica, eluting with ethyl acetate/hexane (20:80), providing the title compound as a colourless oil.

### b) Methyl 6-cyano-7-methyl-6-phenylsulfonyloctanoate

To a stirred suspension of sodium hydride (60% dispersion in oil)(0.46g, 11.5mmol) in dry DMF (30mL) at room temperature was added dropwise a solution of 3-methyl-2-phenylsulfonylbutanenitrile (2.23g, 10mmol) in DMF (5mL) over five minutes (brisk effervescence and a mild exotherm). After stirring at room temperature for thirty minutes, methyl 5-bromovalerate (2.24g, 11.5mmol) was added and stirring continued for a further sixteen hours. The orange solution was poured into water (150mL) and the product extracted with ethyl acetate (3 x 50mL). The combined extracts were washed with water (3 x 30mL), dried (MgSO₄), filtered and concentrated *in vacuo.* The resulting oil was subjected to flash chromatography on silica, eluting with ethyl acetate/hexane (40:60), providing the title compound as an oil.

### c) 6-Cyano-7-methyl-6-phenylsulfonyloctanoic acid

Methyl 6-cyano-7-methyl-6-methanesulfonyloctanoate (2.2g, 6.5mmol) in methanol (15mL) was treated with 2M aqueous sodium hydroxide solution (10mL) and the resulting mixture stirred at reflux for three hours. The solution was cooled to room temperature, evaporated *in vacuo* until most of the methanol had been removed and then acidified to pH 1 with dilute hydrochloric acid. The suspension was extracted with chloroform (4 x 10mL) . The combined extracts were washed with water (1 x10mL), dried (MgSO₄), filtered and concentrated *in vacuo* furnishing an oil. This was purified by flash chromatography on silica, eluting with ethyl acetate/hexane (40:60), yielding the title acid as a colourless oil that subsequently solidified (m.p. 105-106 °C).

Also prepared by this three step procedure starting from the appropriate sulfonylacetonitrile were:
6-Cyano-7-methyl-6-(pyridine-2-sulfonyl)octanoic acid
6-Cyano-7-methyl-6-(thiophene-2-sulfonyl)octanoic acid
6-Cyano-6-methanesulfonyl-7-methyloctanoic acid.

Also using the methodology of steps (b) and (c), (propane-2-sulfonyl)phenylacetonitrile (preparation below) was converted to 6-cyano-6-phenyl-6-(propane-2-sulfonyl)hexanoic acid and methanesulfonyl phenylacetonitrile (preparation below) was converted to 6-cyano-6-methanesulfonyl-6-phenylhexanoic acid.

### PREPARATION 18

### Preparation of (propane-2-sulfonyl)phenylacetonitrile

A mixture of iodobenzene (13.9g, 68mmol), propane-2-sulfonylacetonitrile (20.0g, 18mmol), copper (I) iodide (1.29g, 6.8mmol) and potassium carbonate (37.6g, 272mmol) in DMSO (100mL) was stirred at 120 °C under an atmosphere of nitrogen for forty hours. The dark blue mixture was allowed to cool to room temperature, poured into dilute hydrochloric acid (300mL) and extracted with ether (200mL) and ethyl acetate (2 x 150mL). The combined organic extracts were washed with water (8 x 125mL), dried (MgSO₄), filtered and evaporated *in vacuo* to yield a black oil (20g). This was dissolved in dichloromethane and filtered through a large pad of "flash" silica, eluting with dichloromethane. Fractions containing the product were combined and concentrated, yielding a yellow oil. This was distilled in a short-path distillation apparatus, providing a yellow oil (B.Pt. 230 °C at 0.3 mBar).

Also prepared in this fashion starting from methanesulfonylacetonitrile was methanesulfonyl phenylacetonitrile.

### PREPARATION 19

### Preparation of 6-methanesulfonyl-6-phenyl-7-methyloctanoic acid

### a) 7-Methanesulfonyl-8-methyl-7-phenyl-1-nonene

To a stirred solution of benzyl methyl sulfone (5.1g, 30mmol) in dry THF (90mL) at room temperature under nitrogen was added dropwise lithium diisopropylamide (2.0M, 16.5mL). The orange solution was stirred at room temperature for one hour when 6-bromohexene (5.1g, 32mmol) was added dropwise. After two hours at room temperature, TLC indicated complete consumption of the starting material, so more lithium diisopropylamide (2.0M, 16.5mL) was added and the cherry red solution stirred for one hour before the addition of 2-bromopropane (4.0g, 32mmol). The solution turned orange then yellow over a period of two hours. Stirring was continued overnight. After pouring the solution into dilute hydrochloric acid (100mL), the layers were separated and the aqueous phase extracted with ethyl acetate (2 x 40mL). The combined organic extracts were washed with water (1 x 50mL), dried (MgSO₄), filtered and concentrated . Flash chromatography on silica, eluting with ethyl acetate/hexane (10:90, then 20:80) provided firstly the title compound as a colourless oil (3.62g), then in later fractions 7-methanesulfonyl-7-phenyl-1-heptene.

### b) 6-Methanesulfonyl-6-phenyl-7-methyloctanoic acid

To a stirred solution of 7-methanesulfonyl-8-methyl-7-phenyl-1-nonene (895mg, 3mmol) in acetonitrile (9mL) was added carbon tetrachloride (9mL) followed by water (18mL). The two phase mixture was stirred vigorously and ruthenium oxide hydrate (10mg) added. After five minutes, sodium periodate (2.55g, 11.9mmol) was added. Vigorous stirring was continued overnight. The mixture was then filtered through a pad of celite, the layers separated and the aqueous phase extracted with chloroform (2 x 10mL). The combined organics were washed with water, dried (MgSO₄), filtered and evaporated to yield an oil. This was flash chromatographed on silica, eluting with ethyl acetate/hexane (40:60) providing the title acid as a viscous oil.

### PREPARATION 20

### Preparation of 4-(1-cyano-2-methyl-1-phenyl-1-propanesulfonyl)-butanoic acid

### a) 4-Pentenesulfonyl phenylacetonitrile.

To a stirred solution of methanesulfonyl phenylacetonitrile (325mg, 1.66mmol) in dry THF (15mL) at -78 °C under nitrogen was added dropwise lithium diisopropylamide (2.0M, 2mL). After thirty minutes, a solution of 1-bromobutene (250mg, 1.85mmol) in THF (1mL) was added and the cooling bath removed. Two hours later, the solution was diluted with ethyl acetate (20mL) and then washed with dilute hydrochloric acid (20mL). The aqueous phase was extracted with ethyl acetate (2 x 10mL), The combined organic extracts were washed with water (1 x 30mL), dried (MgSO₄), filtered and concentrated . Flash chromatography on silica, eluting with ethyl acetate/hexane (20:80) provided the desired product as an oil.

### b) 3-Methyl-2-(4-pentenesulfonyl)-2-phenylbutanenitrile.

To a suspension of sodium hydride (60% dispersion in oil)(31mg, 0.78mmol) in dry DMF (6mL) at room temperature was added dropwise a solution of 4-pentenesulfonyl phenylacetonitrile (168mg, 0.67mmol) in DMF (lmL). There was brisk effervescence. After thirty minutes, a solution of 2-bromopropane (123mg, 1mmol) in DMF (1mL) was added and the solution then warmed to 70 °C for five hours. Stirring was then continued at room temperature for sixteen hours.
The solution was poured into water (20mL) and the product extracted with ethyl acetate (3 x 5 mL). The combined extracts were washed with water (2 x 5mL), dried (MgSO₄), filtered and concentrated *in vacuo.* The resulting oil was subjected to flash chromatography on silica, eluting with ethyl acetate/hexane (20:80), providing the title compound as a colourless oil.

### c) 4-(1-Cyano-2-methyl-1-phenyl-1-propanesulfonyl) butanoic acid

To a stirred solution of 3-methyl-2-(4-pentenesulfonyl)-2-phenylbutanenitrile (339mg, 1.16mmol) in acetonitrile (3mL) was added carbon tetrachloride (3mL) followed by water (6mL). The two phase mixture was stirred vigorously and ruthenium oxide hydrate (10mg) added. After five minutes, sodium periodate (980mg, 4.6mmol) was added. Vigorous stirring was continued overnight. The mixture was then diluted with dichloromethane, the layers separated and the organic phase washed with water (2 x 5mL), dried (MgSO₄), filtered and evaporated to yield an oil. This was flash chromatographed on silica, eluting with ethyl acetate/hexane (20:80) providing firstly 4-(1-cyano-2-methyl-1-phenyl-1-propanesulfonyl)butanal, then after elution with ethyl acetate/hexane (40:60) the title acid as a viscous oil.

### PREPARATION 21

### Preparation of 6-cyano-7-methyl-6-(2-thienyl)octanoic acid

### a) 2-(2-Thienyl)-3-methylbutanenitrile

A solution of 2-thienylacetonitrile (2.54g, 20mmol) in dry THF (50mL) was added to sodium hydride (1.0g, 50% dispersion in oil, 2lmmol) under nitrogen. The mixture was stirred for 10 minutes, warmed to 45° then stirred at room temperature for 2h. 2-bromopropane (2.2mL, 23mmol) was added and the mixture stirred at room temperature for 20h. The mixture was poured onto ice, extracted into ethyl acetate, washed with water and dried (MgSO₄). The crude product was purified by flash chromatography on silica , eluting with ethyl acetate/hexane(10:90).

### b) Methyl 6-cyano-7-methyl-6-(2-thienyl)octanoate

A solution of 2-(2-thienyl)-3-methylbutanenitrile (2.12g, 12mmol) in dry DMF (25mL) under nitrogen was added to sodium hydride (0.86g, 50% dispersion in oil, 18mmol) followed by methyl 5-bromovalerate (2.75g, 14mmol). The mixture was stirred at room temperature for 30min., poured onto ice, acidified with 2N hydrochloric acid, and extracted into ethyl acetate. After concentration the title compound was purified by flash chromatography on silica eluting with dichloromethane.

### c) 6-Cyano-7-methyl-6-(2-thienyl)octanoic acid

To methyl 6-cyano-7-methyl-6-(2-thienyl)octanoate (2.54g, 9mmol) in THF (100mL) was added 1M aqueous lithium hydroxide (25mL). The mixture was stirred at room temperature under nitrogen for 72h. After concentration the mixture was acidified to pH1 with 2M HCl and extracted into ethyl acetate. The title compound was purified on silica eluting with ethyl acetate: dichloromethane.

Similarly prepared:
6-cyano-7-methyl-6-(2-pyridyl)octanoic acid

### PREPARATION 22

### Preparation of 3-(2-cyano-3-methyl-2-phenylbutyl)benzoic acid

### a) Methyl 3-(2-cyano-3-methyl-2-phenylbutyl)benzoate

To sodium hydride (600mg, 50% dispersion in mineral oil, 12.5mmol) was added slowly a solution of 3-methyl-2-phenylbutanenitrile (1.59g, 10mmol) in dry THF (25mL). The mixture was stirred at room temperature for 10min. then a solution of methyl 3-(bromomethyl)benzoate (2.5g, 11mmol) in dry THF (10mL) added. The mixture was stirred for 30min., poured onto ice and extracted into ethyl acetate (3x 25mL). The combined organic extracts were washed with brine, dried (MgSO₄) and concentrated *in vacuo.* The crude product was purified by flash chromatography on silica eluting with 10-100% ethyl acetate in hexane.

### b) 3-(2-Cyano-3-methyl-2-phenylbutyl)benzoic acid

To methyl 3-(2-cyano-3-methyl-2-phenylbutyl)benzoate (2.5g, 8mmol) in diethyl ether (25mL) was added potassium *tert*-butoxide (5.5g, 48mmol) and water (0.1mL). The mixture was stirred at room temperature for 72h., poured onto ice and extracted into ethyl acetate (3x 25mL). The combined extracts were washed with brine, dried (MgSO₄) and concentrated *in vacuo.* The title compound was purified by flash chromatography on silica eluting with ethyl acetate.

### PREPARATION 23

### Preparation of 5-[3-(1-methylethyl)-1,3-dihydro-2-oxo-2H-indol-3-yl]pentanoic acid

### a) 3-Isopropylideneoxindole

A mixture of oxindole (6.65g, 50mmol) and piperidine (10mL, 100mmol) was heated under reflux in acetone (75mL) for 20 hours. The mixture was concentrated under reduced pressure and the residue crystallised from ethyl acetate.

### b) 3-(1-Methylethyl)oxindole

3-Isopropylideneoxindole (6g, 34mmol) was hydrogenated in ethanol at 60psi using 5% palladium on carbon (0.25g). The catalyst was removed by filtration and the solvent removed *in vacuo* to give the title compound.

### c) Methyl 5-[3-(1-methylethyl)-1,3-dihydro-2-oxo-2H-indol-3-yl]pentanoate

To a solution of N,N,N',N'-tetramethylethylenediamine (3.3mL, 22mmol) in dry THF (50mL) under a nitrogen atmosphere at -70° was added *n*-butyllithium (13.75mL of 1.6M solution in hexanes, 22mmol). The mixture was stirred for 30min. at -70° then a solution of 3-(1-methylethyl)oxindole (1.75g, 10mmol) in dry THF (25mL) added. This mixture was stirred at -70° to -50° for 30 min., then a solution of methyl 5-iodopentanoate (3g, 12mmol) in dry THF (25mL) added. The mixture was stirred, allowing the temperature to rise to room temperature over 1h. After 2h. at room temperature excess 10% aqueous ammonium chloride solution was added and the mixture extracted into ethyl acetate. The combined organic extracts were washed with brine, dried (MgSO₄) and concentrated under reduced pressure. The product was purified by flash chromatography on silica , eluting with ethyl acetate/dichloromethane (20:80).

### d) 5-[3-(1-Methylethyl)-1,3-dihydro-2-oxo-2H-indol-3-yl]pentanoic acid

The title compound was prepared from methyl 5-[3-(1-methylethyl)-1,3-dihydro-2-oxo-2H-indol-3-yl]pentanoate using the method employed for the hydrolysis of methyl 3-(2-cyano-3-methyl-2-phenylbutyl)benzoate.

### PREPARATION 24

### Preparation of 6-methoxymethoxy-7-methyl-6-phenyloctanoic acid

### a) 1-(1-Methylethyl)-1-phenylhept-6-en-1-ol

To a Grignard solution prepared from 6-bromohexene (7.16g, 44mmol) and magnesium turnings (1.08g, 45mmol) in THF (100mL) was added isobutyrophenone (3.58g, 24mmol). The mixture was stirred at room temperature for 20h., excess 10% aqueous ammonium chloride solution added and the product extracted into ethyl acetate. The title compound was purified by flash chromatography on silica eluting with dichloromethane.

### b) 7-Methoxymethoxy-8-methyl-7-phenylnon-1-ene

A solution of 1-(1-methyIethyI)-1-phenyIhept-6-en-1-ol (4.2g, 18mmol) in dry DMF (50mL) was added to sodium hydride (1.25g, 50% dispersion in mineral oil, 26mmol). The mixture was warmed to 60° for 15 min., then cooled to 0ºC and chloromethyl methyl ether (2.13mL, 28mmol) added. The mixture was stirred at room temperature for 20h., poured onto ice and extracted into ethyl acetate. The title product was purified by flash chromatography on silica gel eluting with toluene.

The following ether was similarly prepared from 1-(1-methylethyl)-1-phenylhept-6-en-1-ol:
8-Methyl-7-(2-methylprop-2-en-1-oxy)-7-phenylnon-1-ene.

### c) 6-Methoxymethoxy-7-methyl-6-phenyloctanoic acid

To an efficiently stirred mixture of 7-methoxymethoxy-8-methyl-7-phenylnon-1-ene (3g, 11mmol) in carbon tetrachloride (24mL), acetonitrile (24mL) and water (36mL) was added ruthenium(IV)oxide hydrate (50mg) followed by sodium periodate (10.5g, 50mmol), portionwise. The mixture was vigorously stirred at room temperature for 20h. and extracted into dichloromethane. The title product was purified by flash chromatography on silica gel eluting with ethyl acetate.

The following acid was similarly prepared by oxidative cleavage of the corresponding unsaturated ethers:
7-Methyl-6-(2-oxopropyloxy)-6-phenyloctanoic acid, from 8-methyl-7-(2-methylprop-2-en-1-oxy)-7-phenylnon-1-ene.

### PREPARATION 25

### Preparation of 1-tert-Butoxycarbonyl-N',N'-dimethyl-N-(2-phenylethyl)-(R)-proline hydrazide

### a) N,N-Dimethyl-N'-(2-phenylethyl)hydrazine

Phenylacetaldehyde (5.5g, 46mmol) and dimethylhydrazine (2.9g, 48mmol) in methanol (60mL) were stirred for 18h at room temperature. Sodium cyanoborohydride (5.8g, 92mmol) was added and the solution stirred as acetic acid (2.76g, 46mmol) was added dropwise over 5min. After stirring for a further 1h, the reaction mixture was evaporated *in vacuo ,* the oil taken up in ethyl acetate and washed with 0.5M sodium hydroxide, then brine. The organic phase was dried (MgSO₄), filtered and evaporated. The crude product was purified by flash chromatography on silica, eluting with 0-10% methanol in ethyl acetate, to yield *N,N*-dimethyl-*N*'-(2-phenylethyl)hydrazine as an oil.

### b) 1-tert-Butoxycarbonyl-N',N'-dimethyl-N-(2-phenylethyl)-(R)-proline hydrazide

1-*tert*-Butoxycarbonyl-(*R*)-proline (1.08g, 5mmol) was dissolved in dichloromethane (50mL) containing triethylamine (1.53mL, 11mmol) and stirred as 2-chloro-1-methyl pyridinium iodide (1.41g, 5.5mmol) was added. After 1h *N,N*-dimethyl-*N*'-(2-phenylethyl)hydrazine (0.90g, 5.5mmol) was added rapidly and the reaction left to stir overnight. The reaction mixture was diluted with ethyl acetate, washed twice with water, then brine, dried, filtered and evaporated *in vacuo.* The crude product was purified by flash chromatography on silica, eluting with ethyl acetate, then methanol/ethyl acetate (10:90), to yield the product as a white crystalline product.

The same method was employed to prepare 1-*tert*-butoxycarbonyl-*N*'-benzyl-(*R*)-proline hydrazide.

### PREPARATION 26

### Preparation of N-diphenylmethyl-(R)-prolinamide

### a) 1-(tert-Butoxycarbonyl)-N-diphenylmethyl-(R)-prolinamide

*N,N*'-Carbonyldiimidazole (1.95g, 12mmol) was added to 1-(*tert*-butoxycarbonyl)-(*R*)-proline (2.15g, 10.0mmol) in dichloromethane (40mL). After 2 h aminodiphenylmethane (1.83g, 10mmol) was added and the reaction mixture stirred overnight. The organic phase was washed with water, then dried (MgSO₄) and evaporated *in vacuo* to give an oil . The product was purified by flash chromatography on silica, eluting with hexane/ethyl acetate (20:80) to yield an oil.

The following amides were similarly prepared:
1-(*tert*-Butoxycarbonyl)-*N*-(3-chlorobenzyl)-(*R*) -prolinamide
1-(*tert*-Butoxycarbonyl)-*N*-(3-trifluoromethylbenzyl)-(*R*)-prolinamide
1-(*tert*-Butoxycarbonyl)-*N*-(3-methyl-1-butyl)-(*R*)-prolinamide
1-(*tert*-Butoxycarbonyl)-*N*-[1-(*R*)-phenylethyl]-(*R*)-prolinamide
1-(*tert*-Butoxycarbonyl)-*N*-(3-methyl-1-butyl)-thiazolidine-4-(*R*)-carboxamide
1-(*tert*-Butoxycarbonyl)-*N*-benzyl-*N*-methyl-(*R*)-prolinamide
(±)-trans-1-(*tert*-Butoxycarbonyl)-*N*-(diphenylmethyl)-3-azabicyclo[3.1.0]hexane-2-carboxamide

### b) N-Diphenylmethyl-(R)-prolinamide

1-(*tert*-Butoxycarbonyl)-*N*-diphenylmethyl- (*R*)-prolinamide (2.75g, 7.2mmol) was dissolved in dichloromethane (30mL) and trifluoroacetic acid (5.5mL) was added. After stirring overnight, the mixture was evaporated, dissolved in ethyl acetate and washed with diluted ammonia solution, brine, dried (MgSO₄) and evaporated *in vacuo .*

The following amines were similarly prepared by deprotection of the corresponding 1-(*tert*-butoxycarbonyl) derivatives:
*N*-(3-chlorobenzyl)-(*R*)-prolinamide
*N-*(3-Trifluoromethylbenzyl)-(*R*)-prolinamide
*N*-(3-Methyl-1-butyl)-(*R*)-prolinamide
*N*-[1-(*R*)-phenylethyl]-(*R*)-prolinamide
*N',N'*-Dimethyl-*N*-(2-phenylethyl)-(*R*)-proline hydrazide
*N*'-Benzyl-(*R*)-proline hydrazide
*N*-(3-Methyl-1-butyl)-thiazolidine-4-(*R*)-carboxamide
*N*-Benzyl-*N*-methyl-(*R*)-prolinamide
(±)-*trans*-*N*-(Diphenylmethyl)-3-azabicyclo[3.1.0]hexane-2-carboxamide

### EXAMPLE 12

### 1-[6-(S)-Cyano-7-methyl-6-phenyloctanoyl]-N-diphenylmethyl-(R)-prolinamide

To a solution of 6-(*S*)-cyano-7-methyl-6-phenyloctanoic acid (0.53g, 2.1mmol) in dry dichloromethane (10mL) at 0 °C was added 2-chloro-1-methylpyridinium iodide (0.59g, 2.3mmol) and triethylamine (0.6mL, 4.2mmol). The reaction mixture was stirred at room temperature for 40 minutes before *N*-diphenylmethyl-(*R*)-prolinamide (0.46g, 2.1mmol) was added in dichloromethane (1mL). The reaction mixture was stirred at room temperature overnight, then diluted with ethyl acetate. The organic extract was washed with aqueous sodium carbonate, brine, and aqueous citric acid, dried (MgSO₄), filtered and evaporated *in vacuo.* The product was purified by flash chromatography on silica, eluting with ethyl acetate/hexane (1:1).(mp = 45-50ºC).

The following compounds were similarly prepared:
1-[6-(*R*-Cyano-7-methyl-6-phenyloctanoyl]-*N*-diphenylmethyl-(*R*)-prolinamide (mp = 160-165ºC)
1-[6-Cyano-7-methyl-6-(2-pyridyl)octanoyl]-*N*-diphenylmethyl-(*R*)-prolinamide (mp = 166-168ºC)
(±)-Trans-3-[6-(*S*)-Cyano-7-methyl-6-phenyloctanoyl]-*N*-diphenylmethyl-3-azabicyclo[3.1.0]hexane-2-carboxamide (mp = 156-158ºC)

1-[6-(*S*)-Cyano-7-methyl-6-phenyloctanoyl]-*N*-benzyl-*N*-methyl-(*R*)-prolinamide
(¹³C-NMR: δ18.6, 18.9, 24.5, 24.7, 24.8, 25.6, 28.9, 29.5, 34.4, 34.7, 47.2, 47.3, 51.2, 53.3, 53.8, 56.3, 77.2, 121.4, 126.4, 126.6, 127.2, 127.5, 127.6, 127.7, 128.6, 128.7, 128.9, 136.8, 137.2, 138.2, 171.3, 171.4, 172.3, 172.8).

1-(6-Cyano-7-methyl-6-phenyloctanoyl)-*N*-[1-(*R*)-phenylethyl]-(*R*)-prolinamide
(¹³C-NMR: δ18.6, 19.0, 22.2, 24.9, 27.2, 30.9, 32.5, 38.0, 47.3, 48.9, 53.1, 59.9, 120.9, 126.0, 126.2, 127.1, 127.9, 128.6, 129.0, 137.4, 143.5, 170.1, 171.8).

1-(6-Cyano-7-methyl-6-phenyloct-2-enoyl)-*N*-[1-(*R*)-phenylethyl]-(*R*)-prolinamide
(¹³C-NMR: δ18.9, 22.4, 25.0, 26.8, 28.7, 36.5, 37.9, 47.5, 49.0, 53.6, 59.9, 120.7, 121.8, 126.0, 126.3, 127.0, 127.8, 128.5, 128.9, 137.5, 143.7, 145.1, 166.0, 170.1).

1-(6-Carbomethoxy-7-methyl-6-phenyloctanoyl)-*N*-diphenylmethyl-(*R*)-prolinamide
(¹³C-NMR: δ18.1, 19.2, 24.9, 25.1, 25.7, 26.7, 33.4, 34.7, 36.5, 46.9, 47.6, 51.5, 56.7, 57.0, 59.3, 59.6, 61.7, 126.4, 127.1, 127.3, 127.4, 127.6, 128.5, 128.6, 128.7, 128.8, 139.8, 141.9, 142.0, 170.1, 173.7, 175.7).

1-(6-Methoxymethyl-7-methyl-6-phenyloctanoyl)-*N*-diphenylmethyl-(*R*)-prolinamide
(¹³C-NMR: δ17.9, 18.1, 24.1, 25.1, 26.1, 26.5, 33.7, 34.8, 35.1, 47.4, 47.5, 56.9, 59.1, 59.5, 75.4, 125.6, 127.1, 127.2, 127.4, 127.7, 128.5, 128.6, 142.0, 143.5, 170.0, 174.0).

1-(6-Acetoxymethyl-7-methyl-6-phenyloctanoyl)-*N*-diphenylmethyl-(*R*)-prolinamide
(¹³C-NMR: δ18.0, 18.1, 21.1, 24.2, 25.1, 26.0, 26.7, 34.1, 34.8, 35.1, 46.6, 47.6, 57.0, 59.6, 66.3, 126.0, 127.2, 127.3, 127.4, 128.0, 128.5, 128.7, 142.0, 142.3, 170.1, 171.2, 173.8).

1-(6-Acetamidomethyl-7-methyl-6-phenyloctanoyl)-*N*-diphenylmethyl-(*R*)-prolinamide
(¹³C-NMR: δ17.8, 18.2, 23.1, 23.4, 23.5, 25.1, 25.7, 25.8, 27.0, 33.4, 33.6, 34.0, 34.3, 34.4, 42.6, 42.7, 47.1, 47.7, 47.8, 57.0, 57.1, 59.8, 126.2, 127.2, 127.2, 127.3, 127.4, 127.8, 128.1, 128.6, 128.8, 141.9, 141.9, 142.0, 170.2, 170.2, 170.3, 173.8, 173.9).

1-[6-(2,5-Dioxo-1-pyrrolidino)methyl-7-methyl-6-phenyloctanoyl]-*N*-diphenylmethyl-(*R*)-prolinamide
(¹³C-NMR: δ17.8, 19.2, 24.8, 24.9, 25.0, 26.1, 26.3, 26.7, 26.9, 27.9, 33.6, 33.7, 34.0, 34.1, 34.6, 34.8, 44.8, 45.0, 47.5, 48.7, 56.8, 56.9, 59.6, 59.7, 126.0, 127.0, 127.1, 127.2, 127.23, 127.4, 127.7, 128.5, 128.6, 128.7, 141.9, 142.1, 170.2, 173.9, 174.0, 177.4, 177.4).

1-(6-Cyano-6-methanesulfonyl-7-methyloctanoyl)-*N*-diphenylmethyl-(*R*)-prolinamide
(¹³C-NMR: δ17.7, 19.7, 24.9, 25.0, 25.2, 26.9, 29.1, 31.8, 34.0, 40.2, 47.6, 57.0, 59.7, 61.6, 69.6, 116.9, 127.1, 127.2, 127.3, 127.5, 128.6, 128.7, 141.8, 141.84, 170.0, 172.8).

1-(6-Cyano-7-methyl-6-phenylsulfonyloctanoyl)-*N*-diphenylmethyl-(*R*)-prolinamide
(¹³C-NMR: δ17.4, 19.9, 24.9, 25.0, 25.2, 25.3, 26.8, 29.0, 29.1, 31.7, 31.8, 32.1, 34.1, 34.13, 47.5, 57.0, 59.7, 70.6, 117.0, 127.1, 127.2, 127.3, 127.3, 128.5, 128.6, 128.8, 129.4, 130.4, 134.5, 134.7, 141.8, 170.0, 172.9).

1-(6-Cyano-7-methyl-6-phenylsulfonyloctanoyl)-*N*-(diphenylmethyl)-4-(*S*)-hydroxy-(*R*)-prolinamide
(¹³C-NMR: δ17.4, 19.9, 24.7, 24.9, 25.1, 25.3, 29.0, 31.8, 31.84, 34.1, 34.2, 35.7, 35.8, 54.7, 57.2, 58.5, 70.2, 70.6, 117.0, 127.1, 127.2, 127.4, 128.6, 128.7, 129.4, 130.3, 135.0, 135.6, 141.5, 141.6, 169.9, 173.0).

1-[6-Cyano-7-methyl-6-(thiophene-2-sulfonyl)octanoyl]-*N*-diphenylmethyl-(*R*)-prolinamide
(¹³C-NMR: δ17.6, 19.9, 24.9, 25.0, 25.3, 25.4, 26.8, 29.3, 29.4, 32.1, 32.2, 34.1, 47.5, 56.6, 57.0, 59.7, 61.6, 71.8, 116.7, 127.1, 127.2, 127.3, 127.4, 128.4, 128.6, 128.7, 135.7, 136.3, 137.5, 141.8, 141.9, 170.0, 172.9).

1-[6-Cyano-7-methyl-6-(pyridine-2-sulfonyl)octanoyl]-*N*-diphenylmethyl-(*R*)-prolinamide
(¹³C-NMR: δ17.7, 19.7, 24.9, 25.1, 25.2, 25.3, 26.8, 29.3, 29.4, 31.8, 31.9, 34.1, 47.6, 56.6, 57.0, 59.7, 61.6, 70.8, 116.6, 126.2, 127.1, 127.2, 127.3, 127.4, 128.4, 128.6, 128.7, 128.8, 138.4, 141.8, 141.9, 150.4, 154.8, 170.0, 173.0).

1-[6-Cyano-6-methanesulfonyl-6-phenylhexanoyl]-*N*-diphenylmethyl-(*R*)-prolinamide
(¹³C-NMR: δ22.9, 24.3, 24.4, 24.6, 25.0, 25.7, 26.7, 30.9, 32.1, 34.0, 36.6, 46.9, 47.5, 56.5, 56.9, 57.0, 59.6, 70.8, 116.2, 127.0, 127.1, 127.2, 127.3, 127.33, 127.7, 127.9, 128.5, 128.6, 128.8, 129.0, 129.6, 130.6, 141.8, 141.9, 170.0, 172.7).

1-[6-Cyano-6-phenyl-6-(propane-2-sulfonyl)hexanoyl]-*N*-diphenylmethyl-(*R*)-prolinamide
(¹³C-NMR: δ15.8, 17.4, 24.2, 24.3, 24.4, 25.0, 26.6, 26.7, 32.7, 34.1, 46.9, 47.5, 53.7, 56.9, 57.0, 59.6, 69.8, 116.6, 127.0, 127.1, 127.2, 127.3, 127.6, 128.5, 128.6, 128.8, 129.6, 130.4, 141.8, 141.9, 169.9, 172.9).

1-(6-Methanesulfonyl-7-methyl-6-phenyloctanoyl)-*N*-diphenylmethyl-(*R*)-prolinamide
(¹³C-NMR: δ18.6, 19.6, 22.6, 23.0, 25.1, 25.2, 26.1, 26.2, 26.8, 26.9, 27.8, 32.1, 33.1, 33.2, 34.3, 34.4, 40.7, 40.8, 47.0, 47.6, 57.0, 57.8, 59.7, 127.1, 127.3, 128.4, 128.5, 128.6, 128.9, 129.1, 129.3, 136.9, 141.9, 170.1, 173.2).

1-[4-(1-Cyano-2-methyl-1-phenyl-1-propanesulfonyl)butanoyl]-*N*-diphenylmethyl-(*R*)-prolinamide
(¹³C-NMR: δ17.3, 17.4, 19.5, 19.7, 24.9, 26.9, 27.0, 32.1, 32.3, 34.5, 34.6, 46.9, 47.4, 51.4, 51.5, 56.9, 57.0, 59.7, 59.8, 114.7, 127.0, 127.1, 127.2, 127.3, 127.5, 128.6, 128.9, 129.7, 130.3, 130.9, 141.6, 141.8, 169.9, 171.6).

1-[6-(*S*)-Cyano-7-methyl-6-phenyloctanoyl]-4-(*R*)-hydroxy-*N*-diphenylmethyl-(*R*)-prolinamide.
(¹³C-NMR: δ18.5, 18.9, 24.7, 25.5, 34.4, 37.1, 37.6, 37.7, 53.7, 57.5, 57.6, 59.7, 70.8, 121.2, 126.3, 126.9, 127.2, 127.5, 127.6, 128.5, 128.7, 138.0, 140.8, 141.1, 171.7, 173.3).

1-[3-(2-Cyano-3-methyl-2-phenylbutyl)benzoyl-*N*-(diphenylmethyl)-(*R*)-prolinamide
(¹³C-NMR: δ18.8, 19.2, 25.3, 26.4, 26.6, 37.1, 37.4, 43.9, 44.2, 49.9, 50.2, 55.8, 57.2, 59.5, 59.7, 125.6, 125.7, 126.9, 127.0, 127.1, 127.4, 127.6, 128.3, 128.6, 128.7, 132.0, 135.7, 135.8, 169.7).

1-[6-Cyano-7-methyl-6-(2-thienyl)octanoyl]-*N*-(diphenylmethyl)-(*R*)-prolinamide
(¹³C-NMR: δ18.6, 18.7, 24.6, 24.7, 25.0, 25.3, 25.5, 26.7, 34.4, 39.1, 39.3, 47.5, 50.8, 56.9, 59.6, 120.6, 125.0, 126.4, 126.5, 127.0, 127.2, 127.3, 127.6, 127.8, 127.9, 128.5, 128.6, 128.7, 128.9, 141.8, 141.9, 142.5, 170.1, 173.3).

1-[6-Cyano-7-methyl-6-(2-thienyl)octanoyl]-*N*-(3-methylbutyl)-(*R*)-prolinamide
(¹³C-NMR: δ18.6, 18.8, 22.5, 22.7, 24.4, 24.5, 25.0, 25.1, 25.5, 25.9, 26.9, 27.0, 31.9, 33.4, 34.4, 37.8, 38.3, 39.2, 39.3, 46.9, 47.5, 50.9, 59.6, 120.6, 125.0, 126.4, 126.6, 142.5, 173.0).

*N*-(Diphenylmethyl)-1-(6-methoxymethoxy-7-methyl-6-phenyloctanoyl)-(*R*)-prolinamide (¹³C-NMR : δ 17.1, 17.7, 23.2, 25.0, 25.6, 26.6, 34.3, 34.8, 35.1, 47.5, 56.1, 56.9, 59.5, 85.2, 91.6, 126.5, 127.0, 127.2, 127.4, 128.5, 128.6, 141.3, 141.8, 141.9, 170.1, 173.9).

*N*-(Diphenylmethyl)-1-[7-methyl-6-(2-oxopropyloxy)-6-phenyloctanoyl]-(*R*)-prolinamide
(¹³C-NMR: δ16.8, 18.0, 22.7, 25.1, 25.5, 26.7, 27.1, 29.7, 32.8, 34.0, 34.8, 47.6, 56.9, 59.6, 68.4, 84.4, 126.9, 127.1, 127.2, 127.3, 127.6, 128.5, 128.6, 139.8, 141.8, 141.9, 170.1, 173.5, 207.5).

3-[6(*S*)-Cyano-7-methyl-6-phenyloctanoyl]-*N*-(3-methylbutyl)-thiazolidine-4-(*R*)-carboxamide
(¹³C-NMR: δ18.5, 18.8, 22.1, 22.4, 24.4, 24.5, 25.3, 25.6, 25.8, 29.6, 31.4, 31.5, 31.6, 34.4, 34.5, 34.7, 34.9, 35.7, 37.1, 37.4, 37.5, 37.7, 38.0, 38.2, 49.2, 49.4, 49.6, 53.7, 61.7, 63.3, 76.4, 76.6, 76.8, , 121., 121.2, 125.8, 126.0, 126.6, 127.6, 127.8 , 127.8, 128.4, 128.4, 128.5, 137.9, 169.2, 169.5, 171.8, 171.9).

1-[6-(*S*)-Cyano-7-methyl-6-phenyloctanoyl]-*N',N'*-Dimethyl-*N*-(2-phenylethyl)-(*R*)-proline hydrazide
(¹H-NMR: δ0.77 (3H, d), 0.96(1H, m), 1.19 (3H, d), 1.3-2.3 (11H, m), 2.37 (1H, m), 3.2-3.4 (2H, m), 3.92 (2H, dd), 4.45 (1H, dd), 7.2-7.4 (10H, m), 8.34 (1H, bs)).

1-[6-(*S*)-Cyano-7-methyl-6-phenyloctanoyl]-*N*'-Benzyl-(*R*)-proline hydrazide
(¹³C-NMR : δ18.9, 24.6, 24.7, 25.6, 29.2, 34.4, 35.2, 37.6, 37.8, 41.0, 43.8, 44.0, 47.3, 53.8, 56.6, 121.4, 126.3, 126.4, 127.5, 128.4, 128.5, 128.7, 128.9, 138.2, 139.0, 139.6, 171.1, 174.1).

### EXAMPLE

### 1-(6-Cyano-7-methyl-6-phenyloctanoyl)-7-(diphenylmethyl)-1,7-diazaspiro [4,4] nonan-6-one

### a) 2-(Prop-2-en-1-yl)-1-benzyloxycarbonylproline tert-butyl ester

To a solution of 1-benzyloxycarbonyl-(*S*)-proline *tert*-butyl ester (7g, 23mmol) in THF (130mL) at -78 ºC, was added 2M lithium diisopropylamide in THF (12mL, 24mmol). The reaction was stirred at -78 ºC for 30 minutes and allyl bromide (3.05g, 25.2mmol) was added. It was allowed to warm to -40ºC over a period of 3 hours. The reaction mixture was diluted with ethyl acetate and the organic extract washed with brine, dried (MgSO₄), filtered and evaporated *in vacuo.* The product was purified by flash chromatography on silica, eluting with ethyl acetate/hexane (80:20).

### b) 2-Formylmethyl-1-benzyloxycarbonylproline tert-butyl ester

To a solution of 2-(prop-2-en-1-yl)-1-benzyloxycarbonylproline *tert*-butyl ester (3.45g, 10mmol) in dioxane (33mL) was added water (11mL) and osmium tetroxide (0.033g, 0.13mmol). The reaction was stirred at room temperature for ten minutes, then sodium periodate (4.5g, 21mmol) added portion wise. After the last addition the reaction was stirred for two hours, diluted with ethyl ether and the organic extract washed with brine, dried (MgSO₄), filtered and evaporated *in vacuo.* The product was purified by flash chromatography on silica, eluting with ethyl acetate/hexane (80:20).

### c) 2-[2-(Diphenylmethylamino)ethyl]-1-benzyloxycarbonylproline tert-butyl ester

A solution of 2-formylmethyl-1-benzyloxycarbonylproline *tert*-butyl ester ( 4.13g, 12mmol) and aminodiphenylmethane (2.2g, 12mmol) in methanol (120mL) was stirred at room temperature for two hours in the presence of 3Å molecular sieves powder. Sodium borohydride (0.69g,18.2mmol) was then added and the reaction mixture stirred overnight. The reaction was diluted with ethyl acetate and the organic extract washed with brine, dried (MgSO₄), filtered and evaporated *in vacuo.* The product was purified by flash chromatography on silica, eluting with ethyl acetate/hexane (80:20).

### d) 1-Benzyloxycarbonyl-7-(diphenylmethyl)-1,7-diazaspiro [4,4] nonan-6-one

To a solution of 2-[2-(diphenylmethylamino)ethyl]-1-benzyloxycarbonylproline *tert*-butyl ester (1.5g, 3mmol) in dichloromethane (50mL) was added trifluoroacetic acid (10mL) and the reaction mixture stirred at room temperature for seven hours. After evaporation *in vacuo,* the crude amine was dissolved in dichloromethane (25mL) and triethylamine (0.8mL, 9mmol) and 2-chloro-1-methylpyridinium iodide (0.84g, 3.3mmol) added. After stirring at room temperature over 48 hours, the reaction mixture was diluted with ethyl acetate and the organic extract washed with aqueous citric acid, brine, and aqueous potassium carbonate, then dried (MgSO₄), filtered and evaporated *in vacuo.*

### e) 7-(Diphenylmethyl)-1,7-diazaspiro [4,4] nonan-6-one

A solution of 1-benzyloxycarbonyl-7-(diphenylmethyl)-1,7-diazaspiro [4,4] nonan-6-one (0.11g, 0.25 mmol) in HBr/AcOH (2mL) was stirred at room temperature for two hours. The reaction mixture was cooled in an ice bath and solid potassium carbonate added to neutralise. It was then diluted with ethyl acetate, and the organic extract washed with brine, dried (MgSO₄), filtered and evaporated *in vacuo.* The crude reaction product was purified by flash chromatography on silica, eluting with methanol/ethyl acetate (20:80).

### f) 1-(6-(S)-Cyano-7-methyl-6-phenyloctanoyl)-7-(diphenylmethyl)-1,7-diazaspiro [4,4] nonan-6-one

To a solution of 6-cyano-7-methyl-6-phenyloctanoic acid (0.04g, 0.15mmol) in dry dichloromethane (2mL) at 0 °C was added 2-chloro-1-methylpyridinium iodide (0.042g, 0.17mmol) and triethylamine (0.04mL, 0.3mmol). The reaction mixture was stirred at room temperature for 40 minutes before addition of 7-(diphenylmethyl)-1,7-diazaspiro [4,4] nonan-6-one (0.05g, 0.15mmol) in dichloromethane (1mL). The reaction mixture was stirred at room temperature overnight, then diluted with ethyl acetate. The organic extracts were washed with aqueous sodium carbonate, brine and aqueous citric acid, then dried (MgSO₄), filtered and evaporated *in vacuo.* The product was purified by flash chromatography on silica, eluting with 40% ethyl acetate in hexane.
Isomer 1. (¹H-NMR: δ7.38-7.20 (15H,m), 6.59 (1H, s), 3.63-3.48 (2H, m), 3.33 (1H, t), 2.89 (1H, q), 2.76 (1H, q), 2.35-2.21 (1H, m), 2.20-2.02 (6H, m), 1.93-1.65 (5H, m), 1.47-1.25 (1H, m), 1.18 (3H, d), 1.09-0.95 (1H, m), 0.77 (3H, d)).
Isomer 2. (¹H-NMR: δ7.37-7.34 (15H,m), 6.58 (1H, s), 3.53-3.50 (2H, m), 3.35 (1H, t), 2.90 (1H, q), 2.71 (1H, q), 2.23-2.04 (7H, m), 1.93-1.62 (5H, m), 1.47-1.26 (1H, m), 1.17 (3H, d), 1.13-0.86 (1H, m), 0.77 (3H, d)).

### EXAMPLE 14

### 1-(4-Cyano-5-methyl-4-phenylhexyloxycarbonyl)-N-(diphenylmethyl)-(R)-prolinamide

To a solution of *N*-(diphenylmethyl)-(*R*)-prolinamide (1.2g, 4.2mmol) in dry dimethylformamide (20mL) was added 4-cyano-5-methyl-4-phenylhexyl chloride (0.99g, 4.2mmol), potassium carbonate (0.89g, 6.43 mmol) and a catalytic amount of potassium iodide. The reaction mixture was stirred at 60°C for 48 hours, then diluted with ethyl acetate andwashed with brine. The organic extracts were dried (MgSO₄), filtered and evaporated *in vacuo.* The crude product was purified by flash chromatography on silica gel, eluting with ethyl acetate / hexane (1:1). The product was further purified by HPLC on silica, eluting with 80% hexane / 2% ethanol in dichloromethane to give Isomer 1 (m.p. 48-50ºC) and Isomer 2.(m.p. 48-50ºC).

1-(4-Cyano-5-methyl-4-phenylhexyloxycarbonyl)-*N*-(diphenylmethyl)-(*S*)-prolinamide Isomers 1 and 2 (m.p. 57-60ºC and 58-60ºC respectively) were prepared by the same method.

### EXAMPLE 15

### 1-[6-(S)-Cyano-7-methyl-6-phenyloctanoyl]-N-(diphenylmethyl)-4-(S)-hydroxy-(R)-prolinamide

### a) 1-tert-Butoxycarbonyl-4-(R)-hydroxy-(R)-proline

To a solution of 4-(*R*)-hydroxyproline (2g, 15.3mmol) in dioxane (26mL) at 0°C was added 0.5N NaOH (34mL) and di-*tert*-butyl carbonate (3.7g, 16.8mmol) . The reaction was stirred at room temperature overnight, then evaporated *in vacuo.* The crude product was taken up in ethyl acetate, acidified with 1N potassium hydrogen sulfate solution to pH 1-2, then washed with brine, and the organic extracts dried (MgSO₄), filtered and evaporated *in vacuo.*

### b) 1-tert-Butoxycarbonyl-N-(diphenylmethyl)-4-(R)-hydroxy-(R)-prolinamide

To a solution of 1-*tert*-butoxycarbonyl-4-(*R*)-hydroxy-(*R*)-proline (1.35g, 5.9mmol) in dichloromethane (25mL) was added aminodiphenylmethane (1.1g, 5.9mmol) and dicyclohexylcarbodiimide (1.3g, 6.45mmol).The reaction mixture was stirred at room temperature overnight. After evaporating *in vacuo,* the crude product was triturated with ethyl ether, filtered and the filtrate evaporated *in vacuo.* The product was purified by flash chromatography eluting with ethyl acetate/hexane (20:80).

### c) N-(Diphenylmethyl)-4-(S)-(4-nitrophenylacetoxy)-(R)-prolinamide

To a solution of 1-*tert*-butoxycarbonyl-*N*-(diphenylmethyl)-4-(*R*)-hydroxy-(*R*)-prolinamide (0.21g, 0.53mmol) in dry THF (5mL) was added triphenylphosphine (0.15g, 0.58mmol) and 4-nitrophenylacetic acid (0.14g, 0.80mmol). The reaction was cooled to -15ºC and diethyl azodicarboxylate (0.06g, 0.33mmol) added dropwise. The reaction mixture was stirred at this temperature for further 25 minutes, then allowed to warm to room temperature. After stirring at room temperature overnight the reaction was diluted with ethyl acetate and washed with aqueous sodium carbonate, brine and aqueous citric acid, then dried (MgSO₄), filtered and evaporated *in vacuo.* The crude product was dissolved in methanol (1mL) and dichloromethane (2mL), methanesulphonic acid (0.1mL) added, and the reaction mixture stirred overnight. The reaction was diluted with ethyl acetate, washed with aqueous potassium carbonate and brine, then dried (MgSO₄), filtered and evaporated *in vacuo.* The product was purified by flash chromatography on silica, eluting with ethyl acetate.

### d) 1-[6-(S)-Cyano-7-methyl-6-phenyloctanoyl]-N-(diphenylmethyl)-4-(S)-(4-nitrophenylacetoxy)-(R)-prolinamide

To a solution of 6-cyano-7-methyl-6-phenyloctanoic acid (0.06g, 0.25mmol) in dry dichloromethane (2mL) at 0 °C was added 2-chloro-1-methylpyridinium iodide (0.07g, 0.28mmol) and triethylamine (0.07mL, 0.51mmol). The reaction mixture was stirred at room temperature for 40 minutes before *N*-(diphenylmethyl)-4-(*S*)-(4-nitrophenylacetoxy)-(*R*)-prolinamide (0.12g, 0.25mmol) was added in dichloromethane (1mL). The reaction mixture was stirred at room temperature overnight, then diluted with ethyl acetate. The organic extracts were washed with aqueous sodium carbonate, brine, and aqueous citric acid, then dried (MgSO₄), filtered and evaporated *in vacuo.* The product was purified by flash chromatography on silica, eluting with hexane/ethyl acetate (1:1).

### e) 1-[6-(S)-Cyano-7-methyl-6-phenyloctanoyl]-N-(diphenylmethyl)-4-(S)-hydroxy-(R)-prolinamide

To a solution of 1-6-(*S*)-Cyano-7-methyl-6-phenyloctanoyl)-*N*-(diphenylmethyl)-4-(*S*)-(4-nitrophenylacetoxy) (*R*)-prolinamide (0.1g, 0.14mmol) in methanol (30mL) was added saturated potassium carbonate (10mL) and the reaction stirred at room temperature overnight. The reaction was diluted with dichloromethane and washed with brine, then dried (MgSO₄), filtered and evaporated *in vacuo.* The product was purified by flash chromatography on silica, eluting with ethyl acetate. (¹³C-NMR : δ18.8, 24.8, 25.5, 34.5, 35.5, 37.4, 37.7, 53.7, 54.5, 57.1, 58.3, 70.1, 121.2, 126.3, 127.0, 127.3, 127.5, 128.5, 128.6, 128.7, 138, 141.5, 141.6, 169.7, 173.3).

### EXAMPLE 16

### 1-{5-13-(1-Methylethyl)-1,3-dihydro-2-oxo-2H-indol-3-yl]pentanoyl}-N-(diphenylmethyl)-(R)-prolinamide

To a solution of 5-[3-(1-methylethyl)-1,3-dihydro-2-oxo-2H-indol-3-yl]pentanoic acid (0.275g, 1mmol) and *N*-(diphenylmethyl)-(*R*)-prolinamide (0.28g, 1mmol) in dry dichloromethane (25mL) was added 1,3-dicyclohexylcarbodiimide (0.22g, 1.1mmol) . The mixture was stirred at room temperature under nitrogen for 20 hours. The precipitate was removed by filtration and the filtrate purified by flash chromatography on silica, eluting with ethyl acetate to give the title compound as a mixture of diastereomers, (¹³C-NMR : δ17.2, 17.4, 24.2, 24.385, 25.0, 25.3, 26.6, 27.0, 34.2, 34.5, 34.9, 35.2, 35.4, 47.6, 56.7, 56.9, 57.0, 59.5, 59.7, 109.2, 122.2, 124.0, 127.1, 127.2, 127.3, 127.4, 127.6, 128.4, 128.6, 131.5, 141.1, 141.935, 173.6, 181.7).

### EXAMPLE 17

### 3-[6-(S)-Cyano-7-methyl-6-phenyloctanoyl]-N-(3-methylbutyl)-thiazolidine-4-(R)-carboxamide-1-oxide

To a solution of 3-[6-(*S*)-cyano-7-methyl-6-phenyloctanoyl]-*N*-(3-methylbutyl)-thiazolidine-4-(*R*)-carboxamide (0.2g, 0.43mmol) in dry dichloromethane (4mL) was added 3-chloroperbenzoic acid (0.11g, 0.43mmol) . The reaction mixture was stirred at room temperature overnight, diluted with ethyl acetate and washed with sodium bicarbonate solution. The organic extracts were dried (MgSO₄), filtered and evaporated *in vacuo.* The crude product was purified by flash chromatography on silica gel, eluting with 80% hexane in ethyl acetate.
(¹³C-NMR : δ18.4, 18.8, 22.3, 24.5, 25.1, 25.7, 34.0, 37.2, 37.7, 38.0, 38.1, 52.2, 53.6, 57.7, 68.3, 121.1, 126.2, 127.5, 128.6, 137.8).

### EXAMPLE 18

### 1-(4-Cyano-5-methyl-4-phenylhexylaminocarbonyl)-N-(3-chlorobenzyl)-(R)-prolinamide

### a) 4-Cyano-5-methyl-4-phenylhexyl chloride

A mixture of 3-methyl-2-phenylbutanenitrile (37.6g, 0.24mol), 1-bromo-3-chloropropane (71.6g, 0.46mol) and tetrabutylammonium chloride (2.3g) in THF (540mL) was stirred as solid potassium hydroxide (85%) (91.1g, 1.62mol) was added in portions. The stirred mixture was heated at reflux under nitrogen for 1h. The mixture was cooled to room temperature and filtered through Celite. The filtrate was washed with water, dried (MgSO₄), filtered and evaporated *in vacuo.* The crude oil was purified by vacuum distillation to yield 4-cyano-5-methyl-4-phenylhexyl chloride (B.Pt. 100-104°C @ 0.5mBar) .

### b) 4-Cyano-5-methyl-4-phenylhexylamine

To a solution of 4-cyano-5-methyl-4-phenylhexyl chloride (5.0g, 19.6mmol) in dry dimethylformamide (65mL) was added sodium azide (2.55g, 39.2mmol). The reaction mixture was heated to 70ºC for 17 hours. After cooling to room temperature, it was poured into an ice-water mixture with vigorous stirring. The mixture was extracted into diethyl ether and washed with brine The organic extracts were dried (MgSO₄), filtered and evaporated *in vacuo.* The crude azide was dissolved in ethanol (58 mL) and 10% palladium on carbon (0.5g) added. The reaction mixture was hydrogenated for 4 hours under 60 psi of hydrogen. The solution was filtered through celite and evaporated *in vacuo.* The crude product was dissolved in ethyl acetate and extracted into 2N HCl. The aqueous extract was neutralised with 2N NaOH, the amine product extracted into ethyl acetate and the organic solution washed with water. The organic extracts were dried (MgSO₄), filtered and evaporated *in vacuo* to yield the title compound.

### c) 1-(4-Cyano-5-methyl-4-phenylhexylaminocarbonyl)-N-(3-chlorobenzyl)-(R)-prolinamide

To a solution of triphosgene (0.23g, 0.77mmol) in dry dichloromethane (5mL) was added *N*-(3-chlorobenzyl)-(*R*)-prolinamide (0.50g, 2.09mmol) and diisopropylethylamine (0.30g, 2.3mmol) in.dry dichloromethane (5mL). The reaction mixture was stirred at room temperature for 30 min and then 4-cyano-5-methyl-4-phenylhexyl amine (0.45g, 2.09mmol) and diisopropylethylamine (0.30g, 2.3mmol) in dry dichloromethane (5mL) added. The reaction mixture was stirred at room temperature overnight, diluted with ethyl acetate and washed with aqueous citric acid solution. The organic extracts were dried (MgSO₄), filtered and evaporated *in vacuo.* The crude product was purified by flash chromatography on silica gel, eluting with ethyl acetate. The product was further purified by HPLC on silica, eluting with 75% hexane/ 2% ethanol in dichloromethane to give Isomer 2 (mp=155-160ºC).

The following compounds were prepared by the same method:

1-(4-Cyano-5-methyl-4-phenylhexylaminocarbonyl)-*N*-(3-methylbutyl)-(*R*)-prolinamide.
(¹³C-NMR : δ18.6, 18.9, 22.4,24.9 ,25.9, 26.8, 28.1, 34.8, 37.7, 37.8, 38.3, 40.1, 46.3, 53.6, 60.2, 76.6, 121.1, 127.7, 137.8, 157.6, 171.9).

1-(4-Cyano-5-methyl-4-phenylhexylaminocarbonyl)-*N*-diphenylmethyl-(*R*)-prolinamide (Isomer 1).
(¹³C-NMR : δ18.5, 18.8, 24.9, 26.6, 27.7, 34.7, 37.8, 40.2, 46.3, 53.5, 56.8, 60.1, 121.2, 126.3, 127.1, 127.2, 127.2, 127.7, 128.5, 128.5, 128.8, 137.7, 141.8, 141.8, 157.7)

1-(4-Cyano-5-methyl-4-phenylhexylaminocarbonyl)-*N*-diphenylmethyl-(*R*)-prolinamide (Isomer 2).
(¹³C-NMR : δ14.1, 18.5, 18.9, 22.6, 25.0, 26.6, 26.7, 27.6, 31.5, 34.7, 37.7, 40.2, 46.2, 53.6, 56.7, 56.8, 60.1, 121.1, 126.3, 127.0, 127.2, 127.3, 127.7, 128.4, 128.5, 128.8, 137.8, 141.8, 141.8).

1-(4-Cyano-5-methyl-4-phenylhexylaminocarbonyl)-*N*-(3-methylbutyl)-thiazolidine-4-(*R*)-carboxamide
(¹³C-NMR: δ18.5, 18.8, 22.4, 25.8, 26.4, 26.5, 26.6, 33.7, 34.8, 37.7, 37.8, 37.9, 38.1, 38.1, 38.2, 39.8, 40.4, 49.4, 53.6, 62.9, 121.1, 121.2, 126.2, 127.6, 127.7,128.7, 128.8, 137.7, 137.8, 156.4).

### EXAMPLE 19

### 1-[6-(S)-Cyano-7-methyl-6-phenyloctanoyl]-N-(3-methylbutyl)-(R)-prolinamide

### a) 1-(6-(S)-Cyano-7-methyl-6-phenyloctanoyl)-(R)-proline benzyl ester

To a solution of 6-(*S*)-cyano-7-methyl-6-phenyloctanoic acid (1.5g, 5.9mmol) in dichloromethane (75mL) was added (*R*)-proline benzyl ester hydrochloride (1.42g, 5.9mmol) and dicyclohexylcarbodiimide (1.3g, 6.45mmol) . The reaction mixture was stirred at room temperature overnight, then evaporated *in vacuo.* The crude mixture was diluted with ethyl ether, filtered and the filtrate evaporated *in vacuo.* The product was purified by flash chromatography eluting with 40% ethyl acetate in hexane.

### b) 1-(6-(S)-Cyano-7-methyl-6-phenyloctanoyl)-(R)-proline

To a solution of 1-(6-(*S*)-cyano-7-methyl-6-phenyloctanoyl)-(*R*)-proline benzyl ester (3.45g, 10mmol) in ethanol (45mL) was added 10% Pd on charcoal (0.13g), cyclohexene (5mL) and acetic acid (5mL). The reaction mixture was refluxed for 4 hours, then filtered through celite and evaporated *in vacuo.* The product was purified by flash chromatography on silica, eluting with 40% ethyl acetate in hexane.

### c) 1-[6-(S)-Cyano-7-methyl-6-phenyloctanoyl]-N-(3-methylbutyl)-(R)-prolinamide

To polystyrene-bound 1-ethyl-3-(3-dimethylaminopropyl)carbodiimide (loading ca. 0.1 mmole/g) (0.10g) was added a 25 mM solution of 3-methylbutylamine in chloroform (1mL), followed by a 37.5 mM solution of 1-(6-(*S*)-Cyano-7-methyl-6-phenyloctanoyl)-(*R*)-proline in chloroform (1mL). The mixture was shaken at room temperature for 41h, then filtered. The resin was washed with chloroform (2x 2mL) and the combined filtrates concentrated to give the required product as a gum. (TS-MS: m/z 426, [M+H]⁺).

The following products were similarly prepared using polymer-supported coupling reagent:
1-[6-(*S*)-Cyano-7-methyl-6-phenyloctanoyl]-*N*-(benzyl)-(*R*)-prolinamide (TS-MS: m/z 446, [M+H]⁺)
1-[6-(*S*)-Cyano-7-methyl-6-phenyloctanoyl]-*N*-(2-bromobenzyl)-(*R*)-prolinamide (TS-MS: m/z 524, [M+H]⁺)
1-[6-(*S*)-Cyano-7-methyl-6-phenyloctanoyl]-*N*-(2-chlorobenzyl)-(*R*)-prolinamide (TS-MS: m/z 480, [M+H]⁺)
1-[6-(*S*)-Cyano-7-methyl-6-phenyloctanoyl]-*N*-(2-ethoxybenzyl)-(*R*)-prolinamide (TS-MS: m/z 490, [M+H]⁺)
1-[6-(*S*)-Cyano-7-methyl-6-phenyloctanoyl]-*N*-(2-fluorobenzyl)-(*R*)-prolinamide (TS-MS: m/z 464, [M+H]⁺)
1-[6-(*S*)-Cyano-7-methyl-6-phenyloctanoyl]-*N*-(2-methylbenzyl)-(*R*)-prolinamide (TS-MS: m/z 460, [M+H]⁺)
1-[6-(*S*)-Cyano-7-methyl-6-phenyloctanoyl]-*N*-(2-trifluoromethylbenzyl)-(*R*)-prolinamide (TS-MS: m/z 514, [M+H]⁺)
1-[6-(*S*)-Cyano-7-methyl-6-phenyloctanoyl]-*N*-(3-bromobenzyl)-(*R*)-prolinamide (TS-MS: m/z 524, [M+H]⁺)
1-[6-(*S*)-Cyano-7-methyl-6-phenyloctanoyl]-*N*-(3-chlorobenzyl)-(*R*)-prolinamide (TS-MS: m/z 480, [M+H]⁺)
1-[6-(*S*)-Cyano-7-methyl-6-phenyloctanoyl]-*N*-(3-fluorobenzyl)-(*R*)-prolinamide (TS-MS: m/z 464, [M+H]⁺)
1-[6-(*S*)-Cyano-7-methyl-6-phenyloctanoyl]-*N*-(3-iodobenzyl)-(*R*)-prolinamide (TS-MS: m/z 572, [M+H]⁺)
1-[6-(*S*)-Cyano-7-methyl-6-phenyloctanoyl]-*N*-(3-methoxybenzyl)-(*R*)-prolinamide (TS-MS: m/z 476, [M+H]⁺)
1-[6-(*S*)-Cyano-7-methyl-6-phenyloctanoyl]-*N*- (3-methylbenzyl)-(*R*)-prolinamide (TS-MS: m/z 460, [M+H]⁺)
1-[6-(*S*)-Cyano-7-methyl-6-phenyloctanoyl]-*N*-(3-trifluoromethoxybenzyl)-(*R*)-prolinamide (TS-MS: m/z 530, [M+H]⁺)
1-[6-(*S*)-Cyano-7-methyl-6-phenyloctanoyl]-*N*-(3-trifluoromethylbenzyl)-(*R*)-prolinamide (TS-MS: m/z 514, [M+H]⁺)
1-[6-(*S*)-Cyano-7-methyl-6-phenyloctanoyl]-*N*- (4-bromobenzyl)-(*R*)-prolinamide (TS-MS: m/z 524, [M+H]⁺)
1-[6(S)-Cyano-7-methyl-6-phenyloctanoyl]-*N*-(4-chlorobenzyl)-(*R*)-prolinamide (TS-MS: m/z 480, [M+H]⁺)
1-[6-(*S*)-Cyano-7-methyl-6-phenyloctanoyl]-*N*-(4-fluorobenzyl)-(*R*)-prolinamide (TS-MS: m/z 464, [M+H]⁺)
1-[6-(*S*)-Cyano-7-methyl-6-phenyloctanoyl]-*N*-(4-methoxybenzyl)-(*R*)-prolinamide (TS-MS: m/z 476, [M+H]⁺)
1-[6-(*S*)-Cyano-7-methyl-6-phenyloctanoyl]-*N*-(4-methylbenzyl)-(*R*)-prolinamide (TS-MS: m/z 460, [M+H]⁺)
1-[6-(*S*)-Cyano-7-methyl-6-phenyloctanoyl]-*N*-(4-nitrobenzyl)-(*R*)-prolinamide (TS-MS: m/z 491, [M+H]⁺)
1-[6-(S)-Cyano-7-methyl-6-phenyloctanoyl]-*N*-(4-trifluoromethoxybenzyl)-(*R*)-prolinamide (TS-MS: m/z 530, [M+H]⁺)
1-[6-(*S*)-Cyano-7-methyl-6-phenyloctanoyl]-*N*-(4-trifluoromethylbenzyl)-(*R*)-prolinamide (TS-MS: m/z 514, [M+H]⁺)
1-[6-(*S*)-Cyano-7-methyl-6-phenyloctanoyl]-*N*-(2,3-dichlorobenzyl)-(*R*)-prolinamide (TS-MS: m/z 514, [M+H]⁺)
1-[6-(*S*)-Cyano-7-methyl-6-phenyloctanoyl]-*N*-(2,3-dimethoxybenzyl)-(*R*)-prolinamide (TS-MS: m/z 506, [M+H]⁺)
1-[6-(*S*)-Cyano-7-methyl-6-phenyloctanoyl]-*N*-(2,3-dimethylbenzyl)-(*R*)-prolinamide (TS-MS: m/z 474, [M+H]⁺)
1-[6-(*S*)-Cyano-7-methyl-6-phenyloctanoyl]-*N*-(2,4-dichlorobenzyl)-(*R*)-prolinamide (TS-MS: m/z 514, [M+H]⁺)
1-[6-(*S*)-Cyano-7-methyl-6-phenyloctanoyl]-*N*-(2,4-difluorobenzyl)-(*R*)-prolinamide (TS-MS: m/z 482, [M+H]⁺)
1-[6-(*S*)-Cyano-7-methyl-6-phenyloctanoyl]-*N*-(2-chloro-4-fluorobenzyl)-(*R*)-prolinamide (TS-MS: m/z 498, [M+H]⁺)
1-[6-(*S*)-Cyano-7-methyl-6-phenyloctanoyl] -*N*- (4-chloro-2-fluorobenzyl)-(*R*)-prolinamide (TS-MS: m/z 498, [M+H]⁺)
1-[6-(*S*)-Cyano-7-methyl-6-phenyloctanoyl]-*N*-(4-fluoro-2-trifluoromethylbenzyl)-(*R*)-prolinamide (TS-MS: m/z 532, [M+H]⁺)
1-[6-(*S*)-Cyano-7-methyl-6-phenyloctanoyl]-*N*-(2, 5-dichlorobenzyl)-(*R*)-prolinamide (TS-MS: m/z 514, [M+H]⁺)
1-[6-(*S*)-Cyano-7-methyl-6-phenyloctanoyl]-*N*-(2, 5-difluorobenzyl)-(*R*)-prolinamide (TS-MS: m/z 482, [M+H]⁺)
1-[6-(*S*)-Cyano-7-methyl-6-phenyloctanoyl]-*N*-(2-fluoro-5-trifluoromethylbenzyl)-(*R*)-prolinamide (TS-MS: m/z 532, [M+H]⁺)
1-[6-(*S*)-Cyano-7-methyl-6-phenyloctanoyl]-*N*-(2,6-dimethoxybenzyl)-(*R*)-prolinamide (TS-MS: m/z 506, [M+H]⁺)
1-[6-(*S*)-Cyano-7-methyl-6-phenyloctanoyl]-*N*-(2-chloro-6-fluorobenzyl)-(*R*)-prolinamide (TS-MS: m/z 498, [M+H]⁺)
1-[6-(*S*)-Cyano-7-methyl-6-phenyloctanoyl]-*N*-(2-chloro-6-methylbenzyl)-(*R*)-prolinamide (TS-MS: m/z 494, [M+H]⁺)
1-[6-(*S*)-Cyano-7-methyl-6-phenyloctanoyl]-*N*-(3,4-dichlorobenzyl)-(*R*)-prolinamide (TS-MS: m/z 514, [M+H]⁺)
1-[6-(*S*)-Cyano-7-methyl-6-phenyloctanoyl]-*N*-(3,4-difluorobenzyl)-(*R*)-prolinamide (TS-MS: m/z 482, [M+H]⁺)
1-[6-(*S*)-Cyano-7-methyl-6-phenyloctanoyl]-*N*-(3,4-dimethylbenzyl)-(*R*)-prolinamide (TS-MS: m/z 474, [M+H]⁺)
1-[6-(*S*)-Cyano-7-methyl-6-phenyloctanoyl]-*N*-(3,4-methylenedioxybenzyl)-(*R*)-prolinamide (TS-MS: m/z 490, [M+H]⁺)
1-[6-(*S*)-Cyano-7-methyl-6-phenyloctanoyl]-*N*-(3-chloro-4-fluorobenzyl)-(*R*)-prolinamide (TS-MS: m/z 498, [M+H]⁺)
1-[6-(*S*)-Cyano-7-methyl-6-phenyloctanoyl]-*N*-(3-chloro-4-methylbenzyl)-(*R*)-prolinamide (TS-MS: m/z 494, [M+H]⁺)
1-[6-(*S*)-Cyano-7-methyl-6-phenyloctanoyl]-*N*-(3, 5-dichlorobenzyl)-(*R*) -prolinamide (TS-MS: m/z 514, [M+H]⁺)
1-[6-(*S*)-Cyano-7-methyl-6-phenyloctanoyl]-*N*-(3, 5-difluorobenzyl)-(*R*)-prolinamide (TS-MS: m/z 482, [M+H]⁺)
1-[6-(*S*)-Cyano-7-methyl-6-phenyloctanoyl]-*N*-(3,5-dimethylbenzyl)-(*R*)-prolinamide (TS-MS: m/z 474, [M+H]⁺)
1-[6-(*S*)-Cyano-7-methyl-6-phenyloctanoyl]-*N*-[3,5-di(trifluoromethyl)benzyl]-(*R*)-prolinamide (TS-MS: m/z 582, [M+H]⁺)
1-[6-(S)-Cyano-7-methyl-6-phenyloctanoyl]-*N*-(3-fluoro-5-trifluoromethylbenzyl)-(*R*)-prolinamide (TS-MS: m/z 532, [M+H]⁺)
1-[6-(*S*)-cyano-7-methyl-6-phenyloctanoyl]-*N*-(2,4-dichloro-6-methylbenzyl)-(*R*)-prolinamide (TS-MS: m/z 528, [M+H]⁺)
1-[6-(*S*)-Cyano-7-methyl-6-phenyloctanoyl]-*N*-(3,4,5-trimethoxybenzyl)-(*R*)-prolinamide (TS-MS: m/z 536, [M+H]⁺)
1-[6-(*S*)-Cyano-7-methyl-6-phenyloctanoyl]-*N*-(2-phenylethyl)-(*R*)-prolinamide (TS-MS: m/z 460, [M+H]⁺)
1-[6-(*S*)-Cyano-7-methyl-6-phenyloctanoyl]-*N*-[2-(2-chlorophenyl)ethyl]-(*R*)-prolinamide (TS-MS: m/z 494, [M+H]⁺)
1-[6-(*S*)-Cyano-7-methyl-6-phenyloctanoyl]-*N*-[2-(2-methoxyphenyl)ethyl]-(*R*)-prolinamide (TS-MS: m/z 490, [M+H]⁺)
1-[6-(*S*)-Cyano-7-methyl-6-phenyloctanoyl]-*N*-[2-(3-methoxyphenyl)ethyl]-(*R*)-prolinamide (TS-MS: m/z 490, [M+H]⁺)
1-[6-(*S*)-Cyano-7-methyl-6-phenyloctanoyl]-*N*-[2 -(3-trifluoromethylphenyl)ethyl]-(*R*)-prolinamide (TS-MS: m/z 528, [M+H]⁺)
1-[6-(*S*)-Cyano-7-methyl-6-phenyloctanoyl]-*N*-[2-(4-nitrophenyl)ethyl]-(*R*)-prolinamide (TS-MS: m/z 505, [M+H]⁺)
1-[6-(*S*)-Cyano-7-methyl-6-phenyloctanoyl]-*N*-[2-(2-thienyl)ethyl]-(*R*)-prolinamide (TS-MS: m/z 466, [M+H]⁺)
1-[6-(*S*)-Cyano-7-methyl-6-phenyloctanoyl]-*N*-(indan-2-yl)-(*R*)-prolinamide (TS-MS: m/z 472, [M+H]⁺)
1-[6-(*S*)-Cyano-7-methyl-6-phenyloctanoyl]-*N*-(2-phenylpropyl)-(*R*)-prolinamide (TS-MS: m/z 474, [M+H]⁺)
1-[6-(*S*)-Cyano-7-methyl-6-phenyloctanoyl]-*N*-[1-(3,4-methylenedioxyphenyl)prop-2-yl]-(*R*) -prolinamide (TS-MS: m/z 518, [M+H]⁺)
1-[6-(*S*)-Cyano-7-methyl-6-phenyloctanoyl]-*N*-adamantyl-(*R*)-prolinamide (TS-MS: m/z 490, [M+H]⁺)
1-[6-(*S*)-Cyano-7-methyl-6-phenyloctanoyl]-*N*-butyl-(*R*)-prolinamide (TS-MS: m/z 412, [M+H]⁺)
1-[6-(*S*)-Cyano-7-methyl-6-phenyloctanoyl]-*N*-cyclohexyl-(*R*)-prolinamide (TS-MS: m/z 438, [M+H]⁺)
1-[6-(*S*)-Cyano-7-methyl-6-phenyloctanoyl]-*N*-(3-methylcyclohexyl)-(*R*)-prolinamide (TS-MS: m/z 452, [M+H]⁺)
1-[6-(*S*)-Cyano-7-methyl-6-phenyloctanoyl]-*N*-(4-methylcyclohexyl)-(*R*)-prolinamide (TS-MS: m/z 452, [M+H]⁺)
1-[6-(*S*)-Cyano-7-methyl-6-phenyloctanoyl]-*N*-cyclohexylmethyl-(*R*)-prolinamide (TS-MS: m/z 452, [M+H]⁺)
1-[6-(*S*)-Cyano-7-methyl-6-phenyloctanoyl]-*N*-(4-*t*-butylcyclohexyl)-(*R*)-prolinamide (TS-MS: m/z 494, [M+H]⁺)
1-[6-(*S*)-Cyano-7-methyl-6-phenyloctanoyl]-*N*-(3-phenylpropyl)-(*R*)-prolinamide (TS-MS: m/z 474, [M+H]⁺)
1-[6-(*S*)-Cyano-7-methyl-6-phenyloctanoyl]-*N*-(4-phenylbutyl)-(*R*)-prolinamide (TS-MS: m/z 488, [M+H]⁺)
1-[6-(*S*)-Cyano-7-methyl-6-phenyloctanoyl]-*N*-(4-phenylbut-2-yl)-(*R*)-prolinamide (TS-MS: m/z 488, [M+H]⁺)
1-[6-(*S*)-Cyano-7-methyl-6-phenyloctanoyl]-*N*-(1,2,3,4-tetrahydroisoquinolin-2-yl)-(*R*)-prolinamide (TS-MS: m/z 472, [M+H]⁺)
1-[6-(*S*)-Cyano-7-methyl-6-phenyloctanoyl]-*N*-[1-(4-fluorophenyl)-2-methylprop-2-yl]-(*R*)-prolinamide (TS-MS: m/z 506, [M+H]⁺)
1-[6-(*S*)-Cyano-7-methyl-6-phenyloctanoyl]-*N*-[1-(*R*)-(ethoxycarbonyl)benzyl]-(*R*)-prolinamide (TS-MS: m/z 518, [M+H]⁺)
1-[6-(*S*)-Cyano-7-methyl-6-phenyloctanoyl]-*N*-[1-(*S*)-(ethoxycarbonyl)-2-phenylethyl]-(*R*)-prolinamide (TS-MS: m/z 532, [M+H]⁺)

The following formulations can be prepared using a compound of the invention as active ingredient.

### EXAMPLE 20

### Soft gelatin capsule

Each soft gelatin capsule contains:

| | |
|---|---|
| Active ingredient | 150 mg |
| Arachis oil | 150 mg |

After mixing together, the blend is filled into soft gelatin capsules using the appropriate equipment.

### EXAMPLE 21

### Hard gelatin capsule

Each active capsule contains:

| | |
|---|---|
| Active ingredient | 50 mg |
| PEG 4000 | 250 mg |

The PEG 4000 is melted and mixed with the active ingredient. Whilst still molten the mixture is filled into capsule shells and allowed to cool.

### EXAMPLE 22

Tablets each containing 10 mg of active ingredient are made up as follows:

| | |
|---|---|
| Active ingredient | 10 mg |
| Starch | 160 mg |
| Microcrystalline cellulose | 100 mg |
| Polyvinylpyrrolidone (as 10% solution in water) | 13 mg |
| Sodium carboxymethyl starch | 14 mg |
| Magnesium stearate | 3 mg |
| Total | 300 mg |

The active ingredient, starch and cellulose are mixed thoroughly. The solution of polyvinylpyrrolidone is mixed with the resultant powders and passed through a sieve. The granules so produced are dried and re-passed through a sieve. The sodium carboxymethyl starch and magnesium stearate are then added to the granules which, after mixing, are compressed in a tablet machine to yield tablets each weighing 300 mg.

## Claims

1. A compound of the formula: 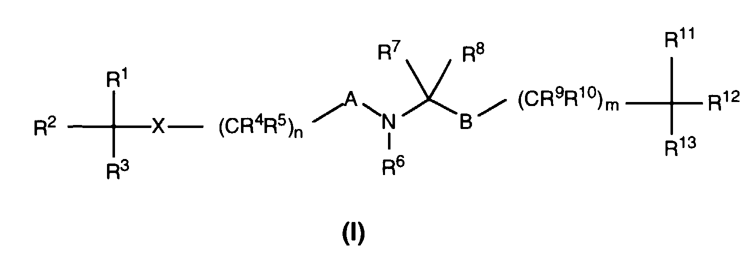 in which
R¹ is alkyl, -SO₂R¹⁴ where R¹⁴ is alkyl, optionally substituted aryl or optionally substituted heteroaryl,
R² is hydrogen, alkyl optionally substituted with one or more hydroxy or C₁₋₄ alkoxy groups, optionally substituted aryl, optionally substituted heteroaryl, -OR¹⁵, -P(O) (OR¹⁵)(OR¹⁶) or -NR¹⁵ R¹⁶ where R¹⁵ and R¹⁶ are each hydrogen or alkyl, or -SO₂R¹⁷ where R¹⁷ is hydrogen, alkyl, optionally substituted aryl or optionally substituted heteroaryl,
R³ is hydrogen, -CN, -CO₂R¹⁸, -CONR¹⁸R¹⁹ ,-CH¹⁸≡CHR¹⁹, -C≡CR¹⁸, -OCH₂CO.R¹⁸, -SO₂R¹⁸, -CH₂OR¹⁸, -CH₂OCO.R¹⁸, -CH₂NHCOR¹⁸, -CH₂N(COR¹⁸)₂, -CHO, -OR¹⁸, -CH₂NR¹⁸R¹⁹, -CH₂OR¹⁸, -CH=NR¹⁸ or -CH=N-OR¹⁸ , where R¹⁸ and R¹⁹ are each hydrogen or alkyl,
X is a bond or -SO₂-,
R⁴, R⁵, R⁶, R⁸, R⁹ and R¹⁰ are each hydrogen or alkyl,
n is 1 to 4,
A is -CH₂- or -YCO- where Y is a bond, -O-, optionally substituted phenylene, -CH=CH-, or -NR²⁰ - where R²⁰ is hydrogen or alkyl,
R⁷ is the C-α substituent of an amino acid or an ester thereof, or R⁶ and R⁷ together form a C₃₋₅ alkylene chain optionally substituted by C₁₋₄ alkyl or hydroxyl, or a group of the formula -CH₂-Z-CH₂- where Z is -CO-, -S-, -SO- or SO₂-,
or R⁷ and R⁸ together form a C₃ to C₅ alkylene chain optionally substituted by C₁₋₄ alkyl or hydroxyl,
B is -CON(R²¹)-, -CON(R²¹)N(R²²)- or 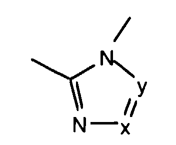 where -x=y- is -C(R²²)=N-,
-N=C(R²²)- or -N=N-, where R²¹, is hydrogen, alkyl or -NR²³ R²⁴ where R²³ and R²⁴ are each hydrogen or alkyl, and where R²² is hydrogen or alkyl, or R⁸ and R²¹ together form a C₂₋₄ alkylene chain,
m is 0 to 2,
R¹¹ is hydrogen or alkyl,
R¹² is hydrogen, alkyl, optionally substituted aryl, optionally substituted heteroaryl, -CN, -OR, -CO₂R²⁵, -CONR²⁵R²⁶ , where R²⁵ and R²⁶ are each hydrogen or alkyl, and
R¹³ is alkyl, optionally substituted aryl or optionally substituted heteroaryl, or R¹² and R¹³ together form a C₃₋₈ cycloalkyl group or C₃₋₈ cycloalkyl fused to optionally substituted phenyl;
or a salt or ester thereof.

2. A compound according to Claim 1 in which in which R¹ and R¹³ are each optionally substituted aryl or optionally substituted heteroaryl, R² is hydrogen, alkyl optionally substituted with one or more hydroxy or C₁₋₄ alkoxy group, optionally substituted aryl, optionally substituted heteroaryl, -OR¹⁵ , -SO₂R¹⁵, -P(O)(OR¹⁵)(OR¹⁶), -NR¹⁵R¹⁶, where R¹⁵ and R¹⁶ are each hydrogen or alkyl,
R³ is hydrogen, -CN, -CO₂R¹⁸, -CONR¹⁸R¹⁹, -CR¹⁸=CHR¹⁹,
-C≡CR¹⁸, -CHO, -OR¹⁸, -CH₂NR¹⁸R¹⁹, -CH₂OR¹⁸, -CH=NR¹⁸ or -CH=N-OR¹⁸, where R¹⁸ and R¹⁹ are each hydrogen or alkyl,
X is a bond,
R⁴, R⁵, R⁶, R⁸, R⁹ and R¹⁰ are each hydrogen or alkyl,
n is 2 to 4 and m is 0 to 2,
A is -CH₂- or 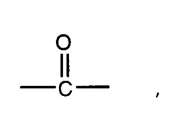
R⁷ is the C-α substituent of an amino acid or an ester thereof, or R⁶ and R⁷ or R⁷ and R⁸, together form a C₃ to C₅ alkylene chain optionally substituted by C₁₋₄ alkyl or hydroxyl,
B is a group of the formula: 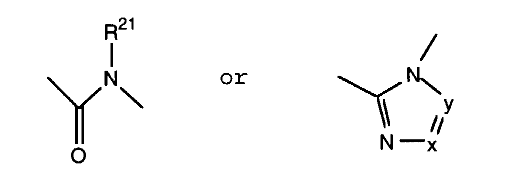 where -x=y- is -C(R²¹)=N-, -N=C (R²¹)- or -N=N-, where R²¹ is hydrogen or alkyl,
R¹¹ is hydrogen or alkyl, and
R¹² is hydrogen, alkyl, optionally substituted aryl, optionally substituted heteroaryl, -CN, -OH, -CO₂R²⁵, -CONR²⁵R²⁶ where R²⁵ and R²⁶ are each hydrogen or alkyl;
or a salt or ester thereof.

3. A compound according to Claim 2, in which R¹ and R¹³ are optionally substituted phenyl, R² is alkyl, R³ is -CN, X is a bond, R⁴, R⁵, R^{6,} R⁸, R⁹ and R¹⁰ are hydrogen, n is 2, m is 0 or 1, A is -CH₂-, R⁷ is hydrogen, or C₁₋₄ alkyl optionally substituted by one or more hydroxy, mercapto, C₁₋₄ alkylthio, optionally substituted phenyl, amino, guanidino, carboxy, imidazolyl or indolyl, or R⁶ and R⁷ together form a C₃ alkylene chain,
B is 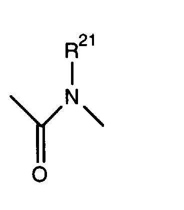 where R²¹ is hydrogen or alkyl, R¹¹ is hydrogen or alkyl, and R¹² is hydrogen, optionally substituted phenyl or -CO₂R²⁵ where R²⁵ is hydrogen or alkyl.

4. A compound according to Claim 1, of the formula: 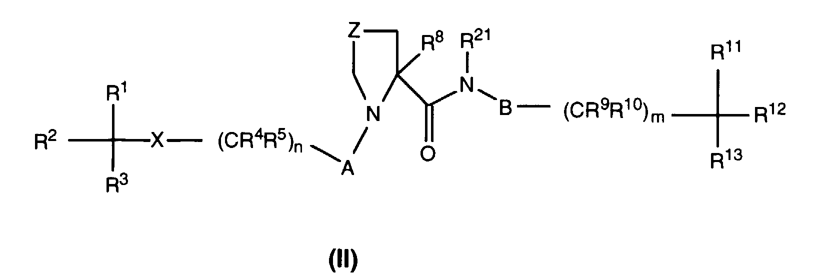
in which R¹ is C₁₋₆ alkyl or -SO₂R¹⁴ where R¹⁴ is C₁₋₆ alkyl,
R² is optionally substituted aryl, optionally substituted heteroaryl or -SO₂R¹⁷ where R¹⁷ is C₁₋₆ alkyl, optionally substituted aryl or optionally substituted heteroaryl,
R³ is -CN, -CO₂R¹⁸, -CONR¹⁸R¹⁹, -C≡CR¹⁸, -OCH₂CO.R¹⁸, -SO₂R¹⁸, -CH₂OR¹⁸, -CH₂OCO.R¹⁸, -CH₂NHCOR¹⁸ or -CH₂N(COR¹⁸)₂, where R¹⁸ and R¹⁹ are each hydrogen or C₁₋₆ alkyl,
R⁴, R⁵, R⁹ and R¹⁰ are each hydrogen or C₁₋₆ alkyl,
R⁸ is hydrogen or C₁₋₆ alkyl,
R²¹ is hydrogen, C₁-₆ alkyl or -NR²³R²⁴ where R²³ and R²⁴ are each hydrogen or C₁₋₆ alkyl,
or R⁸ and R²¹ together form a C₂₋₄ alkylene group,
R¹¹ is hydrogen or C₁₋₆ alkyl,
R¹² is hydrogen, C₁₋₆ alkyl, optionally substituted aryl, optionally substituted heteroaryl, -CN, -OH, -CO₂R²⁵, -CONR²⁵R²⁶ where R²⁵ and R²⁶ are each hydrogen or C₁₋₆ alkyl,
R¹³ is C₁₋₆ alkyl,optionally substituted aryl or optionally substituted heteroaryl,
or R¹² and R¹³ together form a C₃₋₈ cycloalkyl group or C₃₋₈ cycloalkyl fused to optionally substituted phenyl,
n is 1 to 4 and m is 0 to 2,
X is a bond or -SO₂-,
A is -YCO- where Y is a bond or -O-, optionally substituted , phenylene, -CH=CH- or -NR²⁰- where R²⁰ is hydrogen or C₁₋₆ alkyl,
Z is -CH₂-, -CH(OH)-, -CO-, -S-, -SO- or -SO₂-, and
B is a bond or -N(R²²)-
where R²² is hydrogen or C₁₋₆ alkyl;
or a salt or ester thereof.

5. A compound according to Claim 4, in which R¹ is C₁₋₆ alkyl, R² is optionally substituted phenyl, pyridyl, phenylsulphonyl or thienylsulphonyl, R³ is -CN, -CO₂R¹⁸, -CH₂OR¹⁸, -CH₂OCOR¹⁸ or -CH₂NHCOR¹⁸, R⁴, R⁵, R⁹ and R¹⁰ are hydrogen, R⁸ and R²¹ are hydrogen, or R⁸ and R²¹ are combined to form -(CH₂)₂-, R¹¹ is optionally substituted phenyl, R¹² is hydrogen or optionally substituted phenyl, R¹³ is hydrogen, n is 3 or 4, m is 0, Y is a bond, -O- or -NH-, A is -CH₂-, B is a bond, and X is a bond.

6. A compound of the formula: 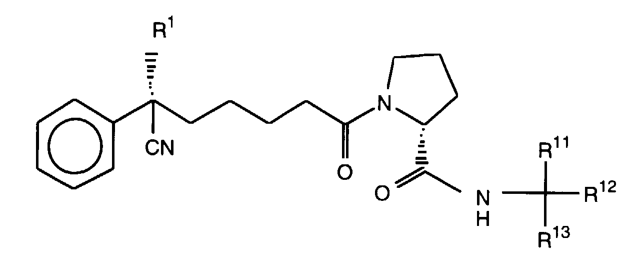
in which R¹ is C₁₋₆ alkyl, R¹¹ is hydrogen, R¹² is hydrogen or optionally substituted phenyl and R¹³ is C₁₋₆ alkyl or optionally substituted phenyl;
or a salt or ester thereof.

7. A pharmaceutical formulation comprising a compound according to any of Claims 1 to 6, or a pharmaceutically acceptable salt or ester thereof, together with a pharmaceutically acceptable diluent or carrier therefor.

8. A compound according to Claim 1, for use as a pharmaceutical.

9. Use of a compound according to any of Claims 1 to 6, for the manufacture of a medicament for treating a disease of the central nervous system.
